(19) Europäisches Patentamt
European Patent Office
Office européen des brevets

(11) Veröffentlichungsnummer: **0 199 845**
**B1**

# (12) EUROPÄISCHE PATENTSCHRIFT

(45) Veröffentlichungstag der Patentschrift: **01.08.90**

(21) Anmeldenummer: **85114205.9**

(22) Anmeldetag: **07.11.85**

(51) Int. Cl.[5]: **C 07 D 233/64,** C 07 D 401/12, C 07 D 403/12, C 07 D 409/12, C 07 D 417/12, A 61 K 31/415, A 61 K 31/44, A 61 K 31/425

## (54) Imidazolylalkylguanidinderivate, Verfahren zu ihrer Herstellung und diese Verbindungen enthaltende Arzneimittel.

(30) Priorität: **06.08.85 DE 3528215**
**06.08.85 DE 3528214**
**02.04.85 DE 3512084**

(43) Veröffentlichungstag der Anmeldung:
**05.11.86 Patentblatt 86/45**

(45) Bekanntmachung des Hinweises auf die Patenterteilung:
**01.08.90 Patentblatt 90/31**

(84) Benannte Vertragsstaaten:
**AT BE CH DE FR GB IT LI LU NL SE**

(56) Entgegenhaltungen:
**DE-A-2 433 625**
**DE-A-2 819 874**

**Burgers Medicinal Chemistry, 4 Ed. Part III, Seiten 541-545**

**Die Akte enthält technische Angaben, die nach dem Eingang der Anmeldung eingereicht wurden und die nicht in dieser Patentschrift enthalten sind.**

(73) Patentinhaber: **HEUMANN PHARMA GMBH & CO**
**Heideloffstrasse 18 - 28**
**D-8500 Nürnberg 1 (DE)**

(72) Erfinder: **Buschauer, Armin, Dr.**
**Bachestrasse 5**
**D-1000 Berlin 41 (DE)**
Erfinder: **Schickaneder, Helmut, Dr. Dipl.-Chem.**
**Moosäcker 25**
**D-8501 Eckental (DE)**
Erfinder: **Schunack, Walter, Prof. Dr. Dr. Dipl.-Chem.**
**Spanische Allee 95**
**D-1000 Berlin 38 (DE)**
Erfinder: **Elz, Sigurd**
**Hans-Böckler-Strasse 35**
**D-6500 Mainz (DE)**
Erfinder: **Szelenyi, Istvan, Dr.**
**Haendelstrasse 32**
**D-8501 Schwaig (DE)**
Erfinder: **Baumann, Gert, Dr.**
**Ismaninger Strasse 22**
**D-8000 München 80 (DE)**
Erfinder: **Ahrens, Kurt Henning, Dr.**
**Praterstrasse 9**
**D-8500 Nürnberg (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 199 845 B1

(74) Vertreter: **Kraus, Walter, Dr. et al**
**Patentanwälte Kraus, Weisert & Partner**
**Thomas-Wimmer-Ring 15**
**D-8000 München 22 (DE)**

**Beschreibung**

Die Erfindung betrifft neue Imidazolylalkylguanidinderivate, die aufgrund ihrer agonistischen Wirkung auf Histamin-$H_2$-Rezeptoren sowie zum Teil wegen ihrer zusätzlichen $H_1$-antagonistischen Rezeptoraktivität bei Erkrankungen des Herzens, bei bestimmten Formen der Hypertonie sowie bei arteriellen Verschlußkrankheiten eingesetzt werden können.

Histamin als spezifischer Stimulator der $H_2$-Rezeptoren löst wegen seiner $H_1$-agonistischen Wirkung negative, zum Teil tödliche Effekte in Form eines Bronchospasmus und anaphylaktischen Schocks aus, so daß die therapeutische Nutzung des Histamins bei der Therapie der genannten Erkrankungen nicht möglich ist.

Aus DE—A—24 33 625 sind Harnstoffderivate der allgemeinen Formel

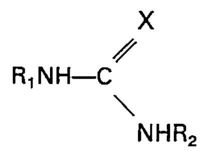

bekannt, die eine $H_2$-antagonistische Wirkung aufweisen und daher zur Hemmung der Magensäuresekretion verwendet werden können.

Der Erfindung liegt die Aufgabe zugrunde, die unvorteilhaften Wirkungen des Histamins zu kompensieren und bessere und selektiv wirksamere $H_2$-Agonisten zur Verfügung zu stellen, bei denen durch eine $H_1$-agonistische Wirkkomponente bedingte schädliche Nebenwirkungen u.U. durch ein zusätzliches $H_1$-antagonistisches Wirkprofil vermieden werden können.

Diese Aufgabe wird durch die Erfindung gelöst.

Gegenstand der Erfindung sind Imidazolylalkylguanidinderivate der allgemeinen Formel I

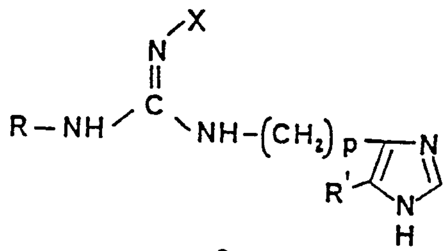 (I)

in der R die Gruppierung

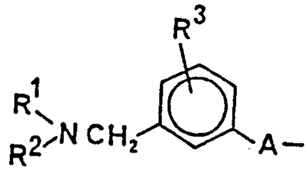

bedeutet, wobei $R^1$ und $R^2$, die gleich oder verschieden sein können, jeweils für Wasserstoff, lineares $C_1$—$C_3$-Alkyl oder $C_5$—$C_6$-Cycloalkylstehen oder $R^1$ und $R^2$ zusammen mit dem Stickstoffatom, an das sie gebunden sind, einen Pyrrolidin-, Piperidin- oder Homopiperidinring bilden, $R^3$ für ein Wasserstoffatom, ein Halogenatom oder eine $C_1$—$C_3$-Alkoxygruppe steht, A die Gruppierung

$$—O—(CH_2)_k—, \quad —O—CH_2CH(OH)CH_2—, \quad —O—CH_2CH(CH_3)—CH_2—,$$

$$—CH_2—O—CH_2—CH(OH)—CH_2 \quad \text{oder} \quad —O—CH_2—CH(OH)—CH(OH)—CH_2$$

bedeutet, worin k den Wert 3 oder 4 hat, oder in der R die Gruppierung

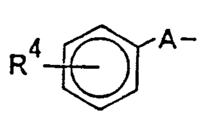 oder 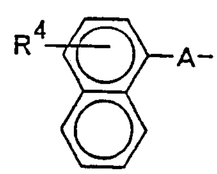

bedeutet, wobei $R^4$ für ein Wasserstoffatom, ein vorzugsweise in para-Stellung zu A gebundenes Halogenatom, eine $C_1$—$C_3$-Alkoxy- oder $C_1$—$C_3$-Alkylgruppe steht und A die oben genannte Bedeutung besitzt,

oder in der R die Gruppierung

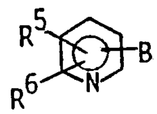

bedeutet, wobei $R^5$ und $R^6$, die gleich oder verschieden sein können, jeweils für ein Wasserstoffatom, ein Halogenatom oder eine lineare $C_1$—$C_3$-Alkyl- oder eine lineare $C_1$—$C_3$-Alkoxygruppe stehen, B in Position 2, 3 oder 4 des Pyridinrings gebunden sein kann und die Gruppierung

$$-\underset{\underset{Y}{|}}{N}-(CH_2)_1$$

bedeutet, wobei 1 den Wert 2, 3 oder 4 hat und Y für ein Wasserstoffatom oder für eine lineare $C_1$—$C_3$-Alkylgruppe steht,

oder in der R die Gruppierung R″—A′—B′— bedeutet, wobei R″ für eine unsubstituierte oder mit einem Halogenatom, einer $C_1$—$C_3$-Alkylgruppe oder einer $C_1$—$C_3$-Alkoxygruppe substituierte Phenyl- oder Naphthylgruppe steht. A′ für eine Einfachbindung oder für die Gruppierung —$CR^{1'}R^{2'}$ oder für ein — mit einer gegebenenfalls mit Halogenatomen substiuierten Phenyl- oder Benzylgruppe oder linearen $C_1$—$C_3$-Alkylgruppe substituiertes — Stickstoffatom steht, wobei $R^{1'}$ ein Wasserstoffatom oder eine Methylgruppe bedeutet. $R^{2'}$ für eine gegebenenfalls mit einem Halogenatom oder einer $C_1$—$C_3$-Alkylgruppe substituierte Phenyl- oder Pyridylgruppe steht.

B′ für die Gruppierungen

—CH(Y)-S-$(CH_2)_{m'}$—, —$CH_2$—S—$CH_2$—CH(Y)—$CH_2$—, —$CH_2$—S—CH(Y)—$CH_2$—,

—$CH_2$—S—$CH_2$—CH(Y)—, —$(CH_2)_{n''}$—, —$CH_2$—CH(Y)—,

—$(CH_2)_{n''}$—CH(Y)—, —O—$(CH_2)_2$—, —$CH_2$—O—$(CH_2)_{o'}$—,

—$CH_2$—O—$CH_2$—CH(Y)—$CH_2$—, —O—$CH_2$—CH(Y)—, —O—CH(Y)—$CH_2$—,

—S—$(CH_2)_q$—, —S—$CH_2$—CH(Y)—, —S—CH(Y)—$CH_2$— oder —S—$CH_2$—CH(Y)—$CH_2$

steht, wobei Y ein Wasserstoffatom oder eine lineare $C_1$—$C_3$-Alkylgruppe bedeutet, m′ und o′ den Wert 2 oder 3 haben, n″ und q den Wert 2, 3, 4 oder 5 haben,

oder in der R die Gruppierung R‴—A″—B″— bedeutet, wobei R‴ für eine unsubstituierte oder mit Halogenatom, $C_1$—$C_3$-Alkylgruppen oder der Gruppe $R^1R^2N$—$CH_2$— substituierte Pyridyl-, Thiophenyl-, Furanyl-, Chinolyl- oder Benzimidazolylgruppe steht, A″ für eine Einfachbindung oder für die Gruppierung —$CR^{1'}R^{2'}$ oder für ein — mit einer gegebenenfalls mit Halogenatom, $C_1$—$C_3$-Alkylgruppen oder $C_1$—$C_3$-Alkoxygruppen substituierte Phenyl- oder Benzylgruppe substituiertes — Stickstoffatom steht, wobei $R^{1'}$ ein Wasserstoffatom oder eine Methylgruppe bedeutet, $R^{2'}$ für eine gegebenenfalls mit Halogenatomen substituierte Phenyl- oder Thiophenylgruppe steht, mit der Maßgabe, daß A″ nicht für eine Einfachbindung steht, wenn R‴ eine Pyridylgruppe ist,

B″ für Gruppierung

—CH(Y)—S—$(CH_2)_{m'}$, —$CH_2$—S—$CH_2$—CH(Y)—$CH_2$—, —$CH_2$—S—CH(Y)—$CH_2$—,

—$(CH_2)_{n''}$—CH(Y)—, —$CH_2$—S—$CH_2$—CH(Y)—, —$(CH_2)_{n''}$—,

—$O(CH_2)_{n''}$—, —S—$CH_2$—CH(Y)—, —S—CH(Y)—$CH_2$—,

—S—$(CH_2)_q$— oder —S—$CH_2$—CH(Y)—$CH_2$—

steht, wobei Y ein Wasserstoffatom oder lineare $C_1$—$C_3$-Alkylgruppe bedeutet, m′ den Wert 2 oder 3 hat und n″ und q den Wert 2, 3, 4 oder 5 haben, X ein Wasserstoffatom oder eine Benzoylgruppe bedeutet, p den Wert 2 oder 3 hat und R′ ein Wasserstoffatom oder eine Methylgruppe bedeutet, sowie die physiologisch annehmbaren Salze davon.

Bei einer Gruppe von erfindungsgemäßen Verbindungen steht R für die Gruppierung

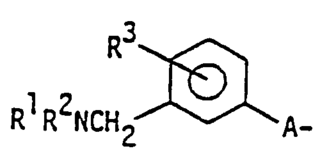

wobei die Substituenten $R^1$ und $R^2$, die gleich oder verschieden sein können, jeweils ein Wasserstoffatom, eine lineare $C_1—C_3$-Alkylgruppe, zum Beispiel eine Methyl-, Ethyl- oder n-Propylgruppe, insbesondere eine Methylgruppe, bedeuten. $R^1$ und $R^2$ können aber auch zusammen mit dem Stickstoffatom, an das sie gebunden sind, einen Pyrrolidin-, Piperidin- oder Homopiperidinring bilden, $R^3$ steht für ein Wasserstoffatom oder ein Halogenatom, das in Ortho-, meta- oder para-Stellung, vorzugsweise in ortho-Stellung, zur $(R^1R^2)N—CH_2$-Gruppierung gebunden ist. $R^3$ kann auch eine $C_1—C_3$-Alkoxygruppe, zum Beispiel eine Methoxy-, Ethoxy- oder Propoxygruppe, vorzugsweise eine Methoxygruppe, die ebenfalls in ortho-, meta- oder para-Stellung, vorzugsweise in para-Stellung, zur $(R^1R^2)N—CH_2$-Gruppierung gebunden ist, sein. A bedeutet eine der folgenden Gruppierungen:

$$—O(CH_2)_k—,\ —O—CH_2CH(OH)CH_2—,\ O—CH_2—CH(CH_3)—CH_2,$$

$$—CH_2—O—CH_2—CH(OH)—CH_2— \text{ oder } —O—CH_2—CH(OH)—CH(OH)—CH_2—.$$

Darin hat k den Wert 3 oder 4, wobei der Wert 3 bevorzugt wird. X bedeutet ein Wasserstoffatom oder eine Benzoylgruppe, vorzugsweise ein Wasserstoffatom, und R' steht für eine Wasserstoffatom oder eine Methylgruppe, vorzugsweise für ein Wasserstoffatom, während p den Wert 2 oder 3 hat.

Unter diesen Verbindungen werden solche bevorzugt, die dadurch gekennzeichnet sind, daß R für die Gruppierung

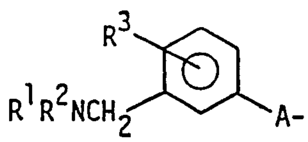

steht, $R^1$ und $R^2$ zusammen mit dem Stickstoffatom einen Pyrrolidin- oder Piperidinring bilden, $R^3$ für ein Wasserstoffatom steht, A die Gruppierung

$$—O—(CH_2)_k—,\ —O—CH_2CH(OH)CH_2— \text{ oder } —O—CH_2CH(CH_3)CH_2—$$

bedeutet, X und R' jeweils für ein Wasserstoffatom stehen und k und p jeweils den Wert 3 haben.

Bei einer weiteren Gruppe von erfindungsgemäßen Verbindungen steht in der allgemeinen Formel I R für die Gruppierung

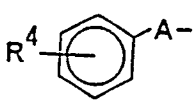

oder

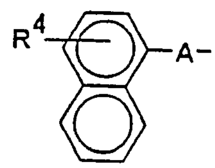

$R^4$ bedeutet ein Wasserstoffatom, ein vorzugsweise in para-Stellung zu A gebundenes Halogenatom, wie zum Beispiel ein Fluor-, Brom- oder Chloratom, vorzugsweise ein Chloratom, eine $C_1—C_3$-Alkoxygruppe, wie eine Methoxy- oder Ethoxygruppe, eine $C_1—C_3$-Alkylgruppe, wie eine Methyl- oder Ethylgruppe, ganz besonders bevorzugt ein Wasserstoffatom. A hat die obige Definition und bedeutet vorzugsweise die Gruppierung $—O—(CH_2)_3—$ oder $—O—CH_2CH(OH)CH_2—$; X hat ebenfalls die obige Definition und bedeutet vorzugsweise ein Wasserstoffatom, p hat die obige Definition und hat vorzugsweise den Wert 3, R' steht wiederum für ein Wasserstoffatom oder eine Methylgruppe, vorzugsweise für ein Wasserstoffatom.

Unter diesen Verbindungen werden solche bevorzugt, die dadurch gekennzeichnet sind, daß R für die Gruppierung

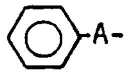

oder

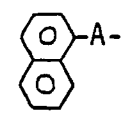

steht, wobei A die oben genannte Bedeutung besitzt, X und R' jeweils für ein Wasserstoffatom stehen und p den Wert 3 hat.

Bei einer weiteren Gruppe von erfindungsgemäßen Verbindungen steht in der allgemeinen Formel I R für die Gruppierung

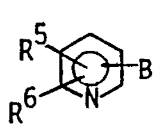

wobei $R^5$ und $R^6$, die gleich oder verschieden sein können, jeweils für ein Wasserstoffatom, ein Halogenatom, zum Beispiel für die Fluor-, Chlor- oder Bromatom, vorzugsweise für ein Bromatom, stehen. Das Halogenatom kann vorzugsweise in 3- und/oder 5-Stellung des Pyridinrings gebunden sein. Wenn $R^6$ ein Wasserstoffatom ist, dann steht $R^5$ vorzugsweise für ein Halogenatom, zum Beispiel ein Fluor-, Chlor- oder Bromatom, insbesondere ein Bromatom. Das Halogenatom kann vorzugsweise in 3- oder 5-Stellung, insbesondere in 5-Stellung, des Pyridinrings gebunden sein. $R^5$ und $R^6$ können weiterhin jeweils eine $C_1$—$C_3$-Alkyl- oder $C_1$—$C_3$-Alkoxygruppe, vorzugsweise eine in 3- und/oder 5-Stellung des Pyridinrings gebundene Methyl- oder Methoxygruppe sein. Steht $R^5$ für ein Wasserstoffatom, dann bedeutet $R^6$ vorzugsweise eine in 3- oder 5-Stellung des Pyridinrings gebundene Methyl- oder Methoxygruppe.

B, das in 2-, 3- oder 4-Position des Pyridinrings, vorzugsweise in 2- oder 3-Position, gebunden sein kann, steht für die Gruppierung

$$\begin{array}{l}—N—(CH_2)_l— \\ \ \ \ \mid \\ \ \ \ Y\end{array}$$

wobei Y ein Wasserstoffatom oder eine lineare $C_1$—$C_3$-Alkylgruppe, vorzugsweise eine Methyl-, Ethyl-, n-Propyl- oder iso-Propylgruppe, insbesondere eine Methylgruppe, bedeutet. l hat den Wert 2, 3 oder 4, vorzugsweise 2 oder 3 hat. X, R' und p haben wiederum die oben angegebenen Definitionen, wobei X vorzugsweise ein Wasserstoffatom bedeutet, R' vorzugsweise ebenfalls ein Wasserstoffatom bedeutet und p vorzugsweise den Wert 3 hat.

Unter diesen Verbindungen werden solche bevorzugt, die dadurch gekennzeichnet sind, daß R für die Gruppierung

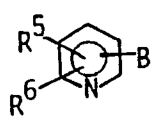

steht, wobei $R^5$ für ein in 5-Stellung des Pyridinrings gebundenes Halogenatom oder Wasserstoffatom steht, $R^6$ für ein in 3-Stellung des Pyridinrings gebundenes Wasserstoffatom oder eine Methyl- oder Methoxygruppe steht, B in Position 2, 3 oder 4 des Pyrindinrings gebunden sein kann und die Gruppierung

$$\begin{array}{l}—N—(CH_2)_3— \\ \ \ \ \mid \\ \ \ \ CH_3\end{array}$$

bedeutet, X und R' jeweils für ein Wasserstoffatom stehen und p den Wert 3 hat.

Bei einer weiteren Gruppe von erfindungsgemäßen Verbindungen steht in der allgemeinen Formel I R für die Gruppierung R''—A'—B'—, wobei R'' für eine unsubstituierte oder mit einem Halogenatom, einer $C_1$—$C_3$-Alkylgruppe oder mit einer $C_1$—$C_3$-Alkoxygruppe substituierte Phenyl- oder Naphthylgruppe steht. Wenn R'' eine substituierte Phenyl- oder substituierte Naphthylgruppe ist, dann kann R'' durch die Gruppierung

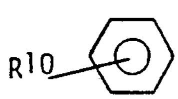 oder 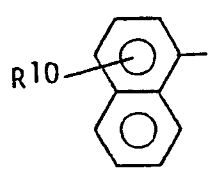

angegeben werden, worin $R^{10}$ ein vorzugsweise in meta- oder para-Stellung zu A' gebundenes Halogenatom, zum Beispiel ein Fluor, Brom-, Chlor- oder Jodatom, vorzugsweise ein Fluoratom oder Chloratom, ganz besonders bevorzugt ein Fluoratom, eine lineare $C_1$—$C_3$-Alkylgruppe, zum Beispiel eine Methyl- oder Ethylgruppe, eine lineare $C_1$—$C_3$-Alkoxygruppe, zum Beispiel eine Methoxygruppe oder eine Trifluormethylgruppe bedeutet. A' steht für eine Einfachbindung oder die Gruppierung —$CR^{1'}R^{2'}$ oder für ein — mit einer gegebenenfalls mit Halogenatomen substituierten Phenyl- oder Benzylgruppe oder linearen $C_1$—$C_3$-Alkylgruppe substituiertes — Stickstoffatom. Die Phenyl- oder Benzylgruppe kann beispielsweise in meta- oder para-Position — vorzugsweise in para-Position — mit einem Halogenatom, zum Beispiel Fluor-, Chlor-, Brom- oder Jodatom, vorzugsweise Fluoratom, substituiert sein.

$R^{1'}$ bedeutet ein Wasserstoffatom oder eine Methylgruppe, während $R^{2'}$ für eine gegebenenfalls mit einem Halogenatom, zum Beispiel einem Fluor-, Brom-, Chlor- oder Jodatom, vorzugsweise einem Fluor- oder Chloratom oder einer linearen $C_1$—$C_3$-Alkylgruppe, z.B. einer Methyl- oder Ethylgruppe oder einer linearen $C_1$—$C_3$-Alkoxygruppe, z.B. einer Methoxygruppe, substituierte Phenyl- oder Pyridylgruppe steht.

B′ steht für eine der Gruppierungen

$-CH(Y)\text{-}S\text{-}(CH_2)_{m'}-$, $-CH_2-S-CH_2-CH(Y)-CH_2-$, $-CH_2-S-CH(Y)-CH_2-$,

$-CH_2-S-CH_2-CH(Y)-$, $-(CH_2)_{n''}-$, $-CH_2-CH(Y)-$,

$-(CH_2)_{n''}-CH(Y)-$, $-O-(CH_2)_2-$, $-CH_2-O-(CH_2)_{o'}-$,

$-CH_2-O-CH_2-CH(Y)-CH_2-$, $-O-CH_2-CH(Y)-$, $-O-CH(Y)-CH_2-$,

$-S-(CH_2)_q-$, $-S-CH_2-CH(Y)-$, $-S-CH(Y)-CH_2-$ oder $-S-CH_2-CH(Y)-CH_2-$.

In diesen Gruppen bedeutet Y ein Wasserstoffatom oder lineare $C_1$—$C_3$-Alkylgruppen, wie oben definiert, vorzugsweise eine Methylgruppe, m′ und o′ haben den Wert 2 oder 3, n″ und q haben den Wert 2, 3, 4 oder 5.

X stellt wiederum ein Wasserstoffatom oder eine Benzoylgruppe dar, p hat den Wert 2 oder 3. R′ steht für ein Wasserstoffatom oder eine Methylgruppe.

Unter diesen Verbindungen werden solche bevorzugt, die dadurch gekennzeichnet sind, daß R die Gruppierung R″—A′—B′— bedeutet, wobei R″ für eine unsubstituierte Phenylgruppe steht, A′ eine Einfachbindung bedeutet, B′ für die Gruppierung $-CH_2-S(CH_2)_{m'}-$ oder $-CH_2-S-CH_2-CH(Y)-CH_2-$ steht, wobei m′ und Y die oben genannten Bedeutungen besitzen, X und R′ jeweils für ein Wasserstoffatom stehen und p den Wert 3 hat.

Unter diesen Verbindungen werden weiterhin solche bevorzugt, die dadurch gekennzeichnet sind, daß R die Gruppierung R″—A′—B′— bedeutet, wobei R″ für eine mit einem Halogenatom, einer $C_1$—$C_3$-Alkylgruppe oder einer $C_1$—$C_3$-Alkoxygruppe substituierte oder unsubstituierte Phenylgruppe steht, A′ ein mit einer Phenyl- oder Benzylgruppe substituiertes Stickstoffatom bedeutet, B′ für die Gruppierung $-(CH_2)_{n''}-$ steht, wobei n″ den Wert 2 oder 3 hat, X und R′ jeweils für ein Wasserstoffatom stehen und p den Wert 3 besitzt.

Unter diesen Verbindungen werden weiterhin solche bevorzugt, die dadurch gekennzeichnet sind, daß R die Gruppierung R″—A′—B′— bedeutet, wobei R″ für eine mit einem Halogenatom, einer $C_1$—$C_3$-Alkylgruppe oder einer $C_1$—$C_3$-Alkoxygruppe substituierte oder unsubstituierte Phenylgruppe steht, A′ für die Gruppierung $-CR^{1'}R^{2'}$, wobei $R^{1'}$ ein Wasserstoffatom oder eine Methylgruppe, bedeutet, $R^{2'}$ für eine gegebenenfalls mit einem Halogenatom oder einer $C_1$—$C_3$-Alkylgruppe substituierte Phenyl- oder Pyridylgruppe steht, B′ die Gruppierung $-(CH_2)_{n''}-$ bedeutet, wobei n″ den Wert 2, 3 oder 4 hat, X und R′ jeweils für ein Wasserstoffatom stehen und p den Wert 3 hat.

Bei einer weiteren Gruppe von erfindungsgemäßen Verbindungen steht in der allgemeinen Formel I R für die Gruppierung R‴—A″—B″—, wobei R‴ für eine unsubstituierte oder mit Halogenatomen, $C_1$—$C_3$-Alkylgruppen oder der Gruppe $R^1R^2N-CH_2-$ substituierte Pyridyl, Thiophenyl-, Furanyl-, Chinolyl- oder Benzimidazolylgruppe steht. Im Falle, daß die wie oben definierte Heterogruppe substituiert ist, kann R‴ durch die Gruppierung

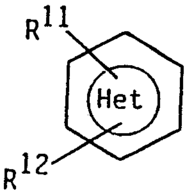

oder

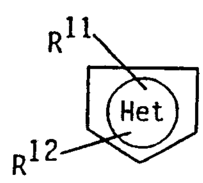

angegeben werden, worin $R^{11}$ und $R^{12}$ unabhängig voneinander jeweils ein Halogenatom wie zum Beispiel ein Fluor-, Brom-, Chlor- oder Jodatom, vorzugsweise ein Fluor- oder Chloratom, eine lineare $C_1$—$C_3$-Alkylgruppe, wie zum Beispiel eine Methyl-, Ethyl- oder Propylgruppe, vorzugsweise eine Methylgruppe, oder eine lineare $C_1$—$C_3$-Alkoxygruppe, vorzugsweise eine Methoxygruppe, bedeuten. A″ steht für eine Einfachbindung, für die Gruppierung $-CR^{1'}R^{2'}$ oder ein Stickstoffatom, das mit einer gegebenenfalls substituierten Phenyl- oder Benzylgruppe oder einem Wasserstoffatom oder einer linearen $C_1$—$C_3$-Alkylgruppe substituiert ist. Die Phenyl- oder Benzylgruppe kann beispielsweise in meta- oder para-Position — vorzugsweise in para-Position — mit einem Halogenatom, zum Beispiel Fluor-, Chlor-, Brom- oder Jodatom, vorzugsweise Fluor- oder Chloratom, oder mit einer linearen $C_1$—$C_3$-Alkylgruppe, zum Beispiel mit einer Methyl-, Ethyl- oder Propylgruppe, vorzugsweise Methylgruppe, oder mit einer linearen $C_1$—$C_3$-Alkoxygruppe, zum Beispiel eine Methoxy-, Ethoxy- oder Propoxygruppe, vorzugsweise Methoxygruppe, substituiert sein.

$R^{1'}$ bedeutet ein Wasserstoffatom oder eine Methylgruppe, während $R^{2'}$ für eine gegebenenfalls mit einem Halogenatom, zum Beispiel einem Fluor-, Brom-, Chlor- oder Jodatom, insbesondere einem Fluor- oder Chloratom, substituierte Phenyl- oder Thiophenylgruppe steht. Wenn A″ eine Einfachbindung ist, dann ist die Einfachbindung an Position 2, 3 oder 3 des wie oben definierten Heteroarlyrestes angeordnet,

d.h. sie verknüpft den Rest B, wie oben definiert, mit dem Heteroarylrest in Position 2, 3 oder 4 des Heteroarylrests. Im Falle, daß der Heteroarylring ein Benzimidazolring ist, kommt als Verknüpfungsstelle nur die Position 2 des Benzimidazolringes in Betracht.

Es gilt die Maßgabe, daß A'' nicht für eine Einfachbindung steht, wenn R''' eine Pyridylgruppe ist.

B'' steht für eine der Gruppierungen

$$—CH(Y)—S—(CH_2)_{m'},\quad —CH_2—S—CH_2—CH(Y)—CH_2—,\quad —CH_2—S—CH(Y)—CH_2—,$$

$$—(CH_2)_{n''}—CH(Y)—,\quad —CH_2—S—CH_2—CH(Y)—,\quad —(CH_2)_{n''}—,\quad —O(CH_2)_{n''}—,$$

$$—S—CH_2—CH(Y)—,\quad —S—CH(Y)—CH_2—,\quad —S—(CH_2)_q—\ \text{oder}\ —S—CH_2—CH(Y)—CH_2—$$

steht, wobei Y ein Wasserstoffatom oder eine lineare $C_1$—$C_3$-Alkylgruppe bedeutet, m' den Wert 2 oder 3 hat und n'' und q den Wert 2, 3, 4 oder 5 haben.

X bedeutet wiederum ein Wasserstoffatom oder eine Benzoylgruppe, während p den Wert 2 oder 3 hat und R' ein Wasserstoffatom oder eine Methylgruppe bedeutet.

Unter diesen Verbindungen werden solche bevorzugt, die dadurch gekennzeichnet sind, daß R die Gruppierung R'''—A''—B''— bedeutet, wobei R''' für einen mit Halogenatomen, $C_1$—$C_3$-Alkylgruppen oder der Gruppe $R^2R^2NCH_2$— substituierten oder unsubstituierten Thiophenring steht, A'' eine Einfachbindung bedeutet, B'' für die Gruppierung $—CH_2—S—(CH_2)_{m'}—$ oder $—CH_2—S—CH_2—CH(Y)—CH_2—$ steht, wobei m' und Y die oben genannten Bedeutungen besitzen, X und R' jeweils für ein Wasserstoffatom stehen und p den Wert 3 besitzt.

Unter diesen Verbindungen werden weiterhin solche bevorzugt, die dadurch gekennzeichnet sind, daß R die Gruppierung R'''—A''—B''— bedeutet, wobei R''' für einen mit Halogenatomen oder $C_1$—$C_3$-Alkylgruppen substituierten oder unsubstituierten Pyridinring steht, A'' die Gruppierung $—CR^{1'}R^{2'}$ bedeutet, wobei $R^{1'}$ und $R^{2'}$ wie oben definiert sind, B'' für die Gruppierung $—(CH_2)_{n''}—$ steht, wobei n'' die oben genannte Bedeutung besitzt, X und R' jeweils für ein Wasserstoffatom stehen und p den Wert 3 besitzt.

Unter diesen Verbindungen werden weiterhin solche bevorzugt, die dadurch gekennzeichnet sind, daß R die Gruppierung R'''—A''—B''— bedeutet, wobei R''' für einen mit Halogenatomen oder $C_1$—$C_3$-Alkylgruppen substituierten oder unsubstituierten Pyridinring steht, A'' für ein — mit einer gegebenenfalls mit Halogenatomen, $C_1$—$C_3$-Alkylgruppen oder $C_1$—$C_3$-Alkoxygruppen substituierten Phenyl- oder Benzylgruppe — substituiertes Stickstoffatom steht, B'' für die Gruppierung $—(CH_2)_{n''}—$ steht, wobei n'' die oben genannte Bedeutung besitzt, X und R' jeweils für ein Wasserstoffatom stehen und p den Wert 3 besitzt.

Bei einer bevorzugten Gruppe von erfindungsgemäßen Verbindungen steht R''' für die Gruppierung

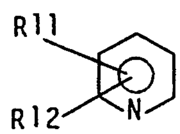

wobei $R^{11}$ und $R^{12}$ wie oben definiert sind. A'' steht in diesem Fall bevorzugt für die Gruppierung $—CR^{1'}R^{2'}$, wobei $R^{1'}$ ein Wasserstoffatom oder eine Methylgruppe bedeutet, vorzugsweise ein Wasserstoffatom ist. $R^{2'}$ steht für eine Phenylgruppe, die gegebenenfalls in 4-Stellung durch ein Halogenatom, wie zum Beispiel Fluor-, Chlor-, Brom- oder Jodatom, vorzugsweise ein Fluor- oder Chloratom, oder durch eine lineare $C_1$—$C_3$-Alkylgruppe, vorzugsweise eine Methylgruppe, substituiert ist. In diesem Fall steht A'' weiterhin vorzugsweise für ein Stickstoffatom, das mit einer Phenyl- oder Benzylgruppe, substituiert ist. B'' steht in diesem Fall, wenn A'' die Gruppierung $—CR^{1'}R^{2'}$ ist, vorzugsweise für die Gruppierung $—(CH_2)_{n''}$, $—O—(CH_2)_2—$ oder $—S—CH_2CH_2—$, insbesondere für — $(CH_2)_{n''}$, wobei n'' wie oben definiert ist und vorzugswise den Wert 2 oder 3 hat. In diesem Falle steht weiterhin B'', wenn A'' ein mit einer Phenyl- oder Benzyl-, Methylgruppe oder Wasserstoffatom substituiertes Stickstoffatom ist, für die Gruppierung $—(CH_2)_{n''}$, wobei n'' vorzugsweise den Wert 2 oder 3 hat. X und R' bedeuten dabei vorzugsweise ein Wasserstoffatom, und p besitzt vorzugsweise den Wert 3.

Bei einer weiteren bevorzugten Gruppe von erfindungsgemäßen Verbindungen steht in der allgemeinen Formel I R''' für die Gruppierung

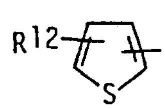

wobei $R^{12}$ ein Wasserstoffatom, ein Halogenatom, vorzugsweise ein Chloratom, eine lineare $C_1$—$C_3$-Alkylgruppe, vorzugsweise eine Methylgruppe, oder die Gruppierung $(CH_3)_2—NCH_2—$ oder

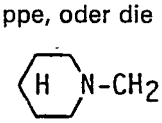

bedeutet, A'' die oben genannte Definition hat und vorzugsweise für eine Einfachbindung in Position 2 steht. B'' steht in diesem Falle für die Gruppierung

$$-CH_2-S-CH_2-CH_2-,\quad -CH_2-S-CH_2-CH(CH_3)-CH_2-$$

$$-CH_2-S-CH(CH_3)-CH_2-\quad \text{oder}\quad -CH_2-S-CH_2-CH(CH_3),$$

vorzugsweise für $-CH_2-S-CH_2CH_2-$. X und R' haben ebenfalls die obige Definition und bedeuten vorzugsweise ein Wasserstoffatom, p besitzt vorzugsweise den Wert 3.

Bei einer weiteren bevorzugten Gruppe von erfindungsgemäßen Verbindungen steht in der allgemeinen Formel I R''' für die Gruppierung

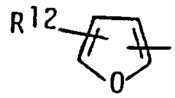

wobei $R^{12}$ wie oben definiert ist. A'' bedeutet vorzugsweise eine Einfachbinding in Position 2, B'' steht für der oben genannten Gruppierungen, vorzugsweise für $-CH_2-S-CH_2CH_2-$, während X und R' vorzugsweise für ein Wasserstoffatom stehen und p vorzugsweise den Wert 3 besitzt.

Bei einer weiteren bevorzugten Gruppe von erfindungsgemäßen Verbindungen steht in der allgemeinen Formel I R''' für die Gruppierung

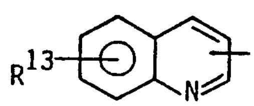 oder 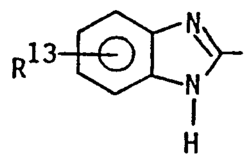

wobei $R^{13}$ die gleiche Bedeutung wie $R^{12}$ hat, A'' vorzugsweise eine Einfachbindung bedeutet, B'' für eine der oben genannten Gruppierungen, vorzugsweise für $-CH_2-S-CH_2CH_2-$, steht, X und R' vorzugsweise für ein Wasserstoffatom stehen und p vorzugsweise den Wert 3 besitzt.

Ganz besonders werden folgende Einzelverbindungen und ihre physiologisch annehmbaren Salze bevorzugt:

N-[3-[(N-5-Methyl-pyridin-2-yl)-methylamino]propyl]-N'-[3-(1H-imidazol-4-yl)propyl]guanidin
N-[3-(Imidazol-4-yl)propyl]-N'-(3,3-diphenylpropyl)guanidin
N-[3-(Imidazol-4-yl)propyl]-N'-[2-[(1-phenylethyl)thio]ethyl]guanidin
N-[3-(Imidazol-4-yl)propyl]-N'-[2-[(pyrid-3-yl)methylthio]ethyl]guanidin
N-[3-(Imidazol-4-yl)propyl]-N'-[2-(2-thenylthio)ethyl]guanidin
N-[3-(Imidazol-4-yl)propyl]-N'-[1-methyl-2-[(pyrid-2-yl)methylthio]ethyl]guanidin
N-[3-(Imidazol-4-yl)propyl]-N'-[3-phenyl-3-(pyrid-2-yl)propyl]guanidin
N-[3-(4-Chlorphenyl)-3-(pyrid-2-yl)propyl]-N'-[3-imidazol-4-yl)propyl]guanidin
N-[3-(4-Bromphenyl)-3-pyrid-2-yl)propyl]-N'-[3-(imidazol-4-yl)propyl]guanidin
N-[3-(4-Fluorphenyl)-3-pyrid-2-yl)propyl]-N'-[3-(imidazol-4-yl)propyl]guanidin

Die Erfindung umfaßt auch alle stereoisomeren Formen und Hydrate der oben beschriebenen Verbindungen der allgemeinen Formel I. Erfindungsgemäße Verbindungen, bei denen R, p und R' wie oben definiert sind und X für eine Benzoylgruppe steht, können nach zwei verschiedenen Verfahrensvarianten hergestellt werden, nämlich

($a_1$) durch Umsetzung einer Verbindung der allgemeinen Formel II

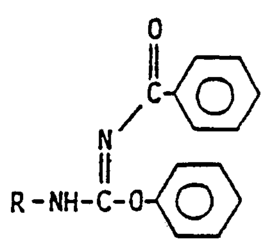

(II)

in der R die oben angegebene Bedeutung besitzt, mit einer Verbindung der allgemeinen Formel III

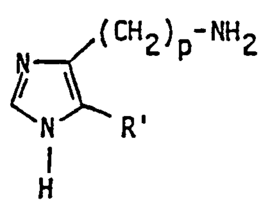

(III)

in der R' und p die oben angegebenen Bedeutungen besitzen, zu einer Verbindung der allgemeinen Formel I.

Die Umsetzung der Reaktanten erfolgt vorzugsweise in äquimolaren Mengen und in einem polaren Lösungsmittel, wie zum Beispiel einem Alkohol, wie Methanol, Ethanol, oder Isopropanol, vorzugsweise Ethanol, oder in Acetonitril, Dimethylsulfoxid, Dimethylformamid, Pyridin, vorzugsweise in Acetonitril oder Pyridin, bei Raum- oder Rücktemperatur des verwendeten Lösungsmittels.

($a_2$) oder durch Umsetzung einer Verbindung der allgemeinen Formel IV

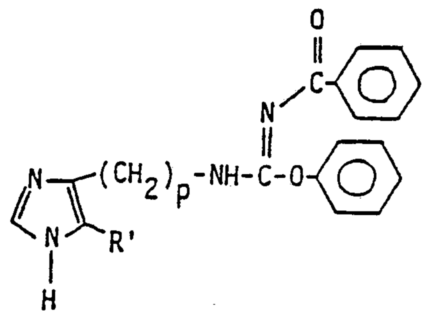

(IV)

in der R' und p die oben angegebenen Bedeutungen besitzen, mit einer Verbindung der allgemeinen Formel V

$$R—NH_2 \qquad (V)$$

in der R die oben angegebene Bedeutung besitzt, zu einer Verbindung der allgemeinen Formel I.

Die verwendeten Mengen und Lösungsmittel sowie Reaktionsbedingungen sind die gleichen wie oben im Zusammenhang mit der Verfahrensvariante $a_1$ beschrieben.

Erfindungsgemäße Verbindungen der allgemeinen Formel I, bei denen R, p und R' wie oben definiert sind und X für ein Wasserstoffatom steht, können nach einer der folgenden vier Verfahrensvarianten hergestellt werden:

($b_1$) durch Hydrolyse einer Verbindung der Formel Ia

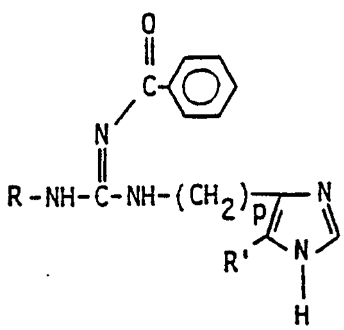

(Ia)

in der R, p und R' die oben angegebenen Bedeutungen besitzen. Die Hydrolyse kann sauer oder basisch durchgeführt werden, wobei eine saure Hydrolyse, zum Beispiel unter Verwendung von verdünnter Schwefelsäure oder verdünnter Salzsäure, insbesondere Salzsäure, bevorzugt wird. Die Hydrolysenreaktion wird bei erhöhter Temperatur, vorzugsweise bei Rückflußtemperatur, durchgeführt.

($b_2$) durch Hydrolyse einer Verbindung der Formel VI

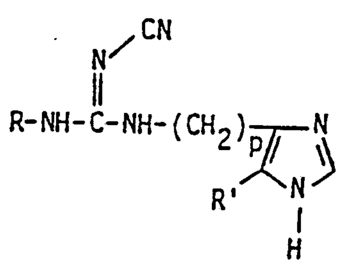

(VI)

in der R, p und R' die obigen Bedeutungen haben, mit Hilfe einer Säure, zum Beispiel verdünnter Schwefelsäure oder verdünnter Salzsäure, vorzugsweise Salzsäure, wie oben angegeben zu einer Verbindung der Formel I hydrolysiert.

($b_3$) durch Umsetzung einer Verbindung der allgemeinen Formel VII

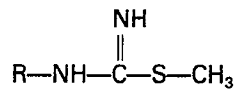

(VII)

in der R die oben angegebene Bedeutung besitzt, mit einer Verbindung der allgemeinen Formel III

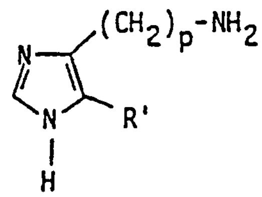

(III)

in der R′ und q die oben angegebenen Bedeutungen besitzen, zu einer Verbindung der allgemeinen Formel I.

Die Umsetzung findet in einem polaren Lösungsmittel, vorzugsweise in Pyridin, über einen Zeitraum von 3 bis 5 Stunden und bei Rückflußtemperatur des verwendeten Lösungsmittels statt.

($b_4$) durch Umsetzung einer Verbindung der allgemeinen Formel VIII

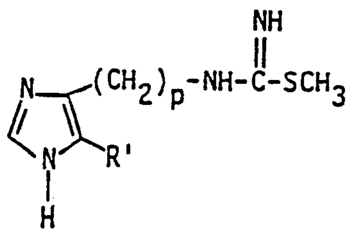

(VIII)

in der R′ und p die oben angegebenen Bedeutungen besitzen, mit einer Verbindung der allgemeinen Formel V, in der R die oben angegebene Bedeutung besitzt, zu einer Verbindung der allgemeinen Formel I.

Auch bei dieser Variante findet die Umsetzung in einem polaren Lösungsmittel, vorzugsweise Pyridin, 3 bis 5 Stunden und bei Rückflußtemperatur des verwendeten Lösungsmittels statt.

Die nach den einzelnen Verfahrensvarianten erhaltenen Verbindungen werden in üblicher Weise isoliert und gereinigt, beispielsweise durch chromatographische Arbeitsweisen, Umkristallisation etc.

Die bei den einzelnen Verfahrensvarianten erhaltenen Verbindungen können gegebenenfalls in ihr physiologisch annehmbares Salz umgewandelt werden.

Die Erfindung umfaßt neben den stereoisomeren Verbindungen und Hydraten der Substanzen der allgemeinen Formel I daher auch die physiologisch annehmbaren Salze dieser Verbindungen. Diese Salze können zum Beispiel mit Mineralsäuren, wie Chlor-, brom-, Jodwasserstoffsäure, Phosphorsäure, Metasphosphorsäure, Salpetersäure oder Schwefelsäure, oder mit organischen Säuren, wie Ameisensäure, Essigsäure, Propionsäure, Phenylessigsäure, Weinsäure, Zitronensäure, Fumarsäure, Methansulfonsäure, Embonsäure etc., gebildet werden.

Die erfindungsgemäßen Verbindungen können zum Verabreichung in jeder beliebigen Weise formuliert werden. Die Erfindung umfaßt daher auch Arzneimitel, die mindestens eine erfindungsgemäße Verbindung zur Verwendung in der Human- oder Veterinärmedizin enthalten. Solche Arzneimittel können herkömmlicherweise unter Verwendung von einem oder mehreren pharmazeutischen Trägern oder Verdünnungsmitteln hergestellt werden.

Die erfindungsgemäßen Verbindungen können daher für die orale, bukkale, topische, parenterale oder rektale Verabreichung formuliert werden.

Für die orale Verabreichung kann das Arzneimittel in Form von beispielsweise Tabletten, Kapseln, Pulvern, Lösungen, Sirups oder Suspensionen vorliegen, die unter Verwendung von annehmbaren Verdünnungsmitteln auf herkommliche Weise hergestellt worden sind.

Für die bukkale Verabreichung kann das Arzneimittel die Form von Tabletten oder Briefchen einnehmen, die in herkömmlicher Weise formuliert worden sind.

Die erfindungsgemäßen Verbindungen können für die parenterale Verabreichung durch Bolusinjektion oder kontinuierliche Infusion formuliert werden. Formulierungen zur Injektion können in Dosiseinheitsform als Ampullen oder in Mehrfachdosenbehältern mit zugesetztem Konservierungsmittel vorliegen.

Die Arzneimittel können solche Formen, wie Suspensionen, Lösungsen oder Emulsionen in öligen oder wäßrigen Trägern, einnehmen, und sie können Formulierungshilfsmittel, wie Suspendierungs-, Stabilisierungs- und/oder Dispergierungsmittel, enthalten.

Alternativ kann der Wirkstoff auch in Pulverform zur Rekonstitution mit einem geeigneten Träger, zum Beispiel sterilem, pyrogenfreim Wasser, vor dem Gebrauch vorliegen.

Die erfindungsgemäßen Verbindungen können auch für rektale Zubereitungen, zum Beispiel Suppositorien oder Retentionsinläufe, formuliert werden, die zum Beispiel herkömmliche Suppositoriengrundlagen, wie Kakaobutter oder andere Glyceride, enthalten.

Zur topischen Anwendung können die erfindungsgemäßen Verbindungen als Salben, Cremes, Gels, Lotionen, Pulver oder Sprays in herkömmlicher Weise formuliert werden.

Für die orale Verabreichung ist eine geeignete Tagesdosis an erfindungsgemäßen Verbindungen 1 bis 4 Dosen bis insgesamt 5 mg bis 1 g/Tag, je nach Zustand des Patienten. Im Einzelfall kann es gegebenenfalls erforderlich sein, von den genannten Mengen abzuweichen, und zwar in Abhängigkeit vom individuellen Verhalten gegenüber dem Wirkstoff bzw. der Art seiner Formulierung und dem Zeitpukt bzw. Intervall, zu welchem die Verabeichung erfolgt. So gibt es zum Beispiel Fälle, wo mit weniger als der oben genannten Mindestmenge ausgekommen werden kann, während in anderen Fällen die genannte obere Grenze überschritten werden muß.

Die erfindungsgemäßen Verbindungen zeichnen sich durch eine neuartige, bisher nicht bekannte und beschriebene pharmakologische Gesamtaktivität aus.

Die erfindungsgemäße neue Strukturklasse zeigt sowohl eine $H_1$-antagonistische, als auch eine $H_2$-agonistische Wirkkomponente.

Dies zeigen die folgenden pharmakologischen Ergebnisse. Eine anerkannte Methode zur Bestimmung $H_1$-antagonistischer Wirksamkeit ist die Ermittlung der $pA_2$-Werte in vitro (ARUNLAKSHANA, O. und SCHILD, H. O. (1959) Some quantitative uses of drug antagonists — Br. J. Pharmacol. Chemother. *14*, 48—58).

Zur Bestimung der $H_2$-agonistischen Aktivität ($pD_2$-Werte) wird die Methode nach VAN ROSSUM, J. M. (1963) Cumulative dose-response curves, II. Technique for the making of dose-response curves in isolated organs and the evaluation of drug parameters. Arch. Int. Pharmacodyn. Ther. *143*, 299—307, herangezogen.

Pharmakologische Daten
(ermittelt am isolierten Atrium bzw. Ileum des Meerschweinchens)

| | $PD_2$-Wert (Atrium) | $PA_2$-Wert (Ileum) |
|---|---|---|
| Beispiel 10 | 6,05 | 6,95 |
| Beispiel 25 | 6,54 | 5,64 |
| Beispiel 43 | 7,17 | 7,20 |
| Beispiel 70 | 7,29 | 5,91 |
| Beispiel 81 | 7,40 | 7,28 |

Beispiel 1

Herstellung der Vorstufen

a) N-Benzoyl-diphenylimidocarbonat

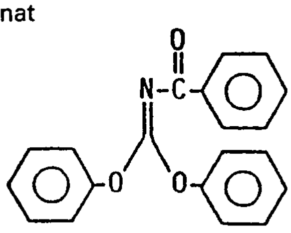

21,0 g (104 mmol) Benzoylisocyaniddichlorid und 20,5 g (218 mmol) Phenol werden in Ethylacetat gelöst und unter Kühlung tropfenweise mit 45 ml Pyridin versetzt. Nach 30 Min. wird der Ansatz i. Vak. eingedampft, der Rückstand mit Benzol gerührt und das ausgefallene Pyridiniumchlorid abfiltriert. Nach dem Abdampfen des Benzols i. Vak. bleibt ein braunes Öl zurück, das bei der Aufbewahrung im Kühlschrank durchkristallisiert. Das rohe Kristallisat wird mehrfach bei Raumtemperatur mit Ether ausgerührt und jeweils der unlösliche Rückstand abfiltriert. Die vereinigten Etherextrakte werden i. Vak. eingeengt und zur Kristallisation im Kühlschrank aufbewahrt. Ausbeute: 23,7 g (72%) farblose Nadeln, die nach Umkristallisation aus Ether bei 108°C schmelzen.

IR (KBr): 1710 (C=0), 1645 $cm^{-1}$

| | | | | |
|---|---|---|---|---|
| $C_{20}H_{15}NO_3$ (317,3) | Ber.: | C 75,70 | H 4,76 | N 4,41 |
| | Gef.: | C 75,72 | H 4,70 | N 4,47 |

MS: m/z (rel. Int. [%]) = 224 ($[M-93]^+$, 24), 105 [$(C_6H_5CO]^+$, 100), 94 [$(C_6H_5OH]^+$, 9), 77 ($[C_6H_5]^+$, 83).

$^1H$-NMR-Daten: ($CDCl_3$, TMS als interner Standard): δ = 7,2—7,55 (m) 13H, 7,93 (m) 2H, ppm.

b) N-Benzoyl-O-phenyl-N'[3-(3-piperidinomethyl-phenoxy)propyl]isoharnstoff

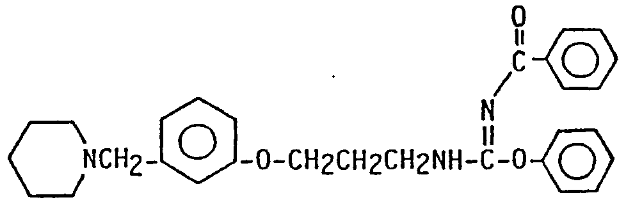

2,48 g (10 mmol) 3-(3-Piperidinomethyl-phenoxy)propylamin und 3,17 g (10 mmol) N-Benzoyl-diphenylimidocarbonat werden in 20 ml Ether 1 h bei Raumtemperatur gerührt. Der Reaktionsansatz wird i. Vak. eingedampft, der Rückstand in Methanol gelöst und bis zur Trübung tropfenweise mit Wasser versetzt. Bei der Aufbewahrung im Kühlschrank kristallisieren 4,40 g (93%) N-Benzoyl-O-phenyl-N'-[3-(3-piperidino-methyl-phenoxy)propyl]isoharnstoff als farblose Nadeln vom Schmp. 67°C aus.

$C_{29}H_{33}N_3O_3$ (471,6) Ber.: C 73,86 H 7,05 N 8,91
Gef.: C 73,82 H 7,15 N 8,89

MS: m/z (rel. Int. [%]) = 471 ($M^+$, 7), 388 ($(M-93)^+$, 35), 105 [$(C_6H_5CO]^+$, 100), 94 ($[C_6H_5OH]^+$, 45), 84 ($[C_5H_{10}N]^+$, 40), 77 ($[C_6H_5]^+$, 55).

IR (KBr): 1640 (C=0) $cm^{-1}$

$^1$H-NMR-Daten: ($CDCl_3$, TMS als interner Standard): δ = 1,2—1,8 (m) 6H, 2,20 (m) 2H, 2,37 (m) 4H, 3,40 (s) 2H, 3,78 (dt) 2H, 4,13 (t) 2H, 6,60—7,55 (m) 12H, 7,87 (m) 2H, 10,25 (t, breit) 1H, austauschbar mit $D_2O$, ppm.

N-Benzoyl-N'-(2-(imidazol-4-yl)ethyl]-N''-[3-(3-piperidinomethylphenoxy)propyl]guanidine

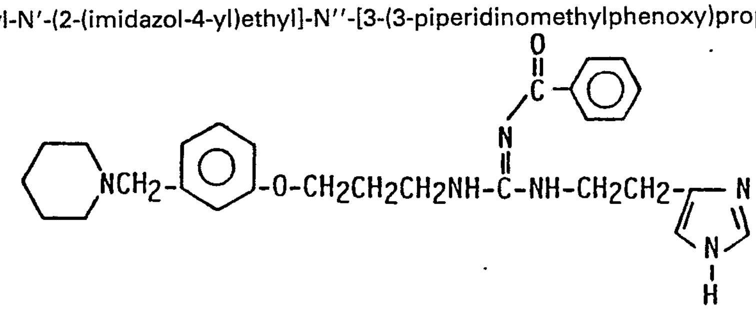

Aus 0,92 g (5 mmol) Histamindihydrochlorid wird mit 10 mmol Natriumethylat in 100 ml Ethanol die Base freigesetzt, das ausgefallene Natriumchlorid abfiltriert und das Filtrat im Vakuum auf etwa 20 ml eingeengt. Nach Zugabe von 2,36 g (5 mmol) N-Benzoyl-O-phenyl-N'-[3-(3-piperidinomethyl-phenoxy)-propyl]isoharnstoff wird 1 h unter Rückfluß erhitzt, i. Vak. eingedampft und der Rückstand in heißem Acetonitril gelöst. Beim Erkalten kristallisieren 1,2 g (49%) N-Benozyl-N'-[2-(imidazol-4-yl)ethyl]-N''-[3-(3-piperidinomethyl-phenoxy)propyl]guanidin vom Schmp. 136°C aus.

Die Verwendung von Pyridin, Acetonitril oder tert. Butanol anstelle von Ethanol als Reaktionsmedium führt bei gleicher Reaktionszeit zu vergleichbaren Ausbeuten.

$C_{28}H_{36}N_6O_2$ (488,6) Ber.: C 68,83 H 7,43 N 17,21
Gef.: C 69,07 H 7,61 N 17,29

MS: m/z (rel. Int. [%]) = 488 ($M^+$, 11), 105 (100, 95 (33), 84 (57), 77 (75).

$^1$H-NMR-Daten: ($d_6$-DMSO, TMS als interner Standard): δ = 1,15—1,85 (m) 6H, 2,00 (m) 2H, 2,28 (m) 4H, 2,77 (t) 2H, 3,32 (s) 2H, 3,0—3,8 (m) 4H, 4,00 (t) 2H, 6,55—7,55 (m) 9H, 8,02 (m) 2H, ppm.

## Beispiel 2

N-Benzoyl-N'-[2-(5-methylimidazol-4-yl)ethyl]-N''-[3-(3-piperidinomethyl-phenoxy)propyl]guanidin

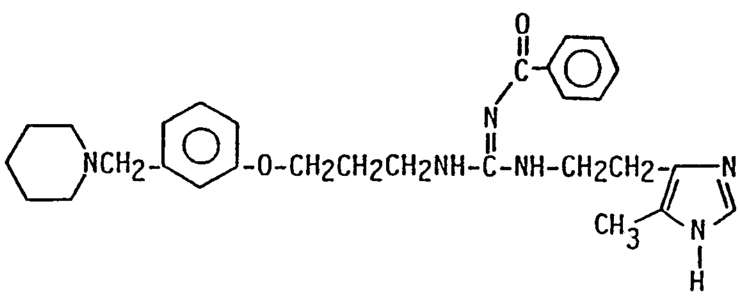

5 mmol 5-Methylhistamin, dargestellt aus 0,99 g Dihydrochlorid mit 10 mmol Natriumethylat in Ethanol, werden mit 2,36 g (5 mmol) N-Benzoyl-O-phenyl-N'-[3-(3-piperidinomethyl-phenoxy)propyl]-isoharnstoff (Beispiel 1b) (1 h in 20 ml Acetonitril unter Rückfluß erhitzt. Nach dem Eindampfen i. Vak. wird

das Reaktionsprodukt durch präparative Schichtchromatographie (Kieselgel 60 $PF_{254}$ gipshaltig, Fließmittel: Ethylactat/methanol. Ammoniak 90 + 10) isoliert. Das Eluat wird i. Vak. eingedampft, der Rückstand in wenig Acetonitril gelöst und mit Ethylacetat versetzt. Bei der Aufbewahrung in Kühlschrank (−20°C) kristallisieren 0,25 g (10%) N-Benzoyl-N'-[2-(5-methylimidazol-4-yl)ethyl]-N''-[3-(3-piperidino-methyl-phenoxy)propyl)guanidin vom Schmp. 118—120°C aus.

$C_{29}H_{38}N_6O_2$ (502,7) Ber.: C 69,29 H 7,62 N 16,72
Gef.: C 69,07 H 7,77 N 16,61

MS: m/z (rel. Int. [%]) = 502 ($M^+$, 1), 109 (6), 105 (8), 95 (25), 84 (42), 77 (7), 44 (100).

$^1$H-NMR-Daten: ($d_6$-DMSO, TMS als interner Standard): δ = 1,15—1,7 (m) 6H, 2,00 (m) 2H, 2,10 (s) 3H, 2,27 (m) 4H, 2,70 (t) 2H, 3,33(s) 2H, 3,0—3,8 (m) 4H, 4,00 (t) 2H, 6,55—7,6 (m) 8H, 8,02 (m) 2H, ppm.

## Beispiel 3

N-Benzoyl-N'-[3-(imidazol-4-yl)propyl]-N''-[3-(3-piperidinomethylphenoxy)propyl]guanidin

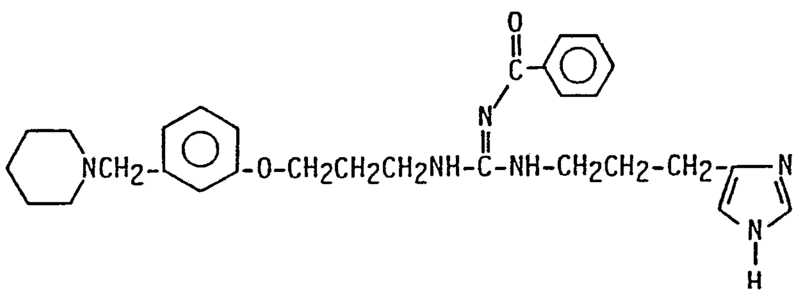

Aus 0,99 g (5 mmol) 3-(Imidazol-4-yl)propylamin-dihydrochlorid wird mit 10 mmol Natriumethylat in Ethanol die Base freigesetzt, ausgefallenes Natriumchlorid abfiltriert, das Filtrat i. Vak. eingedampft und in Pyridin aufgenommen. Nach Zusatz von 2,36 g (5 mmol) N-Benzoyl-O-phenyl-N'-[3-(3-piperidinomethyl-phenoxy)propyl]isoharnstoff (Beispiel 1, Herstellung b) wird 1 h unter Rückfluß erhitzt, anschießend i. Vak. eingedampft und das Produkt durch präparative Schichtchromatographie (vgl. Beispiel 2) isoliert. Das i. Vak. eingedampfte Eluat wird in heißem Acetonitril gelöst und zur Kristallisation stehengelassen. Ausbeute 1,4 g (56%) farblose Nadeln vom Schmp. 115°C.

$C_{29}H_{38}N_6O_2$ (502,7) Ber.: C 69,29 H 7,62 N 16,72
Gef.: C 69,47 H 7,72 N 16,76

MS: m/z (rel. Int. [%]) = 502 ($M^+$, 24), 109 (50), 105 (100), 84 (25), 77 (39).

IR (KBr): 1600 (C=0) $cm^{-1}$.

$^1$H-NMR-Daten: ($d_6$-DMSO, TMS als interner Standard): δ = 1,15—2,4 (m) 14H, 2,58 (t) 2H, 3,32 (s) 2H, 2,75—3,8 (m) 4H, 4,03 (t) 2H, 6,55—7,55 (m) 9H, 8,06 (m) 2H, ppm.

## Beispiel 4

N-[3-(Imidazol-4-yl)propyl]-N'-[3-(3-piperidinomethyl-phenoxy)propyl]guanidin

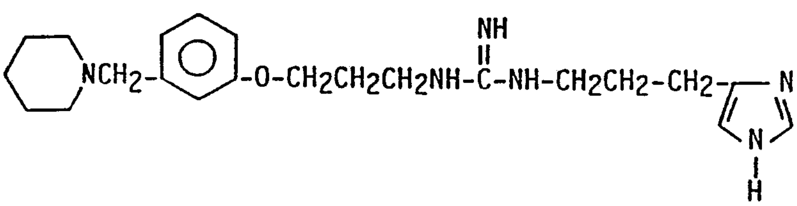

0.90 g (1,79 mmol) N-Benzoyl-N'-[3-(imidazol-4-yl)propyl-N''-[3-(3-piperidinomethyl-phenoxy)propyl]-guanidin (Beispiel 3) werden 7 h in 45 ml 20-proz. Salzsäure unter Rückfluß erhitzt. Nach dem Erkalten des Reaktionsansatzes wird die ausgefallene Benzoesäure abfiltriert, das Filtrat zudem dreimal mit Ether extrahiert und die wäßrige Phase i. Vak. zur Trockne eingedampft. Man erhält 0,8 g (88%) trockenen Schaum.

$C_{22}H_{34}N_6O \times 3\ HCl$ (507,9)

MS: m/z (rel. Int. [%]) = 398 ($M^+$, 3), 109 (39), 95 (50), 84 (67).

$^1$H-NMR-Daten: ($d_6$-DMSO, TMS als interner Standard): δ = 1,3—2,3 (m) 8H, 2,4—3,65 (m) 12H, 4,08 (t) 2H, 4,18 (s) 2H, 6,8—7,5 (m) 5H, 7,6 (s, breit) 2H, austauschbar mit $D_2O$, 7,75—8,25 2H, austauschbar mit $D_2O$, 8,93 (d) 1H, ppm.

Beispiel 5

N-[2-(Imidazol-4-yl)ethyl]-N'-[3-(3-piperidinomethyl-phenoxy)propyl]guanidin

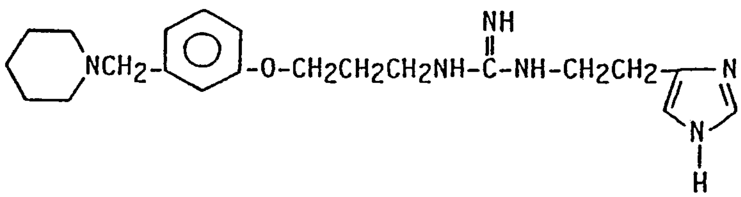

1,4g (3,4 mmol) N-Cyano-N'-[2-(imidazol-4-yl)ethyl]-N''-[3-(3-piperidinomethyl-phenoxy)propyl]-guanidin werden mit 50 ml konzentrierter Salzsäure 4 h unter Rückfluß erhitzt. Anschließend wird der Reaktionsansatz i. Vak. zur Trockne eingedampft und der Rückstand dreimal mit wasserfreiem Aceton ausgerührt. Die vereinigten Extrakte werden i. Vak. eingedampft und ergeben 1,5 g (89%) des stark hygroskopischen Trihydrochlorids, das bei 100°C sintert. Das Tripikrat sintert bei 95—100°C.

$C_{21}H_{32}N_6O \times 3\ C_6H_3N_3O_7$ (1071,8) Ber.: C 43,70 H 3,86 N 19,60

Gef.: C 43,65 H 3,71 N 19,43

$C_{21}H_{32}N_6O \times HCl$ (493,9)

MS: m/z (rel. Int. [%]) = 384 ($M^+$, 40), 302 (82), 107 (100), 95 (21), 84 (98).

$^1$H-NMR-Daten: ($d_6$-DMSO, H-D-Austauch mit $CF_3COOD$; TMS als interner Standard): δ = 1,2—2,4 (m) 8H, 2,6—3,9 (m) 10H, 4,13 (t) 2H, 4,27 (s) 2H, 6,85—7,7 (m) 5H, 9,00 (d) 1H, ppm.

Beispiel 6

N-Benzoyl-N'-[2-hydroxy-3-(3-piperidinomethyl-phenoxy)propyl]-N''-[3-(imidazol-4-yl)-propyl]guanidin

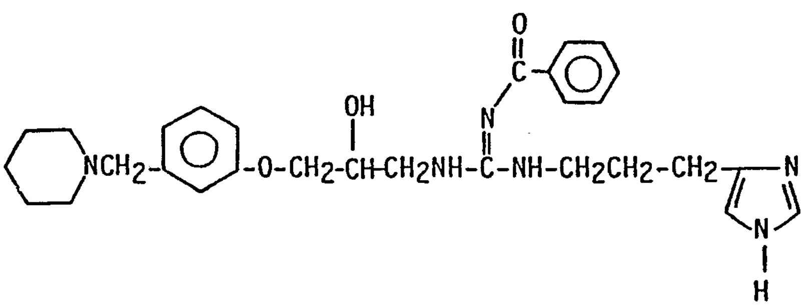

Methode A

1,32 g (5 mmol) 2-Hydroxy-3-(3-piperidinomethyl-phenoxy)propylamin werden mit 1,59 g (5 mmol) N-Benzoyl-diphenylimidocarbonat in 30 ml Acetonitril 40 Min. bei Raumtemperatur gerührt. Nach Zugabe von 0,63 g (5 mmol) 3-(Imidazol-4-yl)propylamin wird 1 h unter Rückfluß erhitzt und anschließend das Reaktionsprodukt durch präparative Schichtchromatographie isoliert (Kieselgel 60 $PF_{254}$ gipshaltig, Fließmittel: Chloroform/methanol. Ammoniak, 94 + 6). Nach dem Eindampfen des Eluats erhält man durch Kristallisation aus Ethylacetat 0,62 g (24%) farblose Kristalle vom Schmp. 75—77°C.

$C_{29}H_{38}N_6O_3$ (518,7) Ber.: C 67,16 H 7,39 N 16,20

Gef.: C 66,89 H 7,50 N 15,91

$^1$H-NMR-Daten: ($d_6$-DMSO, TMS als interner Standard): δ = 1,15—1,7 (m) 6H, 1,83 (m) 2H, 2,27 (m) 4H, 2,57 (m) 2H, 3,32 (s) 2H, 2,9—3,8 (m) 4H, 3,95 (m) 3H, 5,5 (m) 1H, austauschbar mit $D_2O$, 6,5—7,5 (m) 11H, 2H austauschbar mit $D_2O$, 8,03 (m) 2H, 10,2 (m, br.) 1H, austauschbar mit $D_2O$, ppm.

Methode B

Herstellung der Vorstufe

2-Benzoylimino-5-[(3-piperidinomethyl-phenoxy)methyl]oxazolidin

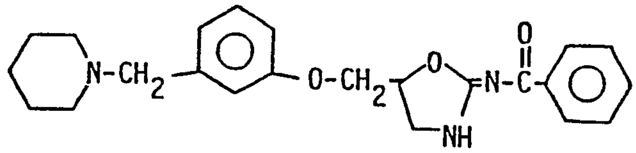

2,64 g (10 mmol) 2-Hydroxy-3-(3-piperidinomethyl-phenoxy)propylamin in 10 ml Methylenchlorid werden bei 0 bis −10°C in eine Lösung von 2,02 g (10 mmol) Benzoylisocyaniddichlorid in 20 ml Methylenchlorid getropft. Nach tropfenweiser Zugabe einer Mischung von 1,5 ml Triethylamin und 10 ml Methylenchlorid wird 30 Min. gerührt, anschließend das entstandene Triethylammoniumchlorid durch Waschen mit

Wasser entfernt, die organische Phase über Natriumsulfat getrocknet und i. Vak. eingedampft. Der Rückstand wird aus Methanol umkristallisiert.

Ausb.: 3,5 g (89%) farblose Nadeln vom Schmp. 126°C.

$C_{23}H_{27}N_3O_3$ (393,5) Ber.: C 70,21 H 6,92 N 10,68

Gef.: C 70,16 H 6,97 N 10,81

$^1$H-NMR-Daten: ($d_6$-DMSO, TMS als interner Standard): δ = 1,38 (m) 2H, 1,48 (m) 4H, 2,30 (m) 4H, 3,38 (s) 2H, 3,69 (dd) 1H, 3,95 (dd) 1H, 4,20 (dd) 1H, 4,29 (dd) 1H, 5,13 (m) 1H, 6,8—7,0 (m) 3H, 7,24 (m) 1H, 7,4—7,6 (m) 3H, 8,09 (m) 2H, 9,67 (s) 1H, austauschbar mit $D_2O$, ppm.

N-Benzoyl-N'-[2-hydroxy-3-(3-piperidinomethyl-phenoxy)propyl]-N''-[3-(imidazol-4-yl)propyl]guanidin

1,97 g (5 mmol) 2-Benzoylimino-5-[(3-piperidinomethyl-phenoxy)methyl]oxazolidin werden mit 0,69 g (5,5 mmol) 3-(Imidazol-4-yl)propylamin in 30 ml Pyridin 8 h unter Rückfluß erhitzt. Das Lösungsmittel wird i. Vak. abdestilliert und das Reaktionsprodukt analog Methode A isoliert und gereinigt.

Ausb. 1,1 g (42%)

## Beispiel 7

N-[2-Hydroxy-3-(3-piperidinomethyl-phenoxy)propyl]-N'-[3-(imidazol-4-yl)propyl]guanidin

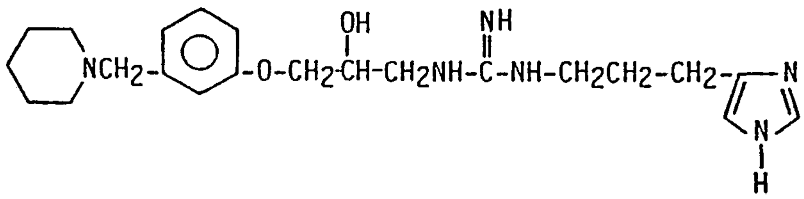

0.4 g (0,77 mmol) N-Benzoyl-N'-[2-hydroxy-3-(piperidinomethyl-phenoxy)propyl]-N''-[3-(imidazol-4-yl)propyl]guanidin (Beispiel 6) werden in 45 ml 15-proz. Salzsäure 6 h unter Rückfluß erhitzt. Der Reaktionsansatz wird analog Beispiel 4 aufgearbeitet. Nach dem Eindampfen der wäßrigen Lösung wird der Rückstand aus Isopropylalkohol/Ether kristallisiert. Man erhält nach Trocknung i. Vak. bei Raumtemperatur 0,42 g (93%) des hygroskopischen Trihydrochlorids, das 1 Mol Isopropylalkohol enthält und bei 75°C sintert.

$C_{22}H_{34}N_6O_2 \times 3\ HCl \times C_3H_8O$ (584,0)

MS (FAB-Methode): m/z (rel. Int. [%]) = 415 [(M + H)$^+$, 91), 331 (13), 265 (22), 192 (37), 109 (100), 84 (87).

$^1$H-NMR-Daten: ($d_6$-DMSO, TMS als interner Standard): δ = 1,03 (d) 6H, Isopropylalkohol, 1,15—2,2 (m) 2,3—4,3 (m) 16H, 5,6 (m, breit) 2H, austauschbar mit $D_2O$, 6,7—8,1 (m), 3H austauschbar mit $D_2O$, 8,96 (d) 1H, ppm.

## Beispiel 8

N$^1$-[3-[N-(5-Methyl-pyrid-2-yl)-methylamino]propyl]-N$^2$-(1H-imidazol-4-yl)ethyl]guanidin-trihydrochlorid

Herstellung der Vostufen

a) O-Phenyl-N$^1$-cyano-N$^2$-[3-[N-(5-methyl-pyrid-2-yl)-methylamino]propyl]-isoharnstoff

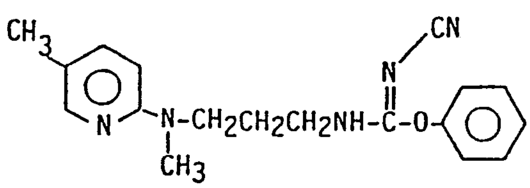

3,60 g (20 mmol) N-Methyl-N-(5-methyl-pyrid-2-yl)-propan-1,3-diamin und 4,76 g (20 mmol) Diphenylcyanimidocarbonat werden in 30 ml i-Propanol 4 h bei Raumteperatur gerührt. Nach Abdampfen des Lösungsmittels i. Vak. wird der Rückstand in 200 ml Methylenchlorid aufgenommen und zweimal mit 100 ml 1 N Natronlauge extrahiert. Nach Trocknen über Natriumsulfat wird die organ. Phase i. Vak. eingedampft. Das erhaltene Öl kristallisiert nach kurzer Zeit zu farblosen Kristallen vom Schmp. 185°C (Zers.)

Ausbeute: 4,19 g (65%)

$C_{18}H_{21}N_5O$ (323,4)

RF: 0,45 ($CH_2Cl_2/CH_3OH$ 98:2)

b) $N^1$-Cyano-$N^2$-[3-[N-(5-methyl-pyrid-2-yl)-methylamino]-propyl]-$N^3$-[2-(1H-imidazol-4-yl)ethyl]-guanidin

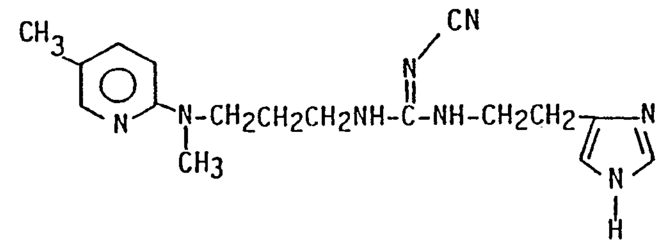

3,00 g (9,3 mmol) O-Phenyl-$N^1$-cyano-$N^2$-[3-[N-(5-methyl-pyrid-2-yl)methylamino]propyl]-isoharnstoff und 1,03 g (9,3 mmol) Histamin werden in 60 ml i-Propanol 10 h unter Rückfluß gekocht. Nach Abdampfen des Lösungsmittels im Vakuum wird der Rückstand an Kieselgel mit Essigester/Ethanol (60:40) chromatographiert. Die Hauptfraktion ergibt nach Abdampfen des Lösungsmittels, 1,76 g (56%) der Titelverbindung. Farbloser Feststoff vom Schmp. 152—153°C (aus Chloroform).

$C_{17}H_{24}N_8$ (340,4)

Rf: 0,41 (EtOAc/EtOH 60:40)

$^1$H-NMR-Daten: ($CD_3OD$, TMS als interner Standard): δ = 1,73 (m) 2H, 2,16 (s) 3H, 2,84 (t) 2H, 2,96 (s) 3H,
3,18 (t) 2H, 3,36—3,69 (m) 4H, 5,0 (breit) 3H, 6,56 (d) 1H, 6,90 (s) 1H, 7,37 (dd) 1H, 7,64 (s) 1H, 7,99 (d) 1H ppm.

$N^1$-[3-[N-(5-Methyl-pyrid-2-yl)-methylamino]propyl]-$N^2$-[2-[1H-imidazol-4-yl)ethyl]guanidin-trihydrochlorid

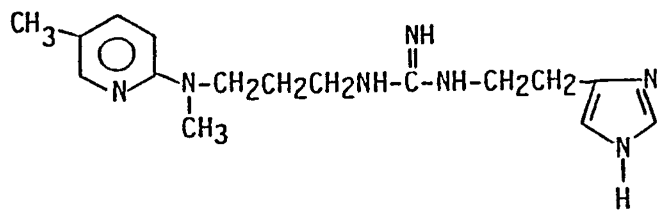

2,0 g (5,9 mmol) $N^1$-Cyano-$N^2$-[3-[N-(5-methyl-pyrid-2-yl)-methylamino]propyl]-$N^3$-[2-(1H-imidazol-4-yl)ethyl]-guanidin werden in 20 ml 4 N Salzsäure 9 h gekocht. Die Lösung wird i. Vak. eingedampft, der verbleibende Rückstand in 10 ml Methanol aufgenommen und mit 3,3 ml 5,5 N Natirummethylat-Lösung 10 Min gerührt. Nach Absaugen des Niederschlags wird nochmals i. Vak. eingedampft. Das erhaltene Rohprodukt wird mit Essigester/Methanol (1:1) an Aluminiumoxid (neutral) gereinigt. Die Hauptfraktion ergibt nach Eindampfen 0,88 g eines Farblosen Öls, das in 20 ml Wasser gelöst wird. Nach Zugabe von 6,1 ml 2 N Salzsäure wird die Lösung i. Vak. Eingedampft und der Rückstand im Hochvakuum getrocknet. Man erhält 1,16 g (47%) der Titelverbindung als farblosen, hygroskopischen Feststoff.

$C_{16}H_{28}C_3N_7$ (424,8)

Rf: 0,2 (Base, Alox, EtOAc/MeOH 1:1)

$^1$H-NMR-Daten: ($CD_3OD$, TMS als interner Standard): δ = 2,01 (m) 2H, 2,29 (s) 3H, 3,10 (t) 2H, 3,34 (s) 3H,
3,40 (t) 2H, 3,52—3,94 (m) 4H, 4,8 (breit) 7H, 7,38 (d) 1H, 7,60 (s) 1H, 7,86 (d) 1H, 7,99 (dd) 1H, 9,00 (s) 1H, ppm.

Beispiel 9

$N^1$-Benzoyl-$N^2$-[3-[N-(5-methyl-pyridin-2-yl)-methylamino]propyl]-$N^2$-[3-(1H-imidazol-4-yl)propyl]-guanidin

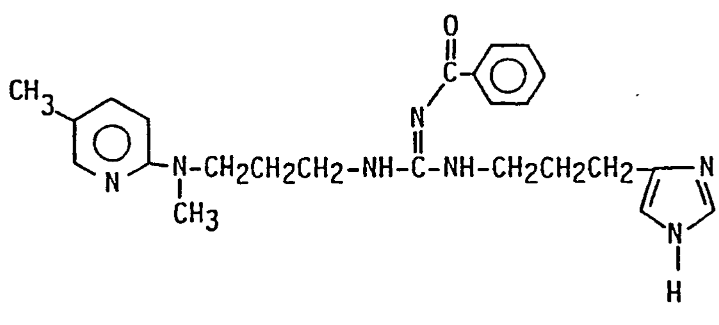

3,48 g (10 mmol) $N^1$-Benzoyl-$N^2$-[3-(4-imidazolyl)propyl]-O-phenylisoharnstoff und 1,79 g (10 mmol) N-Methyl-N-(5-methyl-pyridin-2-yl) 1,3-propandiamin werden in 50 ml Ethanol 20 Stunden unter Rückfluß gekocht. Der nach Abdampfen des Lösungsmittels erhaltene Rückstand wird an Kieselgel mit Essigester/

Ethanol (80:20) chromatographiert. Die Hauptfraktion ergibt nach Abdampfen des Lösungsmittels 3,76 g (76%) eines blaßgelben Öls.

$C_{24}H_{31}N_7O$ (433,56)

$^1$H-NMR-Daten: ($CD_3OD$, TMS als interner Standard): δ = 1,71—2,20 (m) 4H, 2,17 (s) 3H, 2,77 (t) 2H, 3,07 (s) 3H, 3,35—3,61 (m) 4H, 3,71 (t) 2H, 5,0 (breit) 3H, 6,80 (d) 1H, 7,10 (s) 1H, 7,50—7,82 (m) 4H, 7,86 (s) 1H, 8,18 (d) 1H, 8,39—8,53 (m) 2H ppm.

Beispiel 10

$N^1$-[3-[N-(5-Methyl-pyridin-2-yl)-methylamino]propyl]-$N^2$-[3-(1H-imidazol-4-yl)propyl]guanidin-trihydrochlorid

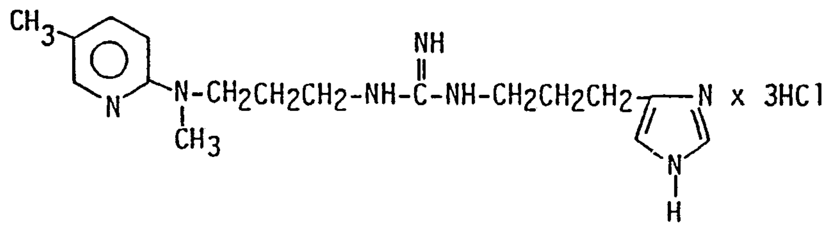

Aus 1,38 g (3,2 mmol) $N^1$-Benzoyl-$N^2$-[3-[(N-5-methyl-pyridin-2-yl)-methylamino]propyl]-$N^2$-[3-(1H-imidazol-4-yl)propyl]-guanidin (Beispiel 9) und 20 ml konz. Salzsäure werden 1,41 g eines braunen Feststoffs erhalten, der nach Überführung in die Base mit Essigester/Methanol (1:1) als Laufmittel an Aluminiumoxid chromatographisch gereinigt wird. Nach Rückführung in das Trihydrochlorid erhält man 0,64 g (46%) eines farblosen, amorphen Feststoffs, der hygroskopisch ist.

$C_{17}H_{30}Cl_3N_7$ (438,83)

$^1$H-NMR-Daten: ($CD_3OD$, TMS als interner Standard): δ = 1,82—2,23 (m) 4H, 2,29 (s) 3H, 2,89 (t) 2H, 3,20—3,55 (m) 4H, 3,30 (s) 3H, 3,80 (t) 2H, 4,8 (breit) 7H, 7,36 (d) 1H, 7,48 (s) 1H, 7,84 (m) 1H, 7,99 (dd) 1H, 8,92 (d) 1H, ppm.

Beispiel 11

$N^1$-Benzoyl-$N^2$-[3-(1H-imidazol-4-yl)propyl]-$N^3$-[2-(pyridin-2-yl-amino)ethyl]-guanidin

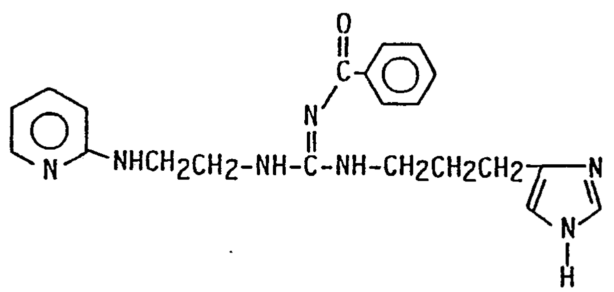

Aus 0,69 g (5 mmol) N-(2-Pydridinyl)-ethylendiamin und 1,74 g (5 mmol) $N^1$-Benzoyl-$N^2$-[3-(1H-imidazol-4-yl)propyl]-O-phenyl-isoharnstoff werden nach 24-stündigem Kochen in 30 ml Ethanol und chromatographischer Reinigung des Rohprodukts (Kieselgel, Essigester/Ethanol 80:20) analog zu Beispiel 16 1,50 g (76%) des Benzoylguanidins in Form eines farblosen Öls erhalten.

$C_{21}H_{25}N_{70}$ (391,47)

$^1$H-NMR-Daten: ($CD_3OD$, TMS als interner Standard): δ = 1,77—2,12 (m) 2H, 2,65 (t) 2H, 3,29 (t) 2H, 3,46—3,89 (m) 4H, 4,85 (breit) 4H, austauschbar mit $D_2O$, 6,45—7,62 (m) 8 H, 7,98—8,30 (m) 3H ppm.

Beispiel 12

$N^1$-[3-(1H-Imidazol-4-yl)propyl]-$N^2$-[2-(pyridin-2-yl-amino)ethyl]-guanidin-trihydrochlorid

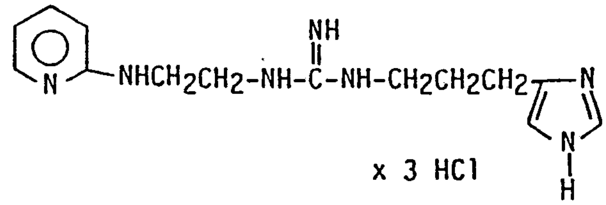

Aus 1,21 g (3,1 mmol) $N^1$-Benzoyl-$N^2$-[3-(1H-imidazol-4-yl)propyl]-$N^3$-[2-pyridin-2-yl-amino)ethyl]-guanidin und 20 ml konz. Salzsäure werden 0,93 g (76%) eines farblosen, hygroskopischen Feststoffes erhalten.

$C_{14}H_{24}Cl_3N_7$ (396,75)

$^1$H-NMR-Daten: ($CD_3OD$, TMS als interner Standard): δ = 1,80—2,21 (m) 2H, 2,69—3,00 (m) 2H, 3,37 (t) 2H, 3,57—3,83 (m) 4H, 4,8 (breit) 8H, austauschbar mit $D_2O$, 6,96 (t) 1H, 7,22 (d) 1H, 7,44 (s) 1H, 7,83—8,16 (m) 2H, 8,87 (s) 1H ppm.

Beispiel 13

N-Benzoyl-N'-[3-(imidazol-4-yl)propyl]-N''-(3-phenoxypropyl)guanidin

Herstellung der Vorstufe

N-Benzoyl-N'-(3-phenoxypropyl)-O-phenyl-isoharnstoff

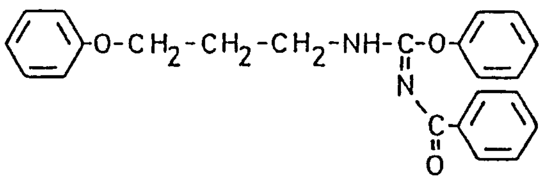

1,0 g (6,6 mmol) 3-Phenoxypropylamin und 2,09 g (6,6 mmol) N-Benzoyl-diphenylimidocarbonat werden 15 min bei Raumtemperatur in 30 ml Ether gerührt. Beim Einengen i. Vak. fällt das Reaktionsprodukt teilweise aus. Davon werden 0,2 g für analytische Zwecke entnommen, der gesamte restliche Reaktionsansatz wird wie unten beschrieben weiter umgesetzt. N-Benzoyl-N'-(3-phenoxypropyl)-O-phenylisoharnstoff kristallisiert aus Ether in farblosen Nadeln vom Schmp. 75°C.

$C_{23}H_{22}N_2O_3$ (374,4) Ber.: C 73,78 H 5,92 N 7,48
Gef.: C 73,63 H 5,85 N 7,44

$^1$H-NMR-Daten: ($d_6$-DMSO, TMS als interner Standard) δ = 2,13 (m) 2H, 3,70 (dt) 2H, 4,10 (t) 2H, 6,7—7,65 (m) 13H, 7,78 (m) 2H, 10,07 (t) 1H, austauschbar mit $D_2O$, ppm.

N-Benzoyl-N'-[3-(imidazol-4-yl)propyl]-N''-(3-phenoxypropyl)guanidin

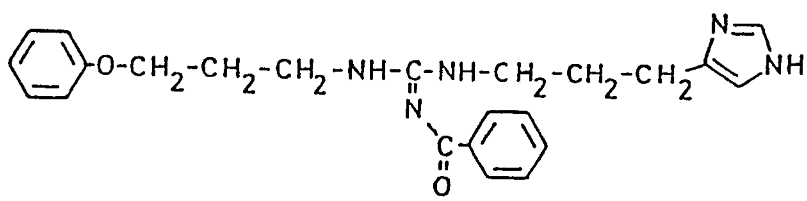

Die zuvor erhaltene Lösung von 6,1 mmol N-Benzoyl-N'-(3-phenoxypropyl)-O-phenylisoharnstoff wird i. Vak. eingedampft, der Rückstand in Acetonitril gelöst und nach Zugabe von 0,8 g (6,4 mmol) 3-(Imidazol-4-yl)propylamin 3 h unter Rückfluß erhitzt. Anschliebend wird i. Vak. eingedampft, der Rückstand in verdünnter Salzsäure aufgenommen und die wäßrige Lösung dreimal mit Ether extrahiert. Nach dem Alkalisieren mit Natronlauge wird das Reaktionsprodukt mit Methylenchlorid aus der wäßrigen Phase extrahiert und aus dem eingedampften Extrakt mit Ethylacetat zur Kristallisation gebracht. Man erhält 0,95 g (38%) farblosen Feststoff, der nach Umkristallisation aus Ethanol/Wasser bei 149°C schmilzt.

$C_{23}H_{27}N_5O_2$ (405,5) Ber.: C 68,13 H 6,71 N 17,27
Gef.: C 68,17 H 6,83 N 17,11

$^1$H-NMR-Daten: ($d_6$-DMSO, TMS als interner Standard) δ = 1,75—2,30 (m) 4H, 2,35—2,8 (m) 2H, 3,0—3,75 (m) 4H, 4,07 (t) 2H, 6,7—7,6 (m) 10H, 8,08 (m) 2H, ppm.

Beispiel 14

N-[3-(Imidazol-4-yl)propyl]-N'-(3-phenoxypropyl)guanidin

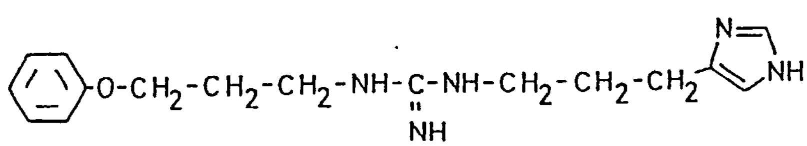

0,42 g (1 mmol) N-Benzoyl-N'-[3-(imidazol-4-yl)propyl]-N''-(3-phenoxypropyl)guanidin werden in 30 ml 20 proz. Salzsäure 5 h unter Rückfluß erhitzt. Die Aufarbeitung des Reaktionsansatzes erfolgt analog Beispiel 4. Man erhält 0,35 g (94%) des Dihydrochlorids als hygroskopischen amorphen Feststoff.

$C_{16}H_{23}N_5O \cdot 2HCl$ (374,3) Molmasse 301 (FAB—MS).

$^1$H-NMR-Daten: ($d_6$-DMSO, TMS als interner Standard) δ = 1,95 (m) 4H, 2,75 (t) 2H, 2,95—3,6 (m) 4H, 4,05 (t) 2H, 6,7—7,5 (m) 6H, 7,5—8,2 (m) 4H, austauschbar mit $D_2O$, 9,00 (d) 1H, ppm.

Beispiel 15

N-Benzoyl-N'-(2-hydroxy-3-phenoxypropyl)-N''-[3-(imidazol-4-yl)propyl]guanidin

Herstellung der Vorstufe

2-Benzoylimino-5-phenoxymethyloxazolidin

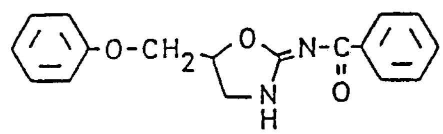

Methode A

1,67 g (10 mmol) 2-hydroxy-3-phenoxypropylamin und 3,17 g (10 mmol) N-Benzoyl-diphenylimidocarbonat werden 5 h bei Raumtemperatur in 20 ml Methyllenchlorid gerührt. Anschließend wird das Lösungsmittel i. Vak. abdestilliert und der Rückstand mit Acetonitril zur Kristallisation gebracht.

Ausb.: 1,8 g (61%) farblose Kristalle vom Schmp. 142°C.

Methode B

1,67 g (10 mmol) 2-Hydroxy-3-phenoxypropylamin in 10 ml Methylenchlorid werden bei 0 bis −10°C in eine Lösung von 2,02 g (10 mmol) Benzoylisocyaniddichlorid in 20 ml Methylenchlorid getropft. Nach tropfenweiser Zugabe einer Mischung von 1,5 ml Triethylamin und 10 ml Methylenchlorid wird 30 min gerührt, anschließend das entstandene Triethylammoniumchlorid durch Waschen mit Wasser entfernt, die organische Phase über Natriumsulfat getrocknet und i. Vak. eingedampf. Der Rückstand wird aus Methanol umkristallisiert.

Ausb.: 2,5 g (84%) farblose Kristalle vom Schmp. 144°C.

| | | | | |
|---|---|---|---|---|
| $C_{17}H_{12}N_2O_3$ (296,3) | Ber.: | C 68,91 | H 5,44 | N 9,45 |
| | Gef.: | C 68,62 | H 5,40 | N 9,59 |

MS: m/z (rel. Int. [%]) = 296 ($M^+$., 17), 105 (23), 85 (93), 83 (100), 58 (75).

$^1$H-NMR-Daten: ($CDCl_3$, TMS als interner Standard) δ = 3,95 (dd) 1H, 4,07 (dd) 1H, 4,25 (d) 2H, 5,11 (m) 1H, 6,91 (m) 2H, 7,03 (m) 1H, 7,2—7,6 (m) 5H, 8,24 (m) 2H, 9,55 (br.) 1H, austauschbar mit $D_2O$, ppm.

N-Benzoyl-N'-(2-hydroxy-3-phenoxypropyl)-N''-[3-(imidazol-4-yl)propyl]guanidin

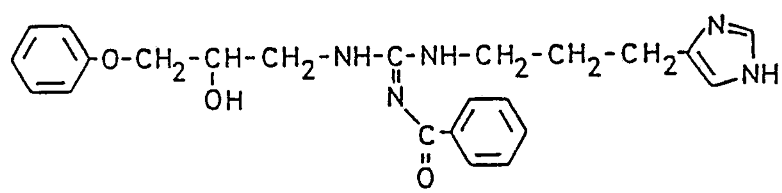

1,48 g (5 mmol) 2-Benzoylimino-5-phenoxymethyloxazolidin werden mit 0,69 g (5,5 mmol) 3-(Imidazol-4-yl) propylamin in einer Mischung aus 40 ml Acetonitril und 10 ml Ethanol 8 h unter Rückfluß erhitzt. Der Reaktionsansatz wird i. Vak. eingedampft und das Produkt durch präparative Schichtchromatographie isoliert und gereinigt (kieselgel 60 $PF_{254}$ gipshaltig; Fließmittel: Chloroform/Methanol, 98 + 2, Ammoniakatmosphäre). Nach Kristallisation aus Acetonitril erhält man 0,8 g (38%) farblose Kristalle von Schmp. 129°C.

| | | | | |
|---|---|---|---|---|
| $C_{23}H_{27}N_5O_3$ (421,5) | Ber.: | C 65,54 | H 6,46 | N 16,62 |
| | Gef.: | C 65,36 | H 6,52 | N 16,41 |

$^1$H-NMR-Daten: ($d_6$-DMSO), TMS als interner Standard) δ = 1,96 (m) 2H, 2,4—2,8 (m) 2H, 3,0—3,7 (m) 4H, 3,7—4,2 (m) 3H, 7,6—6,7 (m) 10H, 8,10 (m) 2H, ppm.

Beispiel 16

N-(2-Hydroxy-3-phenoxypropyl)-N'-[3-(imidazol-4-yl)propyl]guanidin

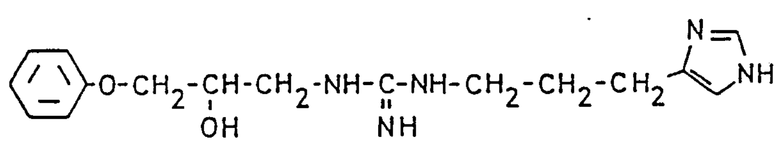

0,5 g (1,2 mmol) N-Benzoyl-N'-(2-hydroxy-3-phenoxypropyl)-N''-[3-(imidazol-4-yl)propyl]guanidin werden in 30 ml 20 proz. Salzsäure 6 h unter Rückfluß erhitzt. Der Reaktionsansatz wird analog Beispiel 4 aufgearbeitet. Das Dihydrochlorid kristallisiert aus Isopropylalkohol/Aceton in farblosen Kristallen vom Schmp. 165—166°C.

Ausbeute 0,45 g (96%). Molmasse 317 (FAB—MS).

| | | | | |
|---|---|---|---|---|
| $C_{16}H_{23}N_5O_2 \cdot 2HCl$ (390,3) | Ber.: | C 49,24 | H 6,46 | N 17,94 |
| | Gef.: | C 48,73 | H 6,64 | N 17,60 |

$^1$H-NMR-Daten: ($d_6$-DMSO, H-D-Austausch mit $CF_3COOD$, TMS als interner Standard) δ = 1,85 (m) 2H, 2,73 (t) 2H, 3,20 (t) 2H, 3,35 (m) 2H, 3,95 (m) 3H, 6,7—7,5 (m) 6H, 8,93 (d) 1H, ppm.

Beispiel 17

N-Benzoyl-N'-[2-hydroxy-3-(1-naphthoxy)propyl]-N''-[3-(imidazol-4-yl)propyl]guanidin

Herstellung der Vorstufe

2-Benzoylimino-5-[(1-naphthoxy)methyl]oxazolidin

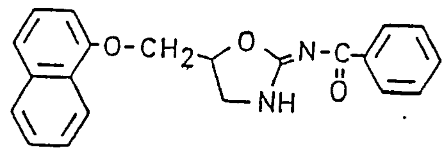

2,17 g (10 mmol) 2-Hydroxy-3-(1-naphthoxy)propylamin und 3,17 g (10 mmol) N-Benzoyldiphenylimidocarbonat werden 5 h bei Raumtemperatur in 20 ml Methylenchorid gerührt. Anschließend wird das Lösungsmittel i. Vak. abdestilliert und der Rückstand aus Acetonitril kristallisiert.

Ausb.: 2,32 g (67%)) Schmp. 183°C.

$C_{21}H_{18}N_2O_3$ (346,4) Ber.: C 72,81 H 5.24 N 8,09

Gef.: C 72,72 H 5,19 N 8,28

$^1$H-NMR-Daten: ($CDCl_3$, TMS als interner Standard) δ = 4,07 (dd) 1H, 4,16 (dd) 1H, 4,35—4,5 (m) 2H, 5,28 (m) 1H, 6,81 (d) 1H, 7.3—7,55 (m) 7H, 7,80 (m) 1H, 8,14 (m) 1H, 8,26 (m) 2H, 9,6 (br.) 1H, austauschbar mit $D_2O$, ppm.

N-Benzoyl-N'-[2-hydroxy-3-(1-naphthoxy)propyl]-N''-[3-(imidazol-4-yl)propyl]guanidin

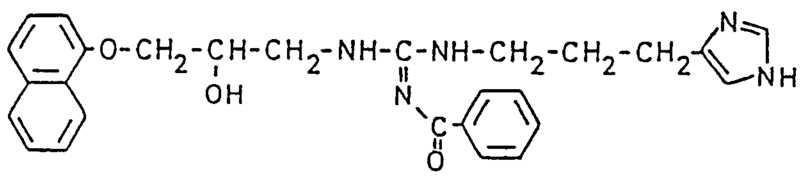

Die Herstellung und Isolierung erfolgt analog Beispiel 15, ausgehend von 1,73 g (5 mmol) 2-Benzoylimino-5-[(1-naphthoxy)methyl]oxazolidin. Das nach Eindampfen des Eluates erhaltene Öl verfestigt sich beim Rühren mit Wasser.

Ausb.: 0,8 g (34%) farbloser Feststoff, der bei 88—90°C sintert.

$C_{27}H_{29}N_5O_3$ (471,6)

MS (FAB-Methode): m/z (rel. Int. [%]) = 472 ([M + H]$^+$, 10), 44 (100).

$^1$H-NMR-Daten: ($d_6$-DMSO, TMS als interner Standard) δ = 1,85 (m) 2H, 2,60 (t) 2H, 3,37 (m) 2H, 3,57 (m) 2H, 3,9—4,5 (m) 3H, 5,7 (br.) 1H, austauschbar mit $D_2O$, 6,6—8,4 (m) 14H, ppm.

Beispiel 18

N-[2-Hydroxy-3-(1-naphthoxy)propyl]-N'-[3-(imidazol-4-yl)propyl]guanidin

Herstellung der Vorstufen

a) N-Benzoyl-N'-[2-hydroxy-3-(1-naphthoxy)propyl]thioharnstoff

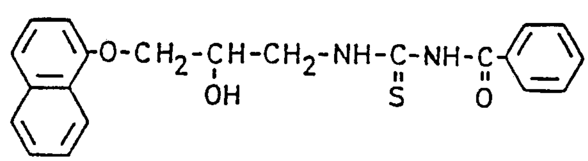

2,17 g (10 mmol) 2-Hydroxy-3-(1-naphthoxy)propylamin und 1,63 g (10 mmol) Benozylisocyanat werden in 120 ml Chloroform 60 min unter Rückfluß erhitzt. Anschließend wird das Lösungsmittel i. Vak. abdestilliert, der Rückstand mit Ether zur Kristallisation gebracht und aus Ethanol umkristallisiert.

Ausb.: 3,4 g (89%); Schmp. 137°C.

$C_{21}H_{20}N_2O_3S$ (380,5) Ber.: C 66,30 H 5,30 N 7,36

Gef.: C 66,07 H 5,16 N 7,35

$^1$H-NMR-Daten: ($CDCl_3$, TMS als interner Standard) δ = 3,10 (d) 1H, austauschbar mit $D_2O$, 4,05 (m) 1H, 4,15—4,35 (m) 3H, 4,56 (m) 1H, 6,84 (d) 1H, 7,3—7,7 (m) 7H, 7,75—7,9 (m) 3H, 8,27 (m) 1H, 9,15 (s) 1H, austauschbar mit $D_2O$, 11,17 (m) 1H, austauschbar mit $D_2O$, ppm.

b) N-[2-Hydroxy-3-(1-naphthoxy)propyl]thioharnstoff

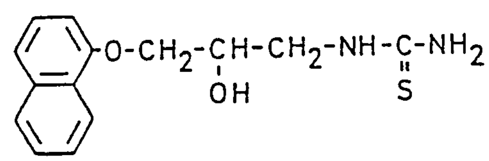

2,85 g (7,5 mmol) N-Benzoyl-N'-[2-hydroxy-3-(1-naphthoxy)propyl]thioharnstoffwerden mit 2,1 g Kaliumcarbonat in einer Mischung aus 30 ml Wasser und 100 ml Methanol 60 min unter Rückfluß erhitzt. Anschließend wird i. Vak. eingedampft, der ausgefallene Feststoff mit Wasser gewaschen und aus Ethanol/Wasser umkristallisiert

Ausb.: 1,82 g (88%) farblose Kristalle von Schmp. 158°C.

$C_{14}H_{16}N_2O_2S$ (276,4) Ber.: C 60,85 H 5,84 N 10,14
Gef.: C 60,70 H 5,82 N 10,12

$^1$H-NMR-Daten: ($d_6$-DMSO, TMS als interner Standard) δ = 3,25—3,65 (m) 2H, 3,7—4,2 (m) 3H, 5,47 (d) 1H, austauschbar mit $D_2O$, 6,96 (d) 1H, 7,1 (br.) 2H, austauschbar mit $D_2O$, 7,3—8,0 (m) 6H, 1H, austauschbar mit $D_2O$, 8,30 (m) 1H, ppm.

c) 2-Imino-5-[(1-naphthoxy)methyl]oxazolidin

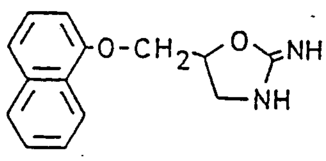

1,38 g (5 mmol) N-[2-Hydroxy-3-(1-napthoxy)propyl]thioharnstoff werden bei Raumtemp. mit 0,4 ml Methyliodid in 100 ml Ethanol über Nacht gerührt. Das Lösungsmittel wird i. Vak. abdestilliert und der Rückstand aus Ethanol/Ether umkristallisiert.

Ausb.: 1,57 g (85%); Schmp. 169°C

$C_{14}H_{14}N_2O_2 \cdot HI$ (370,2) Ber.: C 45,42 H 3,81 N 7,57
Gef.: C 45,22 H 4,05 N 7,63

$^1$H-NMR-Daten: ($d_6$-DMSO, TMS als interner Standard) δ = 3,87 (dd) 1H, 4,08 (dd) 1H, 4,45—4,6 (m) 2H, 5,64 (m) 1H, 7,04 (d) 1H, 7,4—7,6 (m) 4H, 7,92 (d) 1H, 8,09 (d) 1H, 9,25 (br.) 3H, austauschbar mit $D_2O$, ppm.

N-[2-Hydroxy-3-(1-naphthoxy)propyl]-N'-[3-(imidazol-4-yl)propyl]guanidin

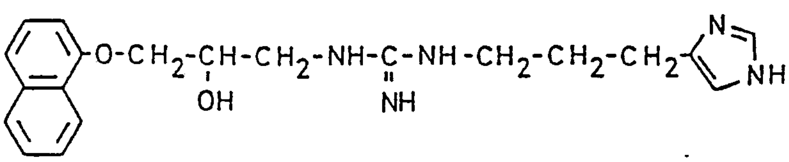

1,30 g (3,5 mmol) 2-Imino-5-[(1-naphthoxy)methyl]oxazolidin und 0,48 g (3,8 mmol) 3-(Imidazol-4-yl)propylamin werden in 40 ml wasserfreiem Pyridin unter Rückfluß erhitzt. Nach dem Eindampfen i. Vak. wird das Reaktionsprodukt durch präparative Schichtchromatographie isoliert und gereinigt (Kieselgel 60 $PF_{254}$, gipshaltig; Fließmittel: Chloroform/Methanol, 85 + 15, Ammoniumatmosphäre). Man erhält 0,85 g (49%) des Hydroiodids als hygroskopischen, nicht kristallinen Feststoff.

$C_{20}H_{25}N_5O_2 \cdot HI$ (495,4)

MS (FAB-Methode): m/z (rel. Int. [%]) = 368 ([M + H]$^+$, 77), 351 (8), 157 (23), 109 (100).

$^1$H-NMR-Daten: ($d_6$-DMSO, TMS als interner Standard) δ = 1,83 (m) 2H, 2,66 (t) 2H, 3,22 (dt) 2H, 3,56 (m) 2H, 4,2—4,4 (m) 3H, 6,99 (d) 1H, 7,3—7,6 (m) 5H, 7,89 (m) 1H, 8,26 (m) 1H, 8,75 (d) 1H, ppm.

## Beispiel 19

N-Benzoyl-N'-[3-(imidazol-4-yl)propyl]-N''-(2-benzyloxyethyl)guanidin

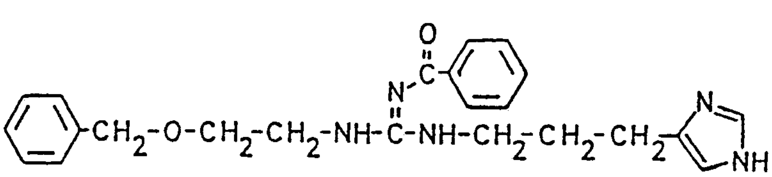

0,76 g (5 mmol) 2-Benzyloxyethylamin und 1,59 g (5 mmol) N-Benzoyl-diphenylimidocarbonat werden in 20 ml Methylenchlorid 15 min bei Raumtemperatur gerührt. Anschließend wird das Lösungsmittel i. Vak. abdestilliert, der Rückstand in 30 ml Pyridin aufgenommen und nach Zusatz von 0,69 g (5,5 mmol) 3-(Imidazol-4-yl)propylamin 60 min unter Rückfluß erhitzt. Der Reaktionsansatz wird i. Vak. eingedampft, der Rückstand in verdünnter Salzsäure gelöst und zur Entfernung des gebildeten Phenols 3 mal mit Ether extrahiert. Nach Alkalisieren der wäßbrigen Phase mit Ammoniak wird 2mal mit Methylenchlorid extrahiert, die organische Phase mit Wasser gewaschen, über Natriumsulfat getrocknet und i. Vak. eingedampft. Das Rohprodukt wird durch präparative Schichtchromatographie gereinigt (Kieselgel 60 $PF_{254}$, gipshaltig; Fließ-

mittel: Chloroform/Methanol, 99 + 1, Ammoniakatmosphäre). Nach Kristallisation aus Ethylacetat erhält man 1,42 g (70%) farblose Kristalle vom Schmp. 117—118°C.

$C_{23}H_{27}N_5O_2$ (405,5) Ber.: C 68,13 H 6,71 N 17,27
Gef.: C 67,94 H 6,68 N 17,24

IR (KBr): 1605 (C=O) $cm^{-1}$.

$^1$H-NMR-Daten: ($CDCl_3$, TMS als interner Standard) δ = 1,75 (m) 2H, 2,56 (t) 2H, 3,2—3,9 (m) 6H, 4,57 (s) 2H, 6,71 (s), 1H, 6,9—7,6 (m) 9H, 8,16 (m) 2H, ppm.

Beispiel 20

N-(2-Benzyloxyethyl-N'-[3-(imidazol-4-yl)propyl]guanidin

Herstellung der Vorstufen

a) N-Benzoyl-N'-(2-benzyloxyethyl)thioharnstoff

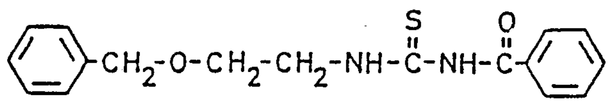

1,51 g (10 mmol) 2-Benzyloxyethylamin und 1,63 g (10 mmol) Benzoylisothiocyanat werden in 120 ml Chloroform 30 min unter Rückfluß erhitzt. Anschließend wird das Lösungsmittel i. Vak. abdestilliert und der ölige Rückstand mit Ether zur Kristallisation gebracht.

Ausb.: 2,97 g (95%); Schmp. 89—90°C (Ether).

$C_{17}H_{18}N_2O_2S$ (314,4) Ber.: C 64,94 H 5,77 N 8,91
Gef.: C 65,00 H 5,81 N 8,76

MS: m/z (rel, Int. [%]) = 314 ($M^{+\cdot}$, 3), 105 (100), 91 (96), 77 (83).

$^1$H-NMR-Daten: ($CDCl_3$, TMS als interner Standard) δ = 3,74 (t) 2H, 3,95 (dt) 2H, 4,61 (s) 2H, 7,25—7,7 (m) 8H, 7,86 (m) 2H, 9,05 (br.) 1H, austauschbar mit $D_2O$, 11,0 (br.) 1H, austauschbar mit $D_2O$, ppm.

b) N-(2-Benzyloxyethyl)-S-methyl-isothiouroniumiodid

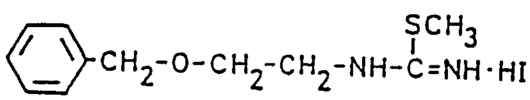

2,36 g (7,5 mmol) N-Benzoyl-N'-(2-benzyloxyethyl)thionharnstoff werden mit 2,1 g Kaliumcarbonat in einer Mischung aus 30 ml Wasser und 100 ml Methanol 40 min unter Rückfluß erhitzt. Anschließend wird i. Vak. eingedampft, der Rückstand in Ether aufgenommen und 3mal mit Wasser gewaschen. Die organische Phase wird über Natriumsulfat getrocknet, i. Vak. eingedampft, der ölige Rückstand in 100 ml Ethanol aufgenommen und nach Zusatz von 0,6 ml Methyliodid über Nacht bei Raumtemperatur gerührt. Das Lösungsmittel wird i. Vak. abdestilliert und der Rückstand mit Aceton und Ether verrührt. Man erhält 2,40 g (91%) Isothiouroniumsalz als farblosen Feststoff von Schmp. 116—117°C.

$C_{11}H_{16}N_2OS \cdot HI$ (352,2) Ber.: C 37,51 H 4,87 N 7,95
Gef.: C 37,33 H 4,91 N 7,76

IR (KBr): 1655 ($C=N^+$) $cm^{-1}$.

$^1$H-NMR-Daten: ($d_6$-DMSO, TMS als interner Standard) δ = 2,60 (s) 3H, 3,45—3,65 (m) 4H, 4,52 (s) 2H, 7,35 (m) 5H, 9,2 (br.) 3H, austauschbar mit $D_2O$, ppm.

N-(2-Benzyloxyethyl)-N'-[3-(imidazol-4-yl)propyl]guanidin

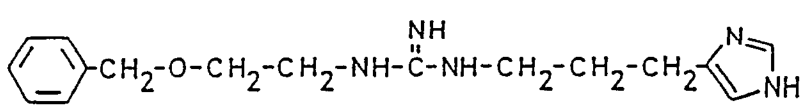

1,76 g (5 mmol) N-(2-Benzyloxyethyl)-S-methyl-isothiouroniumiodid und 0,69 g (5,5 mmol) 3-(Imidazol-4-yl)propylamin werden in 40 ml wasserfreiem Pyridin 3 h unter Rückfluß erhitzt. Nach dem Eindampfen i. Vak. wird das Reaktionsproudukt durch präparative Schichtchromatographie isoliert und gereinigt (Kieselgel 60 $PF_{254}$, gipshaltig: Fließmittel: Chloroform/Methanol, 85:15, Ammoniakatmosphäre). Man erhält 1,5 g (70%) N-(2-Benzyloxyethyl)-N'-[3-(imidazol-4-yl)propyl]guanidin-hydroiodid als zähes Öl.

$C_{16}H_{23}N_5O \cdot HI$ (429,3)

MS: m/z (rel. Int [%] = 301 ($M^{+\cdot}$, 1) 128 (70), 127 (40), 95 (44), 91 (100), 81 (38).

$^1$H-NMR-Daten: ($d_6$-DMSO, TMS als interner Standard) δ = 1,79 (m) 2H, 2,59 (t) 2H, 3,20 (dt) 2H, 3,40 (dt) 2H, 3,55 (t) 2H, 4,53 (s) 2H, 6,94 (m) 1H, 7.25—7,45 (m) 5H, 7,85 (d) 1H, ppm.

Das Dipikrat schmilzt nach Umkristallisation aus Ethanol bei 131—133°C.

$C_{16}H_{23}N_5O \cdot 2C_6H_3N_3O_7$ (759,6) Ber.: C 44,27 H 3,85 N 20,28
Gef.: C 44,03 H 3,85 N 20,12

Beispiel 21

N-Benzoyl-N'-[3-(imidazol-4-yl)propyl]-N''-(2-benzylthioethyl)guanidin

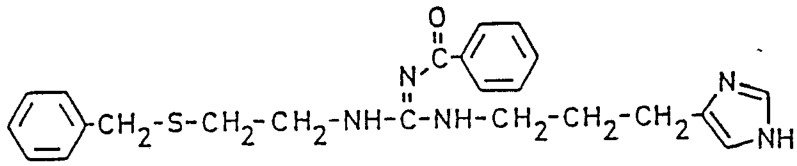

Die Herstellung erfolgt analog Beispiel 19 ausgehend von 0,84 g (5 mmol) 2-Benzylthioethylamin.

Ausbeute: 1,53 g (73%); Schmp 133—134°C (Ethylacetat).

$C_{23}H_{27}N_5OS$ (421,6) Ber.: C 65,53 H 6,46 N 16,61
Gef.: C 65,50 H 6,46 N 16,60

$^1$H-NMR-Daten: ($CDCl_3$, TMS als interner Standard) δ = 1,94 (m) 2H, 2,69 (t) 2H, 2,73 (t) 2H, 3,2—3,7 (m) 4H, 3,77 (s) 2H, 6,78 (s) 1H, 7,05—7,5 (m) 9H, 8,20 (m) 2H, ppm.

Beispiel 22

N-Benzoyl-N'-[3-(imidazol-4-yl)propyl]-N''-{2-[(naphth-1-yl)methylthio]ethyl}guanidin

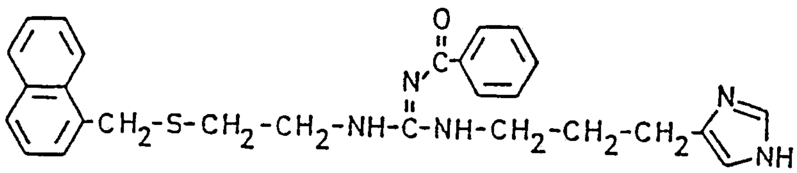

Die Herstellung erfolgt analog Beispiel 19 ausgehend von 1,09 g (5 mmol) 2-[(Naphth-1-yl)methylthio]-ethylamin.

Ausbeute: 1,5 g (64%); Schmp. 128—130°C (Ethylacetat).

$C_{27}H_{29}N_5OS$ (471,16) Ber.: C 68,76 H 6,20 N 14,85
Gef.: C 68,70 H 6,16 N 14,82

IR (KBr): 1605 (C=O) $cm^{-1}$.

$^1$H-NMR-Daten: ($CDCl_3$, TMS als interner Standard) δ = 1,83 (m) 2H, 2,67 (t) 2H, 2,78 (t) 2H, 3,32 (dt) 2H, 3,62 (dt) 2H, 4,20 (s) 2H, 6,71 (s) 1H, 7,1—8,4 (m) 13H, ppm.

Beispiel 23

N-[3-(Imidazol-4-yl)propyl]-N'-{2-[(naphth-1-yl)methylthio]ethyl}guanidin

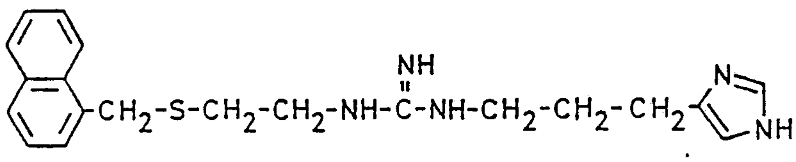

0,85 g (1,8 mmol) N-Benzoyl-N'-[3-(imidazol-4-yl)propyl]-N''-{2-[(naphth-1-yl)methylthio]ethyl}-guanidin (Beispiel 57) werden in 45 ml 20proz. Salzsäure 7 h unter Rückfluß erhitzt. Nach dem Erkalten des Reaktionsansatzes wird die entstandene Benzosäure durch Extraktion mit Ether entfernt, die wäßbrige Phase i. Vak. zur Trockne eingedampft und der Rückstand im Hockvakuum getrocknet. Man erhält 0,72 g (91%) trockene stark hygroskopischen Schaum.

$C_{20}H_{25}N_5S \cdot 2HCl$ (440,4) Molmasse (MS): Ber: 367,18307; Gef: 367,18191

MS: m/z (rel. Int. [%]) = 367 ($M^2$., 2), 242 (30), 226 (9), 141 (84), 95 (32)

$^1$H-NMR-Daten: ($d_6$-DMSO, TMS als interner Standard) δ = 1,85 (m) 2H, 2,4—3,0 (m) 4H), 3,0—3,7 (m) 4H, 4,30 (s) 2H, 7,2—8,4 (M) 12H, 4H austauschb. mit $D_2O$, 8.86 (d) 1H, ppm.

Beispiel 24

N-Benzoyl-N'-[3-(imidazol-4-yl)propyl]-N'']-{2-[(1-phenylethyl)thio]ethyl}guanidin

Herstellung der Vorstufe

a) 2-[(1-Phenylethyl)thio]ethylamin

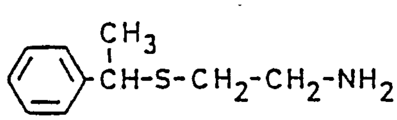

In eine Lösung von 2,3 g (0,1 mol) Natrium in 150 ml Methanol werden unter Begasung mit Stickstoff 5,68 g (50 mmol) Cysteamin-hydrochlorid eingetragen und anschließend 9,25 g (50 mmol) 1-Phenylethylbromid in 30 ml Methanol zugetropft. Das Reaktionsgemische wird 1 h bei Raumtemperatur gerührt, das Lösungsmittel i. Vak. abdestilliert, der Rückstand in 5proz. Salzsäure gelöst und mit Ether extrahiert. Nach Akalisieren mit 15proz. Natronlauge wird die wäßrige Phase mit Methylenchlorid ausgeschüttelt, die organische Phase mit Wasser gewaschen, über Natriumsulfat getrocknet und i. Vak. eingedampft. Es bleiben 6,65 g (73%) Öl zurück, das dür weitere Umsetzungen rein genug ist. Sdp. 77—79°C/0,25 mm.

$C_{10}H_{15}NS$ (181,3)

$^1$H-NMR-Daten: ($CDCl_3$, TMS als interner Standard) δ = 1,58 (d) 3H, 2,43 (m) 2H, 2,74 (t) 2H, 3,97 (q) 1H, 7,2—7,4 (m) 5H, ppm.

Das Hydrochlorid schmilzt nach Umkristallisation aus Ethanol/Ether bei 135—136°C.

$C_{10}H_{15}NS \cdot HCl$ (217,8) Ber.: C 55,16 H 7,41 N 6,43
Gef.: C 55,24 H 7,62 N 6,38

N-Benzoyl-N'-[3-(imidazol-4-yl)propyl]-N''{2-[(1-phenylethyl)thio]ethyl}guanidin

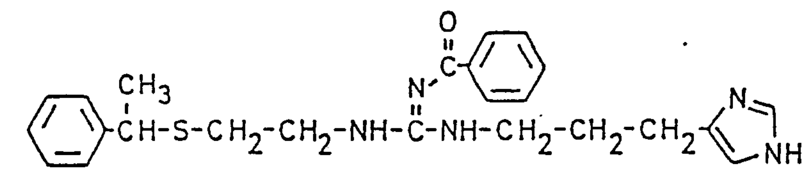

Die Herstellung erfolgt analog Beispiel 19 aus 0,91 g (5 mmol) 2-[(1-Phenylethyl)thio]ethylamin.

Ausb.: 1,6 g (73%); Schmp. 128—130°C (Ethylacetat).

$C_{24}H_{29}N_5OS$ (435,6) Ber.: C 66,18 H 6,71 N 16,08
Gef.: C 65,99 H 6,73 N 15,88

MS: m/z (rel. Int. [%]) = 435 (M$^+$., 1), 109 (23), 105 (100), 95 (24), 77 (69).

$^1$H-NMR-Daten: ($CDCl_3$, TMS als interner Standard) δ = 1,56 (d) 3H, 1,91 (m) 2H, 2,55—2,75 (m) 4H, 3,37 (m) 2H, 3,60 (m) 2H, 4,03 (q) 1H, 6,75 (s), 1H, 7,1—7,5 (m) 9H, 8,20 (m) 2H, ppm.

Beispiel 25

N-[3-(Imidazol-4-yl)propyl]-N'-{2-[(1-phenylethyl)thio]ethyl}guanidin

Die Herstellung erfolgt analog Beispiel 20 ausgehend von 1,81 g (10 mmol) 2-[(1-Phenylethyl)thio]-ethylamin (Beispiel 24 a).

Vorstufen

a) N-Benzoyl-N'-{2[(1-phenylethyl)thio]ethyl}thioharnstoff

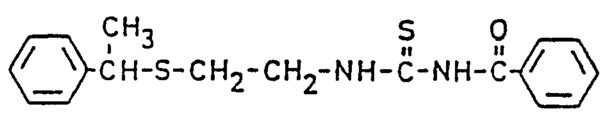

Ausb.: 3,13 g (91%); Schmp. 72—73°C (Ether/Petrolether).

$C_{18}H_{20}N_2OS_2$ (344,5) Ber.: C 62,76 H 5,85 N 8,13
Gef.: C 62,57 H 5,89 N 7,95

MS: m/z (rel. Int. [%]) = 344 (M$^+$., 1), 239 (100), 105 (96).

$^1$H-NMR-Daten: ($CDCl_3$, TMS als interner Standard) δ = 1,61 (d) 3H, 2,66 (t) 2H, 3,78 (dt) 2H, 4,07 (q) 1H, 7,15—7,7 (m) 8H, 7,85 (m) 2H, 8,98 (br.) 1H, austauschbar mit $D_2O$, 10,9 (br.) 1H, austauschbar mit $D_2O$, ppm.

b) S-Methyl-N-{2-[(1-phenylethyl)thio]ethyl}-isothiouroniumiodid

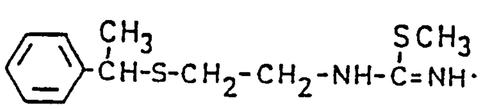

Aus 2,58 g (7,5 mmol) N-Benzoyl-N'-{2-[(1-phenylethyl)thio]ethyl}thioharnstoff erhält man 2,44 g (85%) Öl, das für die weitere Umsetzung rein genug ist.

$C_{12}H_{18}N_2S_2 \cdot HI$ (382,3) Molmasse (MS): Ber: 254,09115, Gef: 254,09119

MS: m/z (rel. Int. [%]) = 254 (M$^+$., 6), 149 (100), 128 ([HI]$^+$, 38), 105 (94).

$^1$H-NMR-Daten: ($d_6$-DMSO, TMS als interner Standard) δ = 1,50 (d) 3H, 2,4—2,7 (m) 2H, 2,60 (s) 3H, 3,4 (m) 2H, 4,11 (q) 1H, 7,33 (m) 5H, ppm.

N-[3-(Imidazol-4-yl)propyl]-N'-{2-[(1-phenylethyl)thio]ethyl}guanidin

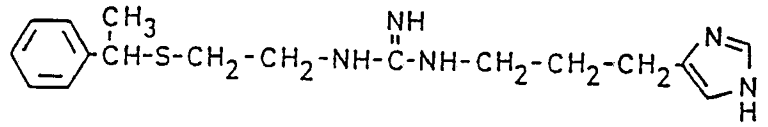

Aus, 1,91 g (5 mmol) S-Methyl-N-{2-[(1-phenylethyl)thio]ethyl}isothiouroniumiodid erhält man 1,4 g (61%) des Guanidin-Hydroiodids in Form eines zähen Öls.

$C_{17}H_{25}N_5S \cdot HI$ (459,4)

MS: m/z (rel. Int. [%]) = 332 [(M + H)$^+$, 43), 109, (46), 105 (100) (FAB-Methode).

$^1$H-NMR-Daten: ($d_6$-DMSO, TMS als interner Standard) δ = 1,51 (d) 3H, 1,78 (m) 2H, 2,25—2,8 (m) 4H, 2,9—3,5 (m) 4H, 4,11 (q) 1H, 7,04 (m) 1H, 7,1—7,7 (m) 9H, 4H austauschbar mit $D_2O$, 8,11 (d) 1H, ppm.

## Beispiel 26

N-Benzoyl-N'-[3-(imidazol-4-yl)propyl]-N''-{2-[(p-methyl-α-phenylbenzyl)thio]ethyl}guanidin

Herstellung der Vorstufe

2-[(p-Methyl-α-phenylbenzyl)thio]ethylamin

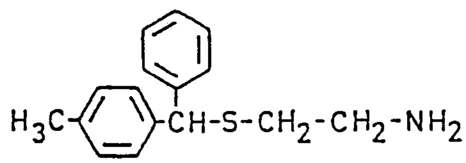

9,41 g (50 mmol) 4-Methylbenzhydrol und 5,68 g (50 mmol) Cysteamin-hydrochlorid werden in 100 ml Isopropylalkohol unter Zusatz von 10 ml konz. Salzsäure 1 h unter Rückfluß erhitzt, anschließend wird das Lösungsmittel i. Vak. abgedampft, der Rückstand mit Wasser verdünnt und 2mal mit Ether ausgeschüttelt. Nach Alkalisieren mit 10proz. Natronlauge wird die wäßrige Phase 2mal mit Ether extrahiert, die etherische Lösung mit Wasser gewaschen, über Natriumsulfat getrocknet und i. Vak. eingedampft. Man erhält 9,4 g (73%) Öl, das für weitere Umsetzungen rein genug ist.

$C_{16}H_{19}NS$ (257,4)

MS: m/z (rel. Int. [%]) = 257 (M$^+$., 2), 181 (100), 166 (38).

$^1$H-NMR-Daten: ($CDCl_3$, TMS als interner Standard) δ = 2.32 (s) 3H, 2,51 (t) 2H, 2,81 (t) 2H, 5,14 (s) 1H, 7,0—7,45 (m) 9H, ppm.

Das Hydrochlorid schmilzt nach Umkristallization aus Aceton/Ether bei 133—135°C

| | | | | |
|---|---|---|---|---|
| $C_{10}H_{19}NS \cdot HCl$ (293,9) | Ber.: | C 65,40 | H 6,86 | N 4,77 |
| | Gef.: | C 65,49 | H 6,85 | N 4,61 |

N-Benzoyl-N'-[3-(imidazol-4-yl)propyl]-N''-{2-[(p-methyl-α-phenylbenzyl)thio]ethyl}guanidin

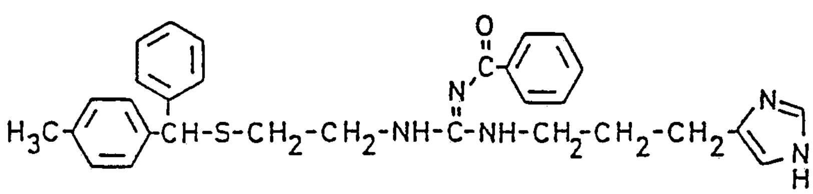

Die Herstellung erfolgt analog Beispiel 19 aus 1,29 g (5 mmol) 2-[(p-Methyl-α-phenylbenzyl)thio]ethylamin. Nach dem Abdampfen des Pyridins i. Vak. kristallisieren auf Zugabe von Ethanol 1,6 g (63%) farbloser Feststoff, der nach Umkristallisieren aus Ethanol bei 155—156°C schmilzt.

| | | | | |
|---|---|---|---|---|
| $C_{30}H_{33}N_5OS$ (511,7) | Ber: | C 70,42 | H 6,50 | N 13,69 |
| | Gef: | C 70,27 | H 6,56 | N 13,51 |

MS: m/z (rel. Int. [%]) = 511 (M$^+$., 1), 181 (81), 166 (25), 109 (12), 105 (100).

$^1$H-NMR-Daten: ($CDCl_3$, TMS als interner Standard) δ = 1,90 (m) 2H, 2,29 (s) 3H, 2,65 (t) 2H, 2,70 (t) 2H, 3,35 (br.) 2H, 3.65 (br.) 2H, 5.22 (s) 1H, 6,73 (s) 1H, 7,0—7,5 (m) 13H, 8,20 (m) 2H, ppm.

Beispiel 27

N-[3-Imidazol-4-yl)propyl]-N'-{2-[(p-methyl-α-phenylbenzyl)thio]ethyl}guanidin

Die Herstellung und Isolierung erfolgt analog Beispiel 20 ausgehend von 2,57 g (10 mmol) 2-[(p-Methyl-α-phenylbenzyl)thio]ethylamin

Vorstufen

a) N-Benzoyl-N'-{2-[(p-methyl-α-phenylbenzyl)thio]ethyl}thioharnstoff

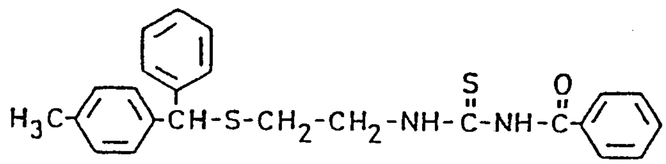

Ausb. 3,7 g (88%); Schmp. 94—95°C (Ether/Petrolether).

$C_{24}H_{24}N_2OS_2$ (420,6) Ber.: C 68,54 H 5,75 N 6,66

Gef.: C 68,37 H 5,77 N 6,61

MS: m/z (rel. Int. [%]) = 239 ([M—$C_{14}H_{13}$]$^+$, 90), 181 (100), 105 (90).

$^1$H-NMR-Daten: ($CDCl_3$, TMS als interner Standard) δ = 2,32 (s) 3H, 2,75 (t) 2H, 3,86 (dt) 2H, 5.28 (s) 1H, 7,0—7,7 (m) 12H, 7,85 (m) 2H, 9,0 (br.) 1H, austauschbar mit $D_2O$, 10,95 (br.) 1H, austauschbar mit $D_2O$, ppm.

b) S-Methyl-N-{2-[(p-Methyl-α-phenylbenzyl)thio]ethyl}isothiouroniumiodid

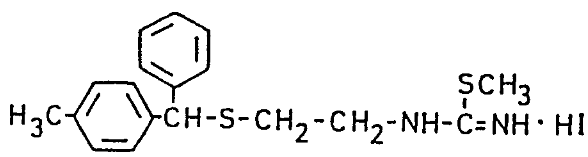

Aus 3,15 g (7,5 mmol) N-Benzoyl-N'-{2-[(p-methyl-α-phenylbenzyl)thio]ethyl}thioharnstoff erhält man 2,85 g (83%) Isothiouroniumsalz als Öl, das für die weitere Umsetzung rein genug ist.

$C_{18}H_{22}N_2S_2\cdot HI$ (458,4) Molmasse (MS): Ber.: 330,12245; Gef: 330,12217

MS: m/z (rel. Int. [%]) = 330 (M$^+$., 9), 254 (5), 181 (90), 149 (100).

$^1$H-NMR-Daten: ($d_6$-DMSO, TMS als interner Standard) δ = 2,26 (s) 3H, 2,4—2,7 (m) 2H, 2,60 (s) 3H, 3,50 (m) 2H, 5,39 (s) 1H, 7,0—7,6 (m) 9H, ppm.

N-[3-(Imidazol-4-yl)propyl]-N'-{2-[(p-methyl-α-phenylbenzyl)thio]ethyl}guanidin

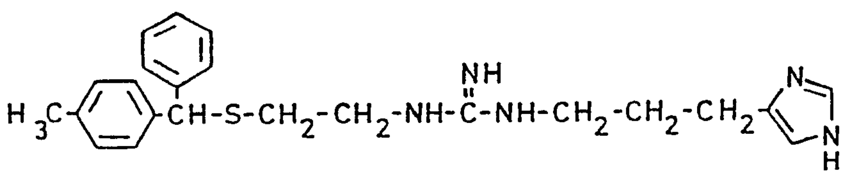

Aus, 2,29 g (5 mmol) N-{2-[(p-Methyl-α-phenylbenzyl)thio]ethyl}-S-methyl-isothiouroniumiodid erhält man 1,5 g (56%) des Guanidin-Hydroiodids in Form eines trockenen Schaumes.

$C_{23}H_{29}N_5S\cdot HI$ (535,5)

MS: m/z (rel. Int. [%]) = 408 ([M + H]$^+$., 25), 181 (100), 109 (32) (FAB-Methode).

$^1$H-NMR-Daten: ($d_6$-DMSO, TMS als interner Standard) δ = 1,77 (m) 2H, 2,26 (s) 3H, 2,3—2,7 (m) 4H, 2,9—3,6 (m) 4H, 5,36 (s) 1H, 6,91 (m) 1H, 7,0—7,7 (m) 13H, 4H austauschbar mit $D_2O$, 7,84 (d) 1H, ppm.

Beispiel 28

N-Benzoyl-N'-[3-(imidazol-4-yl)propyl]-N''-(2-(p-chlorobenzylthio)ethyl]guanidin

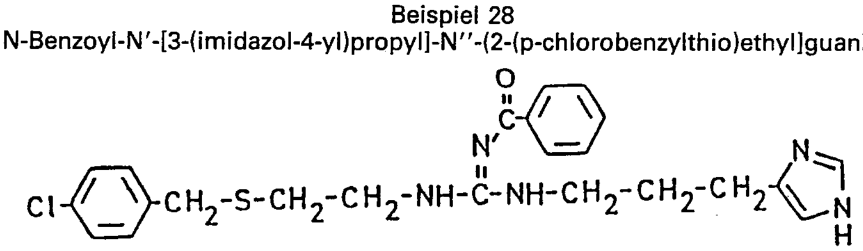

Die Herstellung erfolgt analog Beispiel 19 ausgehend von 1,01 g (5 mmol) 2-(p-Chlorbenzylthio)ethylamin.

Ausb.: 1,14 g (50%); Schmp. 128°C (Acetonitril).
$C_{23}H_{26}ClN_5OS$ (456,0) Ber.: C 60,58 H 5,75 N 15,36
Gef.: C 60,79 H 5,84 N 15,36
MS: m/z (rel. Int. [%] = 455 ($M^{+\cdot}$, 2), 330 (60), 125 (95), 105 (100), 95 (90).
$^1$H-NMR-Daten: ($CDCl_3$, TMS als interner Standard) δ = 1,93 (m); 2H, 2,69 (t) 2H, 2,71 (t) 2H, 3,2—3,7 (m) 4H, 3,73 (S) 2H, 6,82 (s) 1H, 7,2—7,6 (m) 8H, 8,23 (m) 2H, ppm.

Beispiel 29

N-[3-(Imidazol-4-yl)propyl]-N'-[2-(p-chlorbenzylthio)ethyl]guanidin

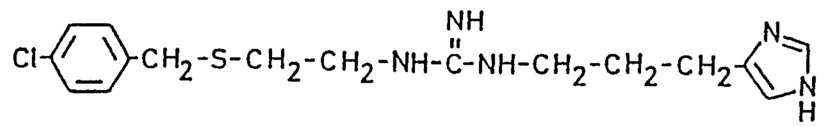

Die Herstellung erfolgt analog Beispiel 58 ausgehend von 0,82 g (1,8 mmol) N-Benzoyl-N'-[3-(imidazol-4-yl)propyl]-N''-[2-(p-chlorbenzylthio)ethyl]guanidin (Beispiel 63).
Ausb.: 0,28 g (37%) trockener, stark hygroskopischer Schaum.
$C_{16}H_{22}ClN_5S \cdot 2HCl$ (424,8)
MS: m/z (rel. Int. [%]) = 352 ($[M + H]^+$, 100), 125 (96), 109 (85) (FAB-Methode).
$^1$H-NMR-Daten: ($d_6$-DMSO, TMS als interner Standard) δ = 1,86 (m) 2H, 2,55 (m) 2H, 2,78 (m) 2H, 3,4—3,6 (m) 4H, 38.5 (s) 2H, 7,39 (m) 4H, 7,55 (m) 1H, 7.74 (s) 2H, austauschbar mit $D_2O$, 7,95 (t) 1H, austauschbar mit $D_2O$, 8,18 (t) 1H, austauschbar mit $D_2O$, 9,12 (d) 1H, ppm.

Beispiel 30

N-Benzoyl-N'-[3-(imidazol-4-yl)propyl]-N''-[2-(m-chlorbenzylthio)ethyl]guanidin

Herstellung der Vorstufe
2-(m-Chlorbenzylthio)ethylamin

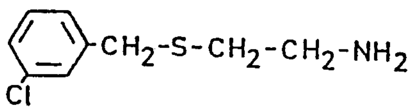

In eine Lösung aus 2,3 g (0,1 mol) Natrium in 60 ml Ethanol werden 5,68 g (50 mmol) Cysteamin-hydrochlorid eingetragen und anschließend 8,05 g (50 mmol) m-Chlorbenzylchlorid zugegeben. Das Reaktionsgemisch wird 1 h unter Rückfluß erhitzt, das Lösungsmittel i. Vak. abdestilliert, der Rückstand mit Wasser versetzt und mit Ether extrahiert. Die organische Phase wird mit 5 N Salzsäure extraheit. Nach Alkalisieren mit wäßrigem Ammoniak wirde die wäßrige Phase mit Ether ausgeschüttelt, die organische Phase mit Wasser gewaschen, über Natriumsulfat getrocknet und i. Vak. eingedampft. Es bleiben 8,2 g (81%) Öl zurück, das für weitere Umsetzungen rein genug ist. Sdp. 96—98°C/0,2 mm.
$C_9H_{12}ClNS$ (201,7)
$^1$H-NMR-Daten: ($CDCl_3$, TMS als interner Standard) δ = 2,50 (m) 2H, 2,85 (m) 2H, 3,67 (s) 2H, 7,1—7,4 (m) 4H, ppm.

N-Benzoyl-N'-[3-(imidazol-4-yl)propyl]-N''[2-(m-chlorbenzylthio)ethyl]guanidin

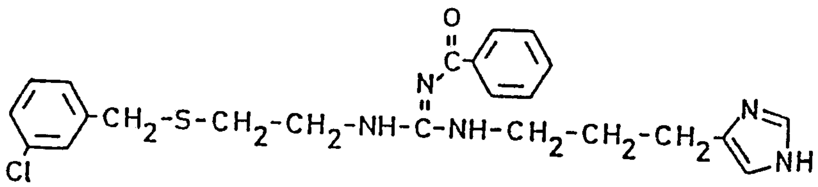

Die Herstellung erfolgt analog Beispiel 19 ausgehend von 1,01 g (5 mmol) 2-(m-Chlorbenzylthio)ethylamin.
Ausb.: 0,95 g (42%); Schmp. 122°C (Acetonitril).
$C_{23}H_{26}ClN_5OS$ (456,0) Ber.: C 60,58 H 5,75 N 15,36
Gef.: C 60,31 H 5,69 N 15,24
MS: m/z (rel. Int. [%]) = 455($M^{+\cdot}$, 5), 330 (60), 125 (90), 105 (100), 95 (93).
$^1$H-NMR-Daten: ($CDCl_3$, TMS als interner Standard) δ = 1,95 (m) 2H, 2,6—2,8 (m) 4H, 3,2—3,6 (m) 4H, 3,71 (s) 2H, 6,78 (s) 1H, 7,1—7,55 (m) 8H, 8,18 (m) 2H, ppm.

Beispiel 31

N-[3-(Imidazol-4-yl)propyl]-N'-[2-(m-chlorbenzylthio)ethyl]guanidin

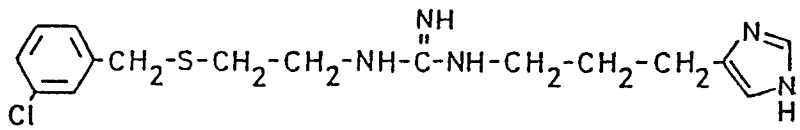

Die Herstellung erfolgt analog Beispiel 23 ausgehend von 0,82 g (1,8 mmol) N-Benzoyl-N'-[3-(imidazol-4-yl)propyl]-N''-[2-(m-chlorbenzylthio)ethyl]guanidin (Beispiel 30).

Ausb.: 0,16 g (21%) trockener, stark hygroskopischer Schaum.

$C_{16}H_{22}ClN_5S \cdot 2HCl$ (424,8)

MS: m/z (rel. Int. [%]) = 352 ($[M + H]^+$, 53), 125 (100), 109 (70) (FAB-Methode)

$^1$H-NMR-Daten: ($d_6$-DMSO, TMS als interner Standard) δ = 1,84 (m) 2H, 2,54 (t) 2H, 2,71 (t) 2H, 3,1—3,6 (m) 4H, 3,82 (s) 2H, 7,3—7,5 (m) 5H, 7,58 (s) 2H, austauschbar mit $D_2O$, 7,69 (t) 1H, austauschbar mit $D_2O$, 7,91 (t) 1H, austauschbar mit $D_2O$, 9,00 (d) 1H, ppm.

Beispiel 32

N-Benzoyl-N'-[3-(imidazol-4-yl)propyl]-N''-[2-(p-methylbenzylthio)ethyl]guanidin

Herstellung der Vorstufe

2-(p-Methylbenzylthio)ethylamin

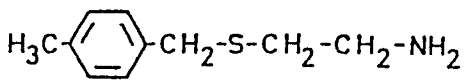

Die Herstellung erfolgt analog Beispiel 30 ausgehend von 7,03 g (50 mmol) p-Methylbenzylchlorid.

Ausb.: 7,08 g (78%) Öl, das für weitere Zwecke rein genug ist.

$C_{10}H_{15}NS$ (181,3)

$^1$H-NMR-Daten: ($CDCl_3$, TMS als interner Standard) δ = 2,32 (s) 3H, 2,53 (t) 2H, 2,82 (t) 2H, 3,66 (s) 2H, 7,1—7,3 (m) 4H, ppm.

N-Benzoyl-N'-[3-(imidazol-4-yl)propyl]-N''-[2-(p-methylbenzylthio)ethyl]guanidin

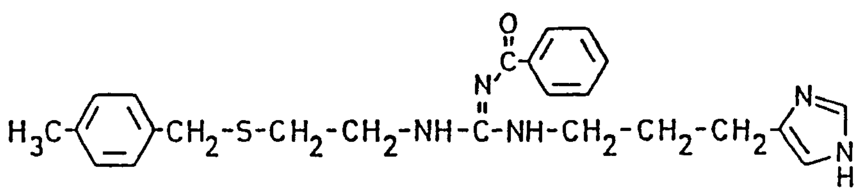

Die Herstellung erfolgt analog Beispiel 19 ausgehend von 0,91 g (5 mmol) 2-(p-Methylbenzylthio)ethylamin.

Ausb.: 1,01 g (46%); Schmp. 155°C (Acetonitril).

$C_{24}H_{29}N_5OS$ (435,6) Ber.: C 66,18 H 6,71 N 16,08

Gef.: C 65,90 H 6,77 N 16,07

MS: m/z (rel. Int. [%]) = 435 ($M^{+\cdot}$, 43), 330 (100), 105 (6).

$^1$H-NMR-Daten: ($CDCl_3$, TMS als interner Standard) δ = 1.96 (m) 2H, 2,32 (s) 3H, 2,65—2,8 (m) 4H, 3,4 (br.) 2H, 3,7 (br.) 2H, 3,74 (s) 2H, 6,78 (s) 1H, 7,06—7,50 (m) 8H, 8,19 (m) 2H, ppm.

Beispiel 33

N-[3-(Imidazol-4-yl)propyl]-N'-[2-(p-methylbenzylthio)ethyl]guanidin

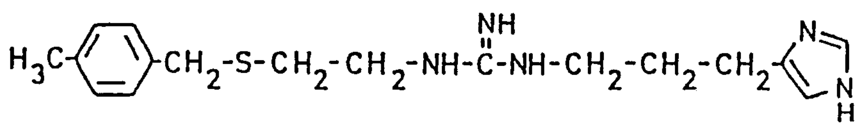

Die Herstellung erfolgt analog Beispiel 23 ausgehend von 0,78 g (1,8 mmol) N-Benzoyl-N'-[3-(imidazol-4-yl)propyl]-N''-[2-(p-methylbenzylthio)ethyl]guanidin (Beispiel 32).

Ausbeute: 0,21 g (29%) trockener, stark hygroskopischer Schaum.

$C_{17}H_{25}N_5S \cdot 2HCl$ (404,4)

MS: m/z (rel. Int. [%]) = 332 ($[M + H]^+$, 26), 109 (27), 105 (100).

$^1$H-NMR-Daten: ($d_6$-DMSO, TMS als interner Standard) δ = 1,86 (m) 2H, 2,28 (s) 3H, 2,52 (m) 2H, 2,72 (t) 2H, 3,20 (m) 2H, 3,38 (m) 2H, 3,77 (s) 2H, 7,05—7,25 (m) 4H, 7,43 (m) 1H, 7,58 (s) 2H, austauschbar mit $D_2O$, 7,74 (t) 1H, austauschbar mit $D_2O$, 7,95 (t) 1H, austauschbar mit $D_2O$, 8,95 (d) 1H, ppm.

Beispiel 34

N-Benzoyl-N'-[(3-(imidazol-4-yl)propyl]-N''-(2-phenylthioethyl)guanidin

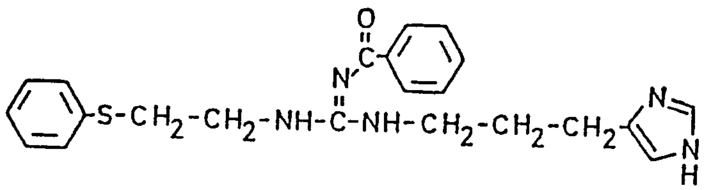

Die Herstellung erfolgt analog Beispiel 19 aus 0,77 g (5 mmol) 2-Phenylthioethylamin. Nach dem Abdampfen des Pyridins wird der Rückstand durch Rühren mit Ether zur Kritallisation gebracht. Das Rohprodukt wird durch mehrfaches Ausrühen mit Ether von anhaftendem Phenol befreit, durch Auflösen in verdünnter Salzsäure und Alkalisieren mit Ammoniak umgefällt und anschließend aus Ethanol umkristallisiert.

Ausb.: 1,9 g (93%); Schmp. 156—158°C

$C_{22}H_{25}N_5OS$ (407,5) Ber.: C 64,84 H 6,18 N 17,18

Gef.: C 64,42 H 6,19 N 16,98

MS: m/z (rel. Int. [%]) = 407 ($M^+$., 3), 190 (25), 137 (13), 124 (27), 109 (32), 105 (100), 95 (30), 81 (25), 77 (57), 58 (53), 43 (54).

$^1$H-NMR-Daten: ($d_6$-DMSO, TMS als interner Standard) δ = 1,83 (m) 2H, 2,58 (t) 2H, 2,9—3,8 (m) 6H, 6,77 (s) 1H, 7,0—7,7 (m) 9H, 8,07 (m) 2H, ppm.

Beispiel 35

N-[3-(Imidazol-4-yl)propyl]-N'-(2-phenylthioethyl)guanidin

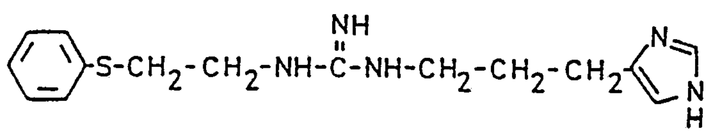

0,82 g (2 mmol) N-Benzoyl-N'-[3-(imidazol-4-yl)propyl]-N''-(2-phenylthioethyl)gaunidin werden 7 h in 18proz. Salzsäure unter Rückfluß erhitzt und analog Beispiel 58 aufgearbeitet. Man erhält 0,72 g (96%) trockenen, hygroskopischen Schaum.

$C_{15}H_{21}N_5S \cdot 2HCl$ (376,4) Molmasse (MS): Ber.: 303,15177; Gef.: 303,15214

MS: m/z (rel. Int. [%]) = 303 ($M^+$., 10), 178 (42), 124 (96), 110 (100), 109 (46), 95 (54).

$^1$H-NMR-Daten: ($d_6$-DMSO, TMS als interner Standard) δ = 1,85 (m) 2H, 2,75 (t) 2H, 2,9—3,7 (m) 6H, 7,0—7,55 (m) 6H, 7,61 (s) 2H, austauschbar mit $D_2O$, 8,00 (t) 1H, austauschbar mit $D_2O$, 8,16 (t) 1H, austauschbar mit $D_2O$, 8,99 (d) 1H, ppm.

Beispiel 36

N-Benzoyl-N'-[3-(imidazol-4-yl)propyl]-N''-[(3-phenylthio)propyl]guanidin

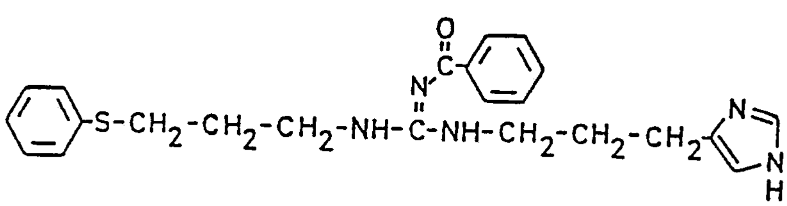

Die Herstellung erfolgt analog Beispiel 19 aus 0,84 g (5 mmol) 3-Phenylthiopropylamin. Nach dem Abdampfen des Pyridins i. Vak. wird der Rückstand durch Rühren mit Ether zu Kristallisation gebracht. Das Rohprodukt wird mehrfach mit Ether ausgerührt, aus Ethanol/Wasser umgefällt und anschließend aus Ethylacetat umkristallisiert.

Ausb.: 1,86 g (88%); Schmp. 130—132°C

$C_{23}H_{27}N_5OS$ (421,6) Ber.: C 65,53 H 6,46 N 16,61

Gef.: C 65,27 H 6,49 N 16,59

MS: m/z (rel. Int. [%]) = 421 ($M^+$., 1), 109 (20), 105 (10), 95 (30), 58 (100).

$^1$H-NMR-Daten: ($CDCl_3$, TMS als interner Standard) δ = 1,6—2,3 (m) 4H, 2,66 (t) 2H, 3,09 (t) 2H, 3,15—3,75 (m) 4H, 6,79 (s) 1H, 6,9—7,6 (m) 9H, 8,22 (m) 2H, ppm.

Beispiel 37

N-[3-(Imidazol-4-yl)propyl]-N'-(3-phenylthiopropyl)guanidin

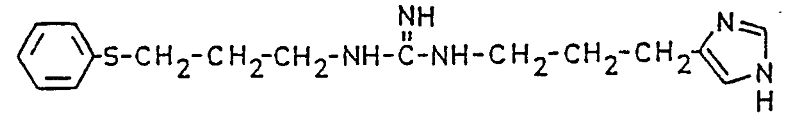

0,84 g (2 mmol) N-Benzoyl-N'-[3-(imidazol-4-yl)propyl)-N''-(3-phenylthiopropyl)guanidin werden 7 h in 18proz. Salzsäure unter Rückfluß erhitzt und analog Beispiel 23 aufgearbeitet. Man erhält 0,71 g (91%) trockenen, hygroskopischen Schaum.

$C_{16}H_{23}N_5S \cdot 2HCl$ (390,4) Molmasse (MS): Ber.: 317,16742; Gef.: 317,16675

MS: m/z (rel. Int. [%]) = 317 ($M^+$., 6), 192 (10), 167 (87), 109 (65), 95 (100).

$^1$H-NMR-Daten: ($d_6$-DMSO, TMS als interner Standard) δ = 1,5—2,2 (m) 4H, 2,4—3,6 (m) 8H, 7,0—7,5 (m) 6H, 7,61 (s) 2H, austauschbar mit $D_2O$, 7,8—8,3 (m) 2H, austauschbar mit $D_2O$, 9,00 (d) 1H, ppm.

Beispiel 38

N-Benzoyl-N'-[3-(imidazol-4-yl)propyl]-N''-(4-phenylbutyl)guanidin

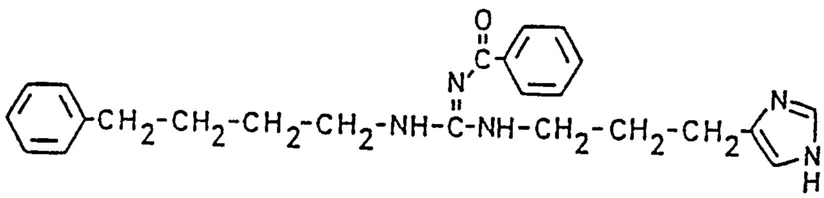

Die Herstellung erfolgt analog Beispiel 19 ausgehend von 0,75 g (5 mmol) 4-Phenylbutylamin.

Ausb.: 1,05 g (52%); 132°C (Acetonitril).

$C_{24}H_{29}N_5O$ (403,5)

MS: m/z (rel. Int. [%]) = 403 ($M^+$., 6), 109 (17), 105 (100), 95 (17), 91 (30).

$^1$H-NMR-Daten: ($CDCl_3$, TMS als interner Standard) δ = 1.6—2,1 (m) 6H, 2,67 (m) 4H, 3,1—3,8 (m) 4H, 6,74 (s) 1H, 7,1—7,55 (m) 9H, 8.19 (m) 2H, ppm.

Beispiel 39

N-[(3-Imidazol-4-yl)propyl]-N'-(4-phenylbutyl)guanidin

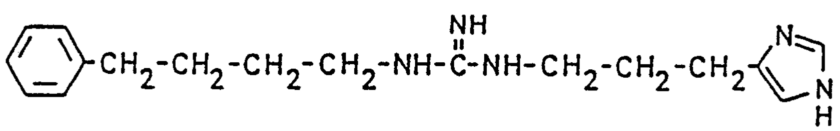

0,8 g (2 mmol) N-Benzoyl-N'-[3-(imidazol-4-yl)propyl]-N''-(4-phenylbutyl)guanidin werden 7 h in 45 ml 20proz. Salzsäure unter Rückfluß erhitz und analog Beispiel 23 aufgearbeitet. Man erhält 0,60 g (81%) stark hygroskopischen, nicht kristallinen Feststoff.

$C_{17}H_{25}N_5 \cdot 2HCl$ (372,3)

MS: m/z (rel. Int. [%]) = 300 ($[M+H]^+$, 100), 109 (83), 91 (88), FAB-Methode).

$^1$H-NMR-Daten: ($d_6$-DMSO, TMS als interner Standard) δ = 1,3—2,2 (m) 6H, 2,4—2,9 (m) 4H, 2,9—3,6 (m) 4H, 7,0—8,4 (m) 10H, 4H austauschbar mit $D_2O$, 9,00 (d) 1H, ppm.

Beispiel 40

N-Benzoyl-N'-[3-(imidazol-4-yl)propyl]-N''-(3-phenylpropyl)guanidin

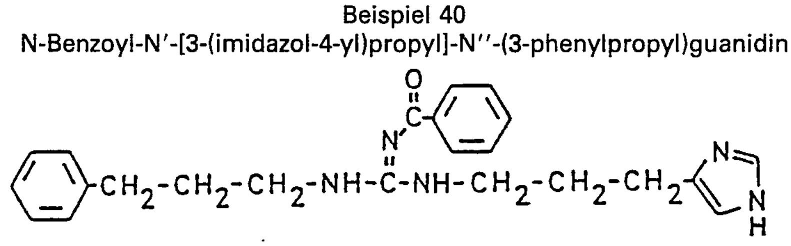

Die Herstellung erfolgt analog Beispiel 19 ausgehend von 0,68 g (5 mmol) 3-Phenylpropylamin.

Ausb.: 1,1 g (56%); Schmp. 146°C (Ethylacetat).

$C_{23}H_{27}N_5O$ (389,5)

MS: m/z (rel. Int. [%]) = 389 ($M^+$., 1), 109 (35), 105 (100), 91 (22), 81 (16).

$^1$H-NMR-Daten: ($CDCl_3$, TMS als interner Standard) δ = 1,90 (m) 2H, 2,04 (tt) 2H, 2,68 (t) 2H, 2,77 (t) 2H, 3,1—3,7 (m) 4H, 6,76 (s) 1H, 7,1—7,55 (m) 9H, 8,18 (m) 2H, ppm.

Beispiel 41

N-[3-(Imidazol-4-yl)propyl]-N'-(3-phenylpropyl)guanidin

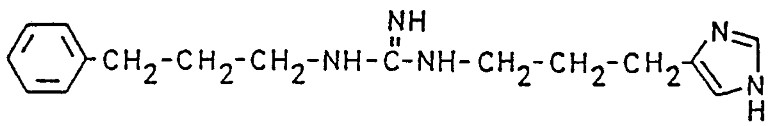

0,73 g (1,9 mmol) N-Benzoyl-N'-[(3-imidazol-4-yl)propyl]-N''-(3-phenylpropyl)guanidin werden in 45 ml 20proz. Salzsäure 7 h unter Rückfluß erhitzt. Die Aufarbeitung erfolgt analog Beispiel 23.

Ausb.: 0,53 g (78%) hygroskopischer, nicht kristalliner Feststoff.

$C_{16}H_{23}N_5 \cdot 2HCl$ (358,3)

MS: m/z (rel. Int. [%]) = 286 $(M+H)^+$., 100), 109 (86), 91 (70), (FAB-Methode).

$^1$H-NMR-Daten: ($d_6$-DMSO, TMS als interner Standard) δ = 1,78 (m) 2H, 1,86 (m) 2H, 2,65 (t) 2H, 2,74 (t) 2H, 3,20 (m) 4H, 7,15—7,4 (m) 5H, 7,50 (m) 1H, 7,65 (s) 2H, austauschbar mit $D_2O$, 8,0—8,2 (m) 2H, austauschbar mit $D_2O$, 9,07 (d) 1H, ppm.

Beispiel 42

N-Benzoyl-N'-[3-(imidazol-4-yl)propyl]-N''-(3,3-diphenylpropyl)guanidin

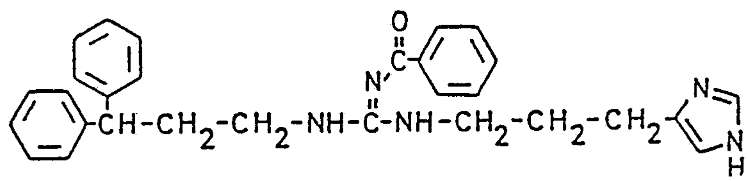

Die Herstellung erfolgt analog Beispiel 19 ausgehend von 1,06 g (5 mmol) 3,3-Diphenylpropylamin in Acetonitril als Lösungsmittel.

Ausb.: 1,2 g (52%); Schmp. 148—149°C (Ethylacetat).

$C_{29}H_{31}N_5O$ (465,6)

MS: m/z (rel. Int. [%]) = 465 ($M^+$., 1), 167 (11), 109 (18), 105 (100), 95 (13)

$^1$H-NMR-Daten: ($CDCl_3$, TMS als interner Standard) δ = 1,86 (m) 2H, 2,44 (dt) 2H, 2,64 (m) 2H, 3,3 (br.) 2H, 3,6 (br.) 2H, 4,06 (t) 1H, 6,72 (s) 1H, 7,15—7,55 (m) 14H, 8,14 (m) 2H, ppm.

Beispiel 43

N-[3-(Imidazol-4-yl)propyl]-N'-(3,3-diphenylpropyl)guanidin

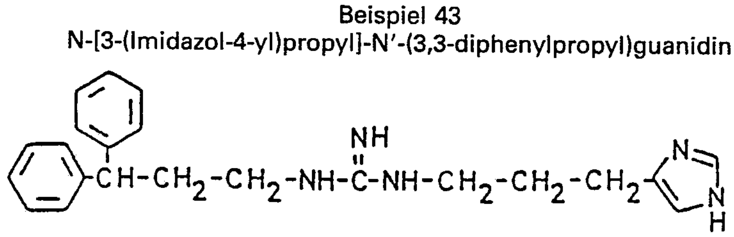

0,84 g (1,8 mmol) N-Benzoyl-N'-[3-(imidazol-4-yl)propyl]-N''(3,3-diphenylpropyl)guanidin werden in 45 ml 20proz. Salzsäure 7 h unter Rückfluß erhitzt. Die Aufarbeitung erfolgt analog Beispiel 23.

Ausb.: 0,67 g (86%) hygroskopischer, nicht kristalliner Feststoff.

$C_{22}H_{27}N_5 \cdot 2HCl$ (434,4)

MS: m/z (rel. Int. [%]) = 362 ([M + $H^+$], 84), 167 (54), 109 (100), 91 (60), (FAB-Methode)

$^1$H-NMR-Daten: ($d_6$-DMSO, TMS als interner Standard) δ = 1,81 (m) 2H, 2,27 (dt) 2H, 2,68 (t) 2H, 3,02 (m) 2H, 3,16 (m) 2H, 4,10 (t) 1H, 7,15—7,6 (m) 13H, 2H, austauschbar mit $D_2O$, 7,80 (m) 2H, austauschbar mit $D_2O$, 8,99 (d) 1H, ppm.

Beispiel 44

N-Benzoyl-N'-[3-(imidazol-4-yl)propyl]-N''-{2-[(4-methylphenyl)thio]ethyl}guanidin

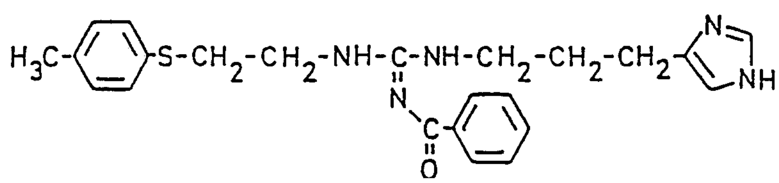

Die Herstellung erfolgt analog Beispiel 19 aus 0,84 g (5 mmol) 2-[(4-Methylphenyl)thio]ethylamin

Ausb.: 1,5 g (71%); Schmp. 151°C (Ethylacetat).

| | | | | |
|---|---|---|---|---|
| $C_{23}H_{27}N_5OS$ (421,6) | Ber.: | C 65,53 | H 6,46 | N 16,61 |
| | Gef.: | C 65,63 | H 6,58 | N 16,64 |

$^1$H-NMR-Daten: ($d_6$-DMSO, TMS als interner Standard) δ = 1,85 (m) 2H, 2,27 (s) 3H, 2,57 (t) 2H, 3,14 (t) 2H, 3,25 (br.) 2H, 3,60 (br.) 2H, 6,81 (S) 1H, 7,13 (d) 2H, 7,3—7,55 (m) 5H, 7,56 (s) 1H, 8,00 (m) 2H, ppm.

Beispiel 45

N-[3-(Imidazol-4-yl)propyl]-N'-{2-[(4-methylphenyl)thio]ethyl}guanidin

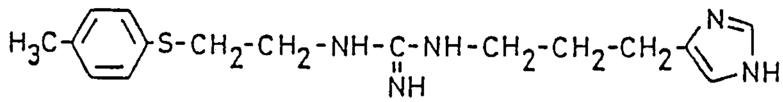

Die Herstellung erfolgt analog Beispiel 23 aus 0,84 g (2 mmol) N-Benzoyl-N'-[3-(imidazol-4-yl)propyl]-N''-{2-[(4-methylphenyl)thio]ethyl}guanidin.

Ausb.: 0,72 g (92%) hygroskopischer, nicht kristalliner Feststoff.

$C_{16}H_{23}N_5S \cdot 2HCl$ (390,4)

MS (FAB-Methode): m/z (rel. Int. [%]) = 318 ([M + H$^+$], 93), 151 (63), 123 (100), 100 (21), (109) (67), 91 (17).

$^1$H-NMR-Daten: ($d_6$-DMSO, TMS als interner Standard) δ = 1,86 (m) 2H, 2,27 (s) 3H, 2,73 (t) 2H, 3,11 (t) 2H, 3,20 (dt) 2H, 3,38 (dt) 2H, 7,15 (d) 2H, 7,31 (d) 2H, 7,48 (s) 1H, 7,67 (s) 2H, austauschbar mit $D_2O$, 7,95 (t) 1H, austauschbar mit $D_2O$, 8,11 (t) 1H, austauschbar mit $D_2O$, 9,06 (s) 1H, 14,8 (br.) 2H, austauschbar mit $D_2O$, ppm.

Beispiel 46

N-Benzoyl-N'-{2-[(4-chlorphenyl)thio]ethyl}-N''-[3-(imidazol-4-yl)propyl]guanidin

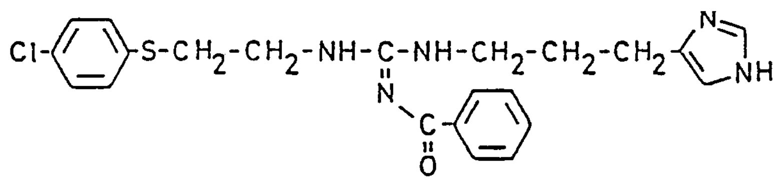

Die Herstellung erfolgt analog Beispiel 19, ausgehend von 0,94 g (5 mmol) 2-[(4-Chlorphenyl)thio]ethylamin.

Ausb.: 1,6 g (72%); Schmp. 152°C (Ethylacetat)

$C_{22}H_{24}ClN_5OS$ (442,0) Ber.: C 59,79 H 5,47 N 15,84

Gef.: C 59,68 H 5,48 N 15,88

$^1$H-NMR-Daten: ($d_6$-DMSO, TMS als interner Standard) δ = 1,86 (m) 2H, 2,58 (t) 2H, 3,21 (t) 2H, 3,3—3,75 (m) 4H, 6,81 (s) 1H, 7,25—7,5 (m) 7H, 7,56 (s) 1H, 7,99 (m) 2H, ppm.

Beispiel 47

N-{2-[(4-Chlorphenyl)thio]ethyl}-N'-[3-(imidazol-4-yl)propyl]guanidin

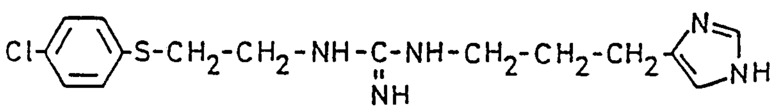

Die Herstellung erfolgt analog Beispiel 23 aus 0,88 g (2 mmol) N-Benzoyl-N'-{2-[(4-chlorphenyl)thio]-ethyl}-N''-[3-(imidazol-4-yl)propyl]guanidin.

Ausb.: 0,76 g (93%) hygroskopischer, nicht kristalliner Feststoff.

$C_{15}H_{20}ClN_5S \cdot 2HCl$ (410,8)

MS (FAB-Methode): m/z (rel. Int. [%]) = 338 ([M + H$^+$], 100), 171 (29), 143 (36), 109 (71)

$^1$H-NMR-Daten: ($d_6$-DMSO, TMS als interner Standard) δ = 1,82 (m) 2H, 2,69 (t) 2H, 3,14 (t) 2H, 3,25—3,6 (m) 4H, 7,40 (m) 4H, 7,46 (s) 1H, 7,59 (s) 2H, austauschbar mit $D_2O$, 7,83 (t) 1H, austauschbar mit $D_2O$, 7,98 (t) 1H, austauschbar mit $D_2O$, 9,03 (s) 1H, 14,8 (br.) 2H, austauschbar mit $D_2O$, ppm.

Beispiel 48

N-Benzoyl-N'-[3-(imidazol-4-yl)propyl]-N''-{3-[(4-methylphenyl)thio]propyl}guanidin

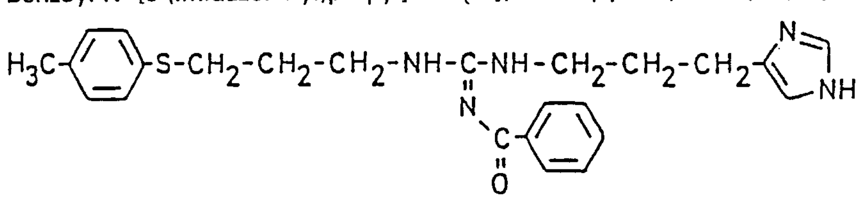

Die Herstellung erfolgt analog Beispiel 19, ausgehend von 0,91 g (5 mmol) 3-[(4-Methylphenyl)thio]propylamin.

Ausb.: 1,4 g (64%); Schmp. 141°C (Ethylacetat).

$C_{24}H_{29}N_5OS$ (435,6 Ber.: C 66,18 H 6,71 N 16,08
Gef.: C 66,37 H 6,80 N 16,11

$^1$H—NMR-Daten ($CDCl_3$, TMS als interner Standard): δ = 1,87 (m) 2H, 1,97 (m) 2H, 2,29 (s) 3H, 2,63 (t) 2H, 2,97 (t) 2H, 3,45 (br.) 2H, 3,65 (br.) 2H, 6,72 (s) 1H, 7,06 (d) 2H, 7,23 (d) 2H, 7,35—7,55 (m) 4H, 8,18 (m) 2H, ppm.

Beispiel 49

N-[3-(Imidazol-4-yl)propyl]-N'-{3-[(4-methylphenyl)thio]propyl}guanidin

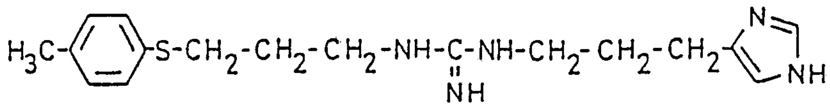

Die Herstellung erfolgt analog Beispiel 23 aus 0,87 g (2 mmol) N-Benzoyl-N'-[3-(imidazol-4yl)propyl]-N''-{3-[(4methylphenyl)thio]propyl}guanidin. Der zunächst erhaltene hygroskopische feste Schaum kristallisiert beim Verreiben mit Aceton.

Ausb.: 0,74 g (91%), Schmp. 152°C.

$C_{17}H_{25}N_5S \cdot 2HCl$ (404,4) Ber.: C 50,49 H 6,73 N 17,32
Gef.: C 50,29 H 6,86 N 17,10

MS (FAB-Methode): m/z (rel. Int. [%]) = 332 ($[M+H]^+$, 100), 165 (13), 151 (10), 137 (37), 123 (30), 109 (80), 100 (30), 91 (13).

$^1$H-NMR-Daten ($d_6$-DMSO, TMS als interner Standard): δ = 1,73 (m) 2H, 1,84 (m) 2H, 2,26 (s) 3H, 2,72 (t) 2H, 2,99 (t) 2H, 3,20 (dt) 2H, 3,28 (dt), 2H, 7,14 (d) 2H, 7,26 (d) 2H, 7,48 (s) 1H, 7,62 (s) 2H, austauschbar mit $D_2O$, 7,98 (t) 1H, austauschbar mit $D_2O$, 8,04 (t) 1H, austauschbar mit $D_2O$, 9,05 (s) 1H, 14,6 (br.) 2H, austauschbar mit $D_2O$, ppm

Beispiel 50

N-Benzoyl-N'-{3-[(4-chlorphenyl)thio]propyl}-N''-[3-(imidazol-4-yl)propyl]guanidin

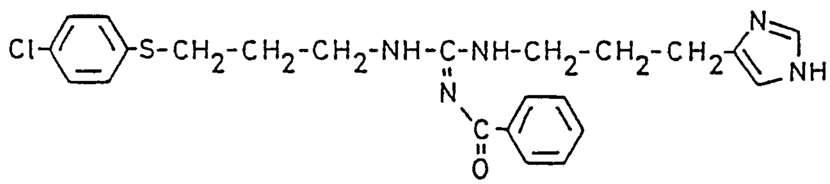

Die Herstellung erfolgt analog Beispiel 19 ausgehend von 1,01 g (5 mmol) 3-[(4-Chlorphenyl)thio]propylamin.

Ausb.: 1,5 g (66%); Schmp. 140°C (Ethylacetat).

$C_{23}H_{26}ClN_5OS$ (456,0) Ber.: C 60,58 H 5,75 N 15,36
Gef.: C 60,51 H 5,78 N 15,29

$^1$H-NMR-Daten ($CDCl_3$, TMS als interner Standard): δ = 1,88 (m) 2H, 1,98 (m) 2H, 2,64 (t) 2H, 2,98 (t) 2H, 3,45 (br.) 2H, 3,65 (br.) 2H, 6,73 (s) 1H, 7,20 (s) 4H, 7,35—7,55 (m) 4H, 8,18 (m) 2H, ppm.

Beispiel 51

N-{3-[(4-Chlorphenyl)thio]propyl}-N'-[3-(imidazol-4-yl)propyl]guanidin

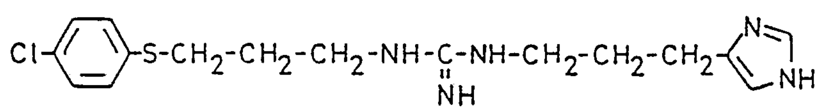

Die Herstellung erfolgt analog Beispiel 23 aus 0,91 g (2 mmol) N-Benzoyl-N'-{3-[(4-Chlorphenyl)thio]propyl}-N''-[3-(imidazol-4-yl)propyl]guanidin. Der zunächst erhaltene hygroskopische Schaum kristallisiert beim Verreiben mit Aceton.

Ausb.: 0,75 g (88%): Schmp. 153°C.

$C_{16}H_{22}ClN_5S \cdot 2HCl$ (424,8) Ber.: C 45,24 H 5,69 N 16,49
Gef.: C 44,98 H 5,79 N 16,19

MS (FAB-Methode): m/z (rel. Int. [%]) = 352 ($[M+H]^+$, 54), 185 (10), 157 (15), 143 (12), 109 (100), 100 (37).

$^1$H-NMR-Daten ($d_6$-DMSO, TMS als interner Standard): δ = 1,76 (m) 2H, 1,84 (m) 2H, 2,72 (t) 2H, 3,05 (t) 2H, 3,19 (dt) 2H, 3,28 (dt) 2H, 7,38 (s) 4H, 7,48 (s) 1H, 7,59 (s) 2H, austauschbar mit $D_2O$, 7,95 (t) 1H, austauschbar mit $D_2O$, 8,00 (t) 1H, austauschbar mit $D_2O$, 9,04 (s) 1H, 14,6 (br.) 2H, austauschbar mit $D_2O$, ppm.

Beispiel 52

N-Benzoyl-N'-[3-(imidazol-4-yl)propyl]-N''-(2-phenoxyethyl)guanidin

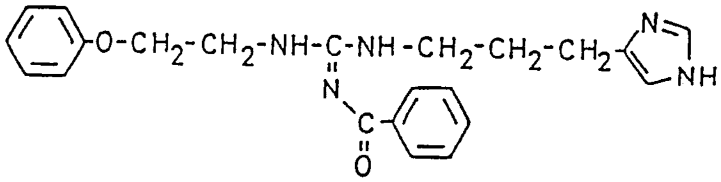

Die Herstellung erfolgt analog Beispiel 19 aus 0,69 g (5 mmol) 2-Phenoxyethylamin.
Ausb.: 1,1 g (56%); Schmp. 144°C (Ethylacetat).
$C_{22}H_{25}N_5O_2$ (391,5) Ber.: C 67,50 H 6,44 N 17,89
Gef.: C 67,33 H 6,40 N 17,86
$^1$H-NMR-Daten ($CDCl_3$, TMs als interner Standard): δ = 1,92 (m) 2H, 2,66 (t) 2H, 3,25—4,1 (m) 4H, 4,19 (t) 2H, 6,73 (s) 1H, 6,8—7,05 (m) 3H, 7,2—7,5 (m) 6H, 8,19 (m) 2H, ppm.

Beispiel 53

N-[3-(Imidazol-4-yl)propyl]-N'-(2-phenoxyethyl)guanidin

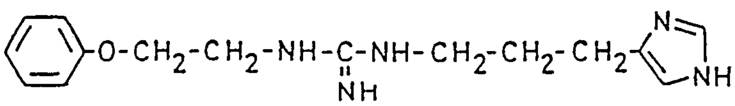

Die Herstellung erfolgt analog Beispiel 23 aus 0,8 g (2 mmol) N-Benzoyl-N'-[3-(imidazol-4-yl)propyl]-N''-(2-phenoxyethyl)guanidin.
Ausb.: 0,66 (90%) hygroskopischer, nicht kristalliner Feststoff.
$C_{15}H_{21}N_5O \cdot 2HCl$ (360,3)
MS (FAB-Methode): m/z (rel. Int. ([%]) = 288 ([M+H]$^+$, 100), 180 (5), 151 (5), 109 (77), 100 (16).
$^1$H-NMR-Daten ($d_6$-DMSO, TMS als interner Standard): δ = 1,86 (m) 2H, 2,73 (t) 2H, 3,22 (dt) 2H, 3,61 (dt) 2H, 4,07 (t) 2H, 6,9—7,05 (m) 3H, 7,47 (m) 2H, 7,47 (s) 1H, 7,72 (s) 2H, austauschbar mit $D_2O$, 7,95 (t) 1H, austauschbar mit $D_2O$, 8,16 (t) 1H, austauschbar mit $D_2O$, 9,05 (s) 1H, 14,6 (br.) 2H, austauschbar mit $D_2O$, ppm.

Beispiel 54

N-Benzoyl-N'-[3-(imidazol-4-yl)propyl]-N''-(1-methyl-2-phenoxyethyl)guanidin

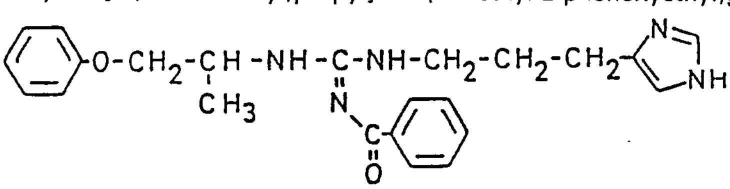

Die Herstellung erfolgt analog Beispiel 19, ausgehend von 0,76 (5 mmol) 1-Methyl-2-phenoxyethylamin.
Aus.: 1,2 g (59%); Schmp. 122°C (Ethylacetat/Ether).
$C_{23}H_{27}N_5O_2$ (405,5) Ber.: C 68,13 H 6,71 N 17,27
Gef.: C 67,99 H 6,72 N 17,23
$^1$H-NMR-Daten ($CDCl_3$, TMS als interner Standard): δ = 1,43 (d) 3H, 1,91 (m) 2H, 2,66 (t) 2H, 3,50 (dt) 2H, 4,03 (d) 2H, 4,5 (br.) 1H, 6,72 (s) 1H, 6,8—7,05 (m) 3H, 7,2—7,5 (m) 6H, 8,18 (m) 2H, ppm.

Beispiel 55

N-[3-(Imidazol-4-yl)propyl]-N'-(1-methyl-2-phenoxyethyl)guanidin

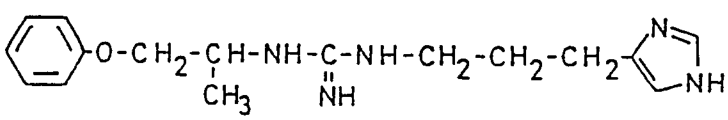

Die Herstellung erfolgt analog Beispiel 23 aus 0,81 g (2 mmol) N-Benzoyl-N'-[3-(imidazol-4-yl)propyl]-N''-(1-methyl-2-phenoxyethyl)guanidin.
Ausb.: 0,68 g (91%) hygroskopischer nicht kristalliner Feststoff.
$C_{16}H_{23}N_5O \cdot 2HCl$ (374,3)
MS (FAB-Methode): m/z (rel. Int. [%]) = 302 ([M+H]$^+$, 93), 194 (9), 151 (9), 135 (7), 126 (20), 109 (100).
$^1$H-NMR-Daten ($d_6$-DMSO, TMS als interner Standard): δ = 1,23 (d) 3H, 1,85 (m) 2H, 2,72 (t) 2H, 3,22 (dt) 2H, 3,96 (d) 2H, 4,17 (m) 1H, 6,9—7,05 (m) 3H, 7,29 (m) 2H, 7,47 (s) 1H, 7,70 (s) 2H, austauschbar mit $D_2O$,

7,88 (d) 1H, austauschbar mit $D_2O$, 8,11 (t) 1H, austauschbar mit $D_2O$, 9,05 (s) 1H, 14,6 (br.) 2H, austauschbar mit $D_2O$, ppm.

Beispiel 56
N-Benzoyl-N'-[3-(imidazol-4-yl)propyl]-N''-(1-methyl-2-benzylthioethyl)guanidin

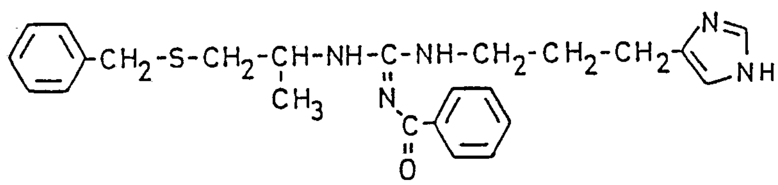

Die Herstellung erfolgt analog Beispiel 19, ausgehend von 0,91 g (5 mmol) 1-Methyl-2-benzylthioethylamin.

Ausb.: 1,3 g (60%) zähes Öl.

$C_{24}H_{29}N_5OS$ (435,6) Ber.: C 66,18 H 6,71 N 16,08
Gef.: C 66,01 H 6,88 N 15,71

$^1$H-NMR-Daten ($CDCl_3$, TMS als interner Standard): δ = 1,31 (d) 3H, 1,89 (m) 2H, 2,65 (m) 4H, 3,2—3,8 (m) 3H, 3,73 (s) 2H, 6,72 (s) 1H, 7,15—7,55 (m) 9H, 8,19 (m) 2H, ppm.

Beispiel 57
N-[3-(Imidazol-4-yl)propyl]-N'-(1-methyl-2-benzylthioethyl)guanidin

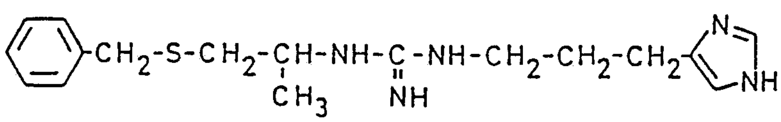

Die Herstellung erfolgt analog Beispiel 23 aus 0,87 g (2 mmol) N-Benzoyl-N'-[3-imidazol-4-yl)propyl]-N''-(1-methyl-2-benzylthioethyl)guanidin.

Ausb.: 0,69 g (85%) hygroskopischer, nicht kristalliner Feststoff.

$C_{17}H_{25}N_5S \cdot 2HCl$ (404,4)

MS (FAB-Methode): m/z (rel. Int. [%]) = 332 ([M+H]$^+$, 22), 109 (42), 91 (100).

$^1$H-NMR-Daten ($d_6$-DMSO, TMS als interner Standard): δ = 1,17 (d) 3H, 1,87 (m) 2H, 2,5—2,9 (m) 4H, 3,22 (dt) 2H, 3,82 (s) 2H, 4,04 (m) 1H, 7,2—7,6 (m) 6H, 7,68 (s) 2H, austauschbar mit $D_2O$, 7,78 (d) 1H, austauschbar mit $D_2O$, 8,08 (m) 1H, austauscher mit $D_2O$, 9,05 (s) 1H, 14,6 (br.) 2H, austauschbar mit $D_2O$, ppm.

Beispiel 58
N-Benzoyl-N'-[3-(imidazol-4-yl)propyl]-N''-{-[(naphth-2-yl)methylthio]ethyl]guanidin

Herstellung der Vorstufe
N-Benzoyl-N'-{-[(naphth-2-yl)methylthio]ethyl}-O-phenyl-isoharnstoff

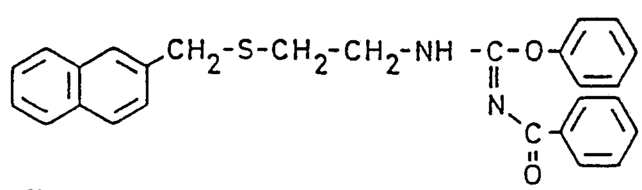

1,63 g (7,5 mmol) 2-[(Naphth-2-yl)methylthio]ethylamin werden mit 2,38 g (7,5 mmol) N-Benzoyl-diphenylimidocarbonat in 30 ml Methylenchlorid 20 min bei Raumtemperatur gerührt. Anschließend wird das Lösungsmittel i. Vak. abdestilliert und der Rückstand aus Ethanol/Ether kristallisiert.

Ausb.: 3,07 g (93%); Schmp. 123°C.

$C_{27}H_{24}N_2O_2S$ (440,6) Ber.: C 73,61 H 5,49 N 6,36
Gef.: C 73,78 H 5,58 N 6,40

$^1$H-NMR-Daten ($CDCl_3$, TMS als interner Standard): δ = 2,73 (t) 2H, 3,67 (dt) 2H, 3,96 (s) 2H, 7,09 (m) 2H, 7,25—8,0 (m) 15H, 10,35 (t) 1H, austauscher mit $D_2O$, ppm.

N-Benzoyl-N'-[3-(imidazol-4-yl)propyl]-N''-{2-[(naphth-2-yl)methylthio]ethyl}guanidin

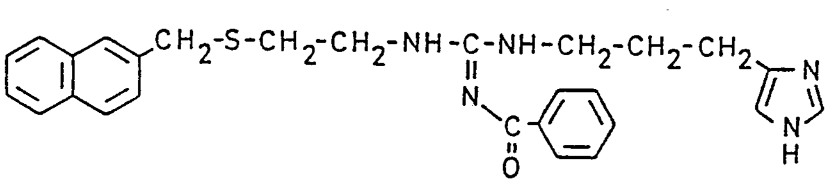

2,2 g (5 mmol) N-Benzoyl-N'-{2-[(naphth-2-yl)methylthio]ethyl}-O-phenyl-isoharnstoff werden mit 0,69 g (5,5 mmol) 3-(Imidazol-4-yl)propylamin 1 h in 40 ml Pyridin unter Rückfluß erhitzt. Der Reaktionsansatz wird analog Beispiel 19 aufgearbeitet. Der beim Einengen des Methylenchloridextraktes ausfallende Feststoff wird aus Methanol umkristallisiert.

Ausb.: 1,6 g (77%); Schmp. 137°C.

$C_{27}H_{29}N_5OS$ (471,6) Ber.: C 68,76 H 6,20 N 14,85
Gef.: C 68,61 H 6,22 N 14,75

$^1$H-NMR-Daten ($CDCl_3$, TMS als interner Standard): δ = 1,86 (m) 2H, 2,62 (t) 2H, 2,71 (t) 2H, 3,34 (br.) 2H, 3,70 (br.) 2H, 3,91 (s) 2H, 6,71 (s) 1H, 7,3—7,55 (m) 7H, 7,6—7,85 (m) 4H, 8,21 (m) 2H, ppm.

## Beispiel 59
N-[3-(Imidazol-4-yl)propyl]-N'-{2-[(naphth-2-yl)methylthio]ethyl}guanidin

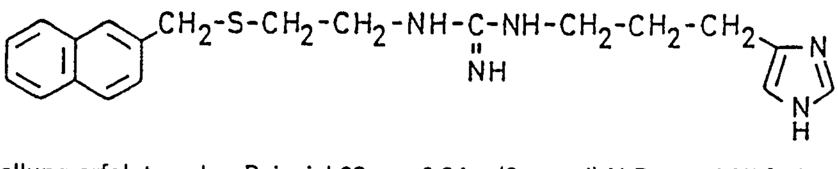

Die Herstellung erfolgt analog Beispiel 23 aus 0,94 g (2 mmol) N-Benzoyl-N'-[3-(imidazol-4-yl)propyl]-N''-{2-[(naphth-2-yl)methylthio]ethyl}guanidin.

Ausb.: 0,77 g (87%) hygroskopischer, nicht kristalliner Feststoff.

$C_{20}H_{25}N_5S \cdot 2HCl$ (440,4)

MS (FAB-Methode): m/z (rel. Int. [%]) = 368 ([M+H]$^+$, 26), 141 (100), 109 (51), 100 (15).

$^1$H-NMR-Daten ($d_6$-DMSO, TMS als interner Standard): δ 1,85 (m) 2H, 2,55 (t) 2H, 2,72 (t) 2H, 3,20 (dt) 2H, 3,43 (dt) 2H, 3,99 (s) 2H, 7,45—7,55 (m) 4H, 7,67 (s) 2H, austauschbar mit $D_2O$, 7,85—8,0 (m) 5H, 1H austauschbar mit $D_2O$, 8,09 (t) 1H, austauschbar mit $D_2O$, 9,05 (s) 1H, 14,6 (br.) 2H, austauschbar mit $D_2O$, ppm.

## Beispiel 60
N-Benzoyl-N'-[3-(5-methylimidazol-4-yl)propyl]-N''-(3,3-diphenylpropyl)guanidin

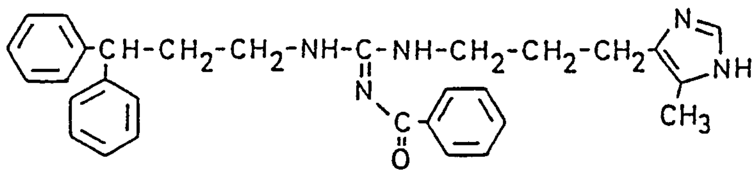

1,06 g (5 mmol) 3,3-Diphenylpropylamin und 1,59 g (5 mmol) N-Benzoyl-diphenylimidocarbonat werden in 20 ml Methylenchlorid 15 min bei Raumtemperatur gerührt. Anschließend wird das Lösungsmittel i. Vak. abdestilliert, der Rückstand in 30 ml Pyridin aufgenommen und unter Zusatz von 0,77 g (5,5 mmol) 3-(5-Methylimidazol-4-yl)propylamin 1 h unter Rückfluß erhitzt. Die Isolierung und Reinigung des Reaktionsproduktes erfolgt analog Beispiel 19.

Ausb.: 1,1 g (46%) trockener Schaum.

$C_{30}H_{33}N_5O$ (479,6)

MS: m/z (rel. Int. [%]) = 479 (M$^+$, 12), 312 (8), 167 (12), 109 (27) 105 (100), 95 (17), 77 (45).

$^1$H-NMR-Daten ($CDCl_3$, TMS als interner Standard): δ = 1,83 (m) 2H, 2,12 (s) 3H, 2,2—2,8 (m) 4H, 3,0—3,6 (m) 4H, 4,08 (t) 1H, 6,9—7,6 (m) 14H, 8,13 (m) 2H, ppm.

## Beispiel 61
N-[3-(5-Methylimidazol-4-yl)propyl]-N'-(3,3-diphenylpropyl)guanidin

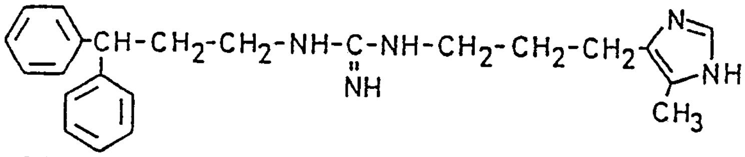

Die Herstellung erfolgt analog Beispiel 23 aus 0,77 g (1,5 mmol) N-Benzoyl-N'-[3-(5-methylimidazol-4-yl)propyl]-N''-(3,3-diphenylpropyl)guanidin.

Ausb.: 0,57 g (85%) hygroskopischer, nicht kristalliner Feststoff.

$C_{23}H_{29}N_5 \cdot 2HCL$ (448,4)

MS (FAB-Methode): m/z (rel. Int. [%]) = 376 ([M+H]$^+$, 100), 254 (14), 208 (4), 167 (26), 123 (76), 100 (30), 91 (28).

$^1$H-NMR-Daten ($d_6$-DMSO, TMS als interner Standard): δ 1,80 (m) 2H, 2,22 (s) 3H, 2,05—2,9 (m) 4H, 2,9—3,5 (m) 4H, 4,16 (t) 1H, 7,1—7,4 (m) 10H, 7,51 (s) 2H, austauschbar mit $D_2O$, 7,98 (m) 2H, austauschbar mit $D_2O$, 8,90 (s) 1H, 14,6 (br.) 2H, austauschbar mit $D_2O$, ppm.

Beispiel 62

N-[3-(Imidazol-4-yl)propyl]-N'-(4,4-diphenylbutyl)guanidin

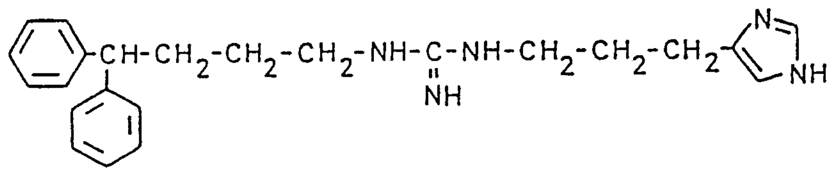

Die Herstellung erfolgt analog Beispiel 23, ausgehend von 0,96 (2 mmol) N-Benzoyl-N'-[3-(imidazol-4-yl)propyl]-N''-(4,4-diphenylbutyl)guanidin.

Ausb.: 0,76 g (85%) hygroskopischer, nicht kristalliner Feststoff.

$C_{23}H_{29}N_5 \cdot 2HCl$ (448,4)

$^1$H-NMR-Daten ($d_6$-DMSO, TMS als interner Standard): δ 1,3—2,1 (m) 4H, 2,2 (m) 2H, 2,73 (t) 2H, 2,9—3,5 (m) 4H, 4,08 (t) 1H, 7,15—7,6 (m) 13H, 2H austauschbar mit $D_2O$, 7,7—8,1 (m) 2H, austauschbar mit $D_2O$ 9,5 (s) 1H, 14,6 (br.) 2H, austauschbar mit $D_2O$, ppm.

Beispiel 63

N-[3-(Imidazol-4-yl)propyl]-N'-{2-[(3-trifluormethylphenyl)methylthio]ethyl}guanidin

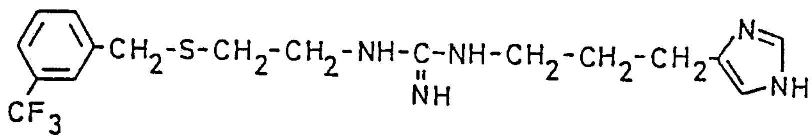

Die Herstellung erfolgt analog Beispiel 23, ausgehend von 0,69 g (1,4 mmol) N-Benzoyl-N'-[3-(imidazol-4-yl)propyl]-N''-{2-[(3-trifluormethylphenyl)methylthio]ethyl}guanidin

Ausb.: 0,59 g (92%) hygroskopischer, nicht kristalliner Feststoff.

$C_{17}H_{22}F_3N_5S \cdot 2HCl$ (458,4)

$^1$H-NMR-Daten ($d_6$-DMSO, TMS als interner Standard): δ 1,85 (m) 2H, 2,59 (t) 2H, 2,73 (t) 2H, 3,1—3,6 (m) 4H, 3,92 (s) 2H, 7,4—8,1 (m) 9H, 4H austauschbar mit $D_2O$, 9,05 (s) 1H, 14,6 (br.) 2H, austauschbar mit $D_2O$, ppm.

Beispiel 64

N-[3-(Imidazol-4-yl)propyl]-N'-{2-[(4-methoxyphenyl)methylthio]ethyl}guanidin

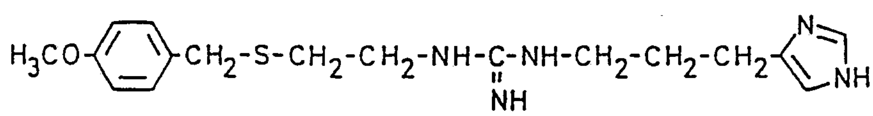

Die Herstellung erfolgt analog Beispiel 23, ausgehend von 0,54 g (1,2 mmol) N-Benzoyl-N'-[3-(imidazol-4-yl)propyl]-N''-{2-[(4-methoxyphenyl)methylthio]ethyl}guanidin.

Ausb.: 0,11 g (22%) hygroskopischer, nicht kristalliner Feststoff.

$C_{17}H_{25}N_5OS \cdot 2HCL$ (420,4)

$^1$H-NMR-Daten ($d_6$-DMSO, TMS als interner Standard): δ 1,83 (m) 2H, 2,59 (t) 2H, 2,73 (t) 2H, 3,1—3,6 (m) 4H, 3,7 (s) 5H, 6,88 (d) 2H, 7,29 (d) 2H, 7,43 (s) 1H, 7,6 (s) 2H, austauschbar mit $D_2O$, 7,7—8,0 (m) 2H, austauschbar mit $D_2O$ 9,05 (s) 1H, 14,6 (br) 2H, austauschbar mit $D_2O$, ppm.

Beispiel 65

$N^1$-Benzoyl-$N^2$-[3-(4-imidazolyl)propyl]-$N^3$-[2-(N-benzyl-N-phenylamino)ethyl]-guanidin

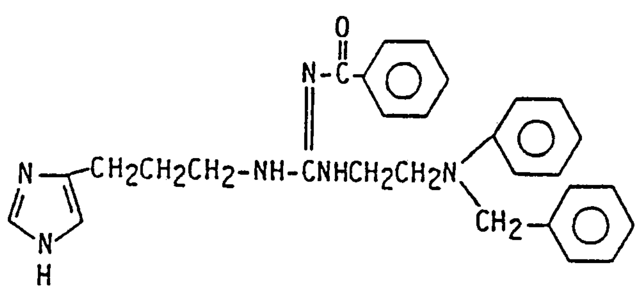

Eine Mischung von 3.48 g (10 mmol) $N^1$-Benzoyl-$N^2$-[3-(4-imidazolyl)propyl]-O-phenyl-isoharnstoff und 2.26 g (10 mmol) N-Benzyl-N-phenyl-ethylendiamin in 50 ml Ethanol wird 17 h unter Rückfluß gekocht. Der

nach Einrotieren erhaltene Rückstand wird mit Essigester/Ethanol (80:20) an Kieselgel chromatographiert. Die Hauptfraktikon ergibt nach Eindampfen 3,14 g (65%) $N^1$-Benzoyl-$N^2$-[3-(4-imidazolyl)propyl]-$N^3$-[2-(N-benzyl-N-phenylamino)ethyl]guanidin als farblosen Feststoff. Nach Umkristallisieren aus Essigester farblose Kristalle vom Schmp. 148,1—149,5°C.

$C_{29}H_{32}N_6O$ (480,62)

$^1$H-NMR-Daten ($CD_3OD$, TMS als interner Standard) δ = 1,88 (m) 2 H, 2,63 (t) 2H, 3,20 (t) 2H, 3,58—3,72 (m) 4H, 4,59 (s) 2H, 4,8 (breit) 3H, austauschbar mit $D_2O$, 6,50—7.58 (m) 15H, 8,04—8,21 (m) 2H, ppm.

Beispiel 66

$N^1$-[3-(4-Imidazolyl)propyl]-$N^2$-[2-(N-benzyl-N-phenylamino)-ethyl]-guanidin-trihydrochlorid

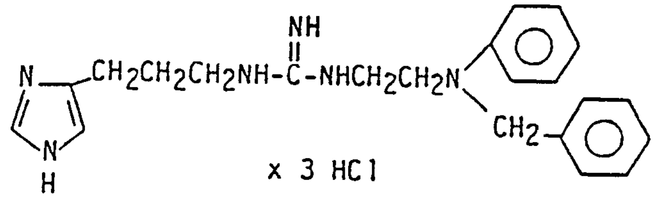

1,60 g (3,3 mmol) $N^1$-Benzoyl-$N^2$-[3-(4-imidazolyl)propyl]-$N^3$-[2-(N-benzyl-N-phenylamino)ethyl]-guanidin (Beispiel 65) werden in 30 ml konz. Salzsäure 14 h gekocht. Nach Abkühlen wird auf ein Drittel eingeengt und die erhaltene wäßrige Lösung dreimal mit 30 ml Ether extrahiert. Die wäßrige Phase wird dann filtriert und im Vakuum eingedampft. Der Rückstand wird zweimal mit 20 ml Ethanol aufgenommen und wiederum eingedampft. Der danach verbleibende Rückstand wird aus abs. Ethanol umkristallisiert. Man erhält 0,92 g (57%) der Titelverbindung als farblosen, hygroskopischen Feststoff.

$C_{22}H_{31}Cl_3N_6$ (485.89)

$^1$H-NMR-Daten ($CD_3OD$, TMS als interner Standard): δ = 1,79—2,20 (m) 2H, 2,88 (t) 2H, 3,32 (t) 2H, 3,60 (m) 2H, 4,13 (t) 2H, 4,83 (s) 2H, 4,9 (breit) 7H, austauschbar mit $D_2O$, 7,28—7,90 (m) 11H, 9,02 (s) 1H, ppm.

Beispiel 67

$N^1$-[3-(4-Imidazolyl)propyl]-$N^2$-[2-(N-benzyl-N-(4-fluor-phenyl)amino)ethyl]-guanidin-trihydrochlorid

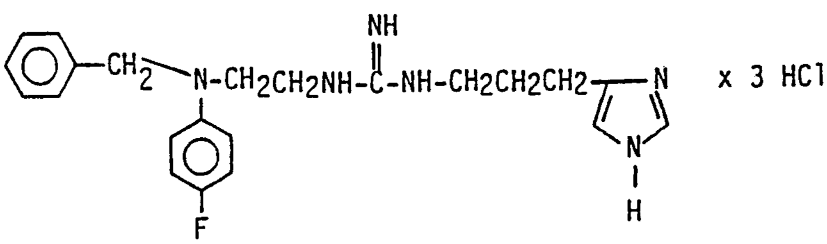

Die Herstellung erfolgt analog Beispiel 66.

$C_{22}H_{30}Cl_3FN_6$ (503,88)

$^1$H-NMR-Daten ($CD_3OD$, TMS als interner Standard): δ = 1,80—2,21 (m) 2H, 2,88 (t) 2H, 3,30 (t) 2H, 3,59 (m) 2H, 4,12 (t) 2H, 4,83 (s) 2H, 4,9 (breit) 7H, 7,14—7,86 (m) 10H, 9,01 (s) 1H ppm.

Beispiel 68

$N^1$-[3-(4-Imidazolyl)propyl]-$N^2$-[2-(N-benzyl-N-(4-chlorphenyl)amino)ethyl]-guanidin-trihydrochlorid

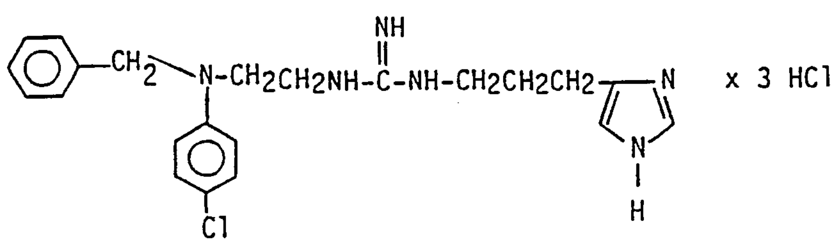

Die Herstellung erfolgt analog Beispiel 66.

$C_{22}H_{30}Cl_4N_6$ (520,33)

$^1$H-NMR-Daten ($CD_3OD$, TMS als interner Standard): δ = 1,79—2,18 (m) 2H, 2,87 (t) 2H, 3,31 (t) 2H, 3,58 (m) 2H, 4,11 (t) 2H, 4,81 (s) 2H, 4,9 (breit) 7H, 7,24—7,89 (m) 10H, 8,99 (s) 1H ppm.

Beispiel 69

$N^1$-[3-(4-Imidazolyl)propyl]-$N^2$-[2-(N-benzyl-N-(4-bromophenyl)amino)ethyl]-guanidin-trihydrochlorid

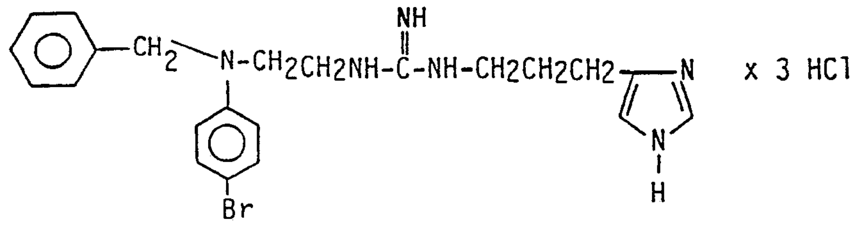

Die Herstellung erfolgt analog Beispiel 66.

$C_{22}H_{30}BrCl_3N_6$ (564,78)

$^1$H-NMR-Daten ($CD_3OD$, TMS als interner Standard): δ = 1,81—2,20 (m) 2H, 2,87 (t) 2H, 3,28 (t) 2H, 3,56 (m) 2H, 4,12 (t) 2H.

Beispiel 70

N-[3-(Imidazol-4-yl)propyl]-N'-[2-(2-thenylthio)ethyl]guanidin

Herstellung der Vorstufen

a) N-Benzoyl-N'-[2-(2-thenylthio)ethyl]thioharnstoff

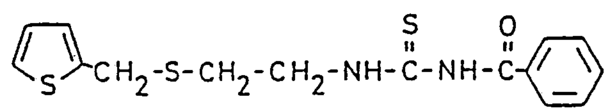

1,73 g (10 mmol) 2-(2-Thenylthio)ethylamin und 1,63 g (10 mmol) Benozylisothiocyanat werden in 120 ml Chloroform 30 min unter Rückfluß erhitzt. Anschließend wird das Lösungsmittel i. Vak. abdestilliert und der ölige Rückstand mit Ether zur Kristallisation gebracht.

Ausb.: 3,06 g (91%); Schmp. 85°C (Ether).

$C_{15}H_{16}N_2OS_3$ (336,5) Ber.: C 53,34 H 4,79 N 8,32

Gef.: C 53,54 H 4,79 N 8,17

MS: m/z (rel. Int. [%]) = 336 ($M^{+\cdot}$, 1) 239 (94), 105 (95), 97 (100).

$^1$H-NMR-Daten ($CDCl_3$, TMS als interner Standard): δ = 2,84 (t) 2H, 3,91 (dt) 2H, 4,03 (s) 2H, 6,9—7,05 (m) 2H, 7.24 (dd) 1H, 7,53 (m) 2H, 7,62 (m) 1H, 7,86 (m) 2H, 9,06 (br.) 1H, austauschbar mit $D_2O$, 10,98 (br.) 1H, austauschbar mit $D_2O$, ppm.

b) S-Methyl-N-[2-(2-thenylthio)ethyl]isothiouroniumiodid

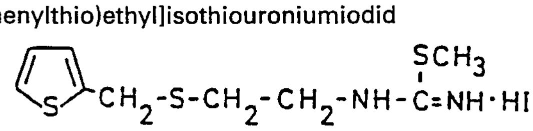

2,52 g (7,5 mmol) N-Benzoyl-N'-[2-(2-thenylthio)ethyl]thioharnstoff werden mit 2,1 g Kaliumcarbonat in einer Mischung aus 30 ml Wasser und 100 ml Methanol 40 min unter Rückfluß erhitzt. Anschließend wird i. Vak. eingedampft, der Rückstand in Ether auf genommen und 3mal mit Wasser gewaschen. Die organische Phase wird über Natriumsulfat getrocknet, i. Vak. eingedampft, der ölige Rückstand in 100 ml Ethanol aufgenommen und nach Zusatz von 0,6 ml Methyliodid über Nacht bei Raumtemperatur gerührt. Das Lösungsmittel wird i. Vak. abdestilliert und der Rückstand mit Aceton und Ether verührt. Man erhält 1,97 g (70%) Isothiouroniumsalz als farblosen Feststoff vom Schmp. 80—81°C.

$C_9H_{14}N_2S_3 \cdot HI$ (374,3) Ber.: C 28,88 H 4,40 N 7,48

Gef.: C 28,80 H 4,06 N 7,43

Molmasse (MS): Ber.: 246,03192; Gef.: 246,03186

MS: m/z (rel. Int. [%]) = 246 ($M^{+\cdot}$, 6), 149 (100), 97 (96).

$^1$H-NMR-Daten ($d_6$-DMSO, TMS als interner Standard) δ = 2,61 (s) 3H, 2,67 (t) 2H, 3,52 (t) 2H, 4,06 (s) 2H, 6,97 (m) 1H, 7,02 (m) 1H, 7,46 (dd) 1H, 9,2 (br.) 3H, austauschbar mit $D_2O$, ppm.

N-[3-(Imidazol-4-yl)propyl]-N'-[2-(2-thenylthio)ethyl]guanidin

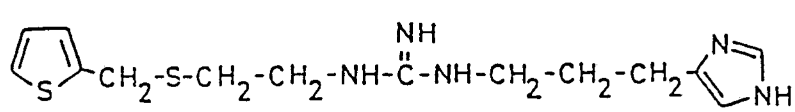

1,87 g (5 mmol) S-Methyl-N-[2-(2-thenylthio)ethyl]isothioureoniumiodid und 0,69 g (5,5 mmol) 3-(Imidazol-4-yl)propylamin werden in 40 ml wasserfreiem Pyridin 3 h unter Rückfluß erhitzt. Nach dem Eindampfen i. Vak. wird das Reaktionsprodukt durch präparative Schichtchromatographie isoliert und

gereinigt (Kieselgel 60 $PF_{254}$, gips haltig; Fließmittel: Chloroform/Methanol, 85 + 15, Ammoniakatmosphäre). Man erhält 1,53 g (68%) N-[3-(Imidazol-4-yl)propyl]-N'-[2-(2-thenylthio)ethyl]guanidin-hydroiodid als zähes Öl.

$C_{14}H_{21}N_5S_2 \cdot HI$ (451,4)

Molmasse (MS): Ber.: 323,12384; Gef.: 323,12405

MS: m/z (rel. Int. [%]) = 323 ($M^+$., 4), 198 (11), 128 ($[HI]^+$, 23), 97 (100).

$^1$H-NMR-Daten ($d_6$-DMSO TMS als interner Standard): δ = 1,80 (m) 2H, 2,35—2,9 (m) 4H, 2,9—3,6 (m) 4H, 4,06 (s) 2H, 6,8—7,15 (m) 3H, 7,2—7,8 (m) 5H, 4H austauschbar mit $D_2O$, 8,07 (d) 1H, ppm.

Das Dipikrat schmilzt nach Umkristallisation aus Ethanol bei 123°C.

| | | | | |
|---|---|---|---|---|
| $C_{14}H_{21}N_5S_2 \cdot 2C_6H_3N_3O_7 \cdot 1/2C_2H_5OH$ (804,7) | Ber.: | C 40,30 | H 3,76 | N 19,15 |
| | Gef.: | C 40,21 | H 3,73 | N 19,25 |

## Beispiel 71

N-Benzoyl-N'-[3-(imidazol-4-yl)propyl]-N''-[2-(2-thenylthio)ethyl]guanidin

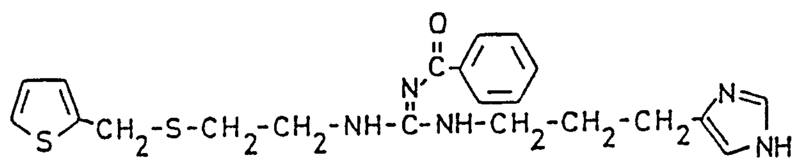

0,87 g (5 mol) 2-(2-Thenylthio)ethylamin und 1,59 g (5 mmol) N-Benzoyl-diphenylimidocarbonat werden in 20 ml Methylenchlorid 15 min bei Raumtemperatur gerührt. Anschließend wird das Lösungsmittel i. Vak. abdestilliert, der Rückstand in 30 ml. Pyridin aufgenommen und nach Zusatz von 0,69 g (5,5 mmol) 3-(Imidazol-4-yl)propylamin 60 min unter Rückfluß erhitzt. Der Reaktionsansatz wird i. Vak. eingedampft, der Rückstand in verdünnter Salzsäure gelöst und zur Entfernung des gebildeten Phenols mit Ether extrahiert. Nach Alkalisieren der wäßrigen Phase mit Ammoniak wird mit Methylenchlorid extrahiert, die organische Phase mit Wasser gewaschen, über Natrium sulfat getrocknet und i. Vak. eingedampft. Das Rohprodukt wird durch präparative Schichtchromatographie gereinigt (Kieselgel 60 $PF_{254}$, gipshaltig; Fließmittel: Chloroform/Methanol, 99 + 1, Ammoniakatmosphäre). Nach Kristallisation aus Ethylacetat erhält man 1,55 g (72%) farblose Kristalle vom Schmp. 128—129°C.

| | | | | |
|---|---|---|---|---|
| $C_{21}H_{25}N_5OS_2$ (427,6) | Ber.: | C 58,99 | H 5,89 | N 16,38 |
| | Gef.: | C 58,91 | H 5,93 | N 16,29 |

$^1$H-NMR-Daten ($d_6$-DMSO, TMS als interner Standard): δ = 1,95 (m) 2H, 2,68 (m) 2H, 2,80 (t) 2H, 3,0—3,7 (m) 4H, 4,02 (s) 2H, 6,7—7,05 (m) 3H, 7,25—7,7 (m) 5H, 8,13 (m) 2H, ppm.

## Beispiel 72

N-Benzoyl-N'-{2-[(chinolin-2-yl)methylthio]ethyl}-N''-[3-(imidazol-4-yl)propyl]guanidin

Herstellung der Vorstufe

2-[(Chinolin-2-yl)methylthio]ethylamin

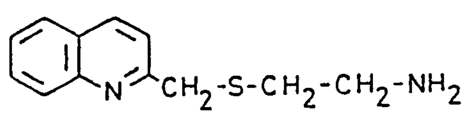

4,28 g (20 mmol) 2-Chlormethylchinolin·HCl und 2,27 g (20 mmol) Cysteamin·HCl werden 5 h in 50 ml 48proz. wäßriger Bromwasserstoffsäure unter Rückfluß erhitzt. Anschließend wird i. Vak. zur Trockne eingedampft und der Rückstand aus Ethanol/Wasser umkristallisiert.

Ausb.: 5,5 g (72%); Schmp. 207—209°C.

| | | | | |
|---|---|---|---|---|
| $C_{12}H_{14}N_2S \cdot 2HBr$ (380,2) | Ber.: | C 37,92 | H 4,24 | N 7,37 |
| | Gef.: | C 37,65 | H 4,26 | N 7,31 |

MS: m/z (rel. Int. [%]) = 218 ($M^+$., 3), 143 (95), 142 (90), 80 (100), 77 (38).

$^1$H-NMR-Daten ($d_6$-DMSO, TMS als interner Standard): δ = 2,6—3,3 (m) 4H, 4,40 (s) 2H, 7,65—8,4 (m) 5H, 9,01 (d) 1H, ppm.

N-Benzoyl-N'-{2-[(chinolin-2-yl)methylthio]ethyl}-N''-[3-(imidazol-4-yl)propyl]guanidin

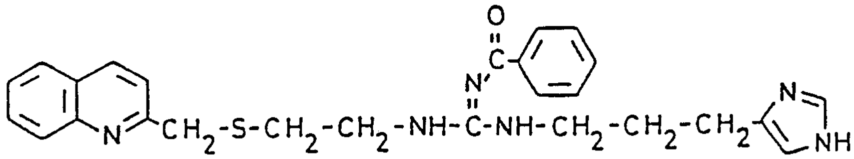

Die Herstellung erfolgt analog Beispiel 71 ausgehend von 1,09 g (5 mmol) 2-[(Chinol-2-yl)methylthio]ethylamin, dargestellt aus dem Dihydrobromid mit 10 mmol Natriumethalat in Ethanol.

Ausb.: 1,77 g (75%), Schmp. 120—122°C (Ethylacetat).

$C_{26}H_{28}N_6OS$ (472,6) Ber.: C 66,08 H 5,97 N 17,78

Gef.: C 65,89 H 6,04 N 17,78

$^1$H-NMR-Daten ($d_6$-DMSO, TMS als interner Standard):δ = 1,88 (m) 2H, 2,73 (t) 2H, 2,86 (t) 2H, 3,43 (dt) 2H, 3,67 (dt) 2H, 4,08 (s) 2H, 6,80 (s) 1H, 7,3—8,4 (m) 12H, ppm.

## Beispiel 73

N-{2-[(Chinolin-2-yl)methylthio]ethyl}-N'-[3-(imidazol-4-yl)propyl]guanidin

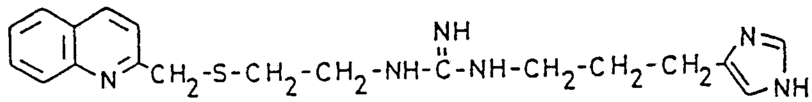

0,95 g (2 mmol) N-Benzoyl-N'-{2-[(chinolin-2-yl)methylthio]ethyl}-N''-[3-(imidazol-4-yl)propyl]guanidin werden 6 h in 45 ml 18proz. Salzsäure unter Rückfluß erhitzt und aufgearbeitet. Man erhält 0,86 g (90%) hygroskopischen, nicht kristallinen Feststoff.

$C_{19}H_{24}N_6S \cdot 3HCl$ (477,9)

MS (FAB-Methode): m/z (rel. Int. [%]) = 369 ([M+H]$^+$, 100), 226 (10), 174 (48), 143 (55), 142 (21), 109 (64), 95 (17).

$^1$H-NMR-Daten ($d_6$-DMSO, TMS als interner Standard): δ = 1,85 (m) 2H, 2,73 (t) 4H, 3,0—3,7 (m) 4H, 4,43 (s) 2H, 7,4—8,6 (m), 10H, 4H austauschbar mit $D_2O$, 8,93 (d) 1H, 9,01 (d) 1H, ppm.

## Beispiel 74

N-{-[(Benzimidazol-2-yl)methylthio]ethyl}-N'-benzoyl-N''-[3-(imidazol-4-yl)propyl]-guanidin

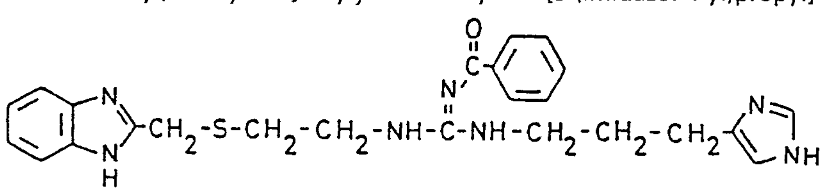

Die Herstellung erfolgt analog Beispiel 71 ausgehend von 1,04 g (5 mmol) 2-[(Benzimidazol-2-yl)methylthio]ethylamin.

Ausb.: 1.15 g (50%) nichtkristalliner Feststoff (Schaum).

$C_{24}H_{27}N_7OS$ (461,6)

MS: m/z (rel. Int. [%]) = 461 (M$^+$., 1), 131 (90), 109 (22), 105 (100).

$^1$H-NMR-Daten ($d_6$-DMSO, TMS als interner Standard): δ = 1,85 (m) 2H, 2,5—3,0 (m) 4H, 3,0—3,7 (m) 4H, 3,99 (s) 2H, 6,77 (s) 1H, 7,0—7,7 (m) 8H, 8,07 (m) 2H, ppm.

## Beispiel 75

N-{2-[(Benzimidazol-2-yl)methylthoi]ethyl}-N-[3-(imidazol-4-yl)propyl]guanidin

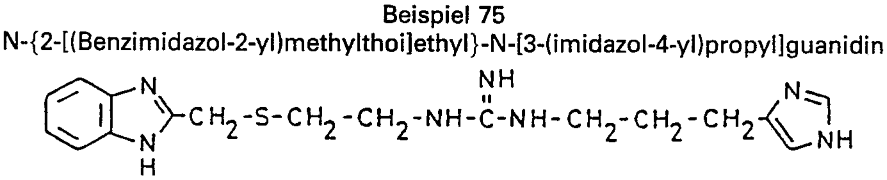

0,65 g (1,4 mmol) N-{2-[(Benzimidazol-2-yl)methylthio]ethyl}-N'-benzoyl-N''-[3-(imidazol-4-yl)propyl]-guanidin werden 6 h in 45 ml 18 proz. Salzsäure unter Rückfluß erhitzt und aufgearbeitet.

Ausb.: 0,57 g (87%) nichtkristalliner hygroskopischer Feststoff (Schaum).

$C_{17}H_{23}N_7S \cdot 3HCl$ (466,9)

MS (FAB-Methode): m/z (rel. Int. [%]) = 358 ([M+H]$^+$, 100), 228 (53), 226 (10), 131 (86), 109 (43).

$^1$H-NMR-Daten ($d_6$-DMSO, TMS als interner Standard) δ = 1,85 (m) 2H, 2,75 (t) 2H, 2,83 (t) 2H, 2,9—3,7 (m) 4H, 4,39 (s) 2H, 7,3—8,3 (m) 9H, 4H austauschbar mit $D_2O$, 9,02 (d) 1H, ppm.

## Beispiel 76

N-Benzoyl-N'-[3-(imidazol-4-yl)propyl]-N''-{2-[(phenyl(pyrid-2-yl)methyl)thio]ethyl}guanidin

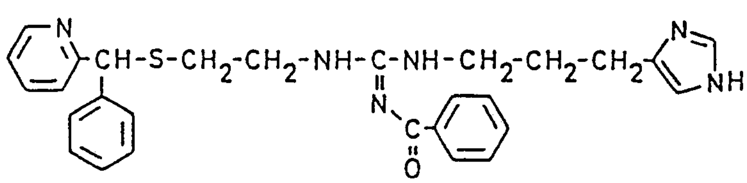

Die Herstellung erfolgt analog Beispiel 71, ausgehend von 1,25 g (5 mmol) 2-{[Phenyl(pyrid-2-yl)methyl]thio}ethylamin.

Ausb.: 1,2 g (48%); Schmp. 134°C (Ethylacetat).
$C_{28}H_{30}N_6OS$ (498,7) Ber.: C 67,44 H 6,06 N 16,85
Gef.: C 67,12 H 6,10 N 16,62
$^1$H-NMR-Daten ($CDCl_3$, TMS als interner Standard): δ = 1,89 (m) 2H, 2,64 (t) 2H, 2,73 (t) 2H, 3,3 (br.) 2H, 3.65 (br.) 2H, 5,34 (s) 1H, 6,72 (s) 1H, 7,05—7,65 (m) 10H, 8,17 (d) 2H, 8,51 (d) 1H, ppm.

Beispiel 77

N-[3-(Imidazol-4-yl)propyl]-N'-{2-[(phenyl(pyrid-2-yl)methyl)thio]ethyl}guanidin

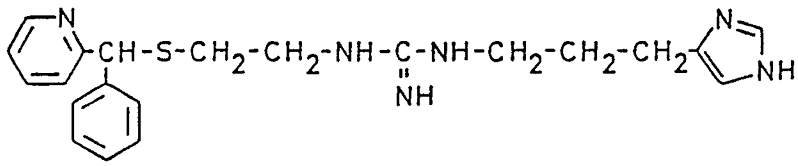

Die Herstellung erfolgt aus 0,85 g (1,7 mmol) N-Benzoyl-N'-[3-(imidazol-4-yl)propyl]-N''-{2-[(phenyl(pyrid-2-yl)methyl)thio]ethyl}guanidin.
Ausb.: 0,78 g (91%) hygroskopischer, nicht kristalliner Feststoff.
$C_{21}H_{26}N_6S \cdot 3HCl$ (503,9)
MS (FAB-Methode): m/z (rel. Int. [%]) = 395 ($[M+H]^+$, 38), 168 (100), 109 (21).
$^1$H-NMR-Daten ($d_6$-DMSO, TMS als interner Standard): δ = 1,83 (m) 2H, 2,57 (t) 2H, 2,70 (t) 2H, 3,18 (dt) 2H, 3,44 (dt) 2H, 5,87 (s) 1H, 7,2—7,8 (m) 10H, 2H austauschbar mit $D_2O$, 7,87 (m) 2H, 1H austauschbar mit $D_2O$, 8,10 (m) 2H, 1H austauschbar mit $D_2O$, 9,05 (s) 1H, 14,4 (br.) 1H, austauschbar mit $D_2O$, 14,7 (br.) 1H, austauschbar mit $D_2O$, ppm.

Beispiel 78

N-{2-[(5-Chlor-2-thenyl)thio]ethyl}-N'-[3-(imidazol-4-yl)propyl]guanidin

Herstellung der Vorstufen

a) 2-[(5-Chlor-2-thenyl)thio]ethylamin

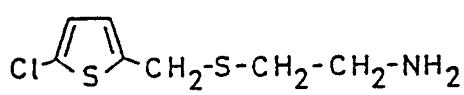

In eine Lösung von 1,38 g (60 mmol) Natrium in 100 ml Methanol werden unter Begasung mit Stickstoff 3,41 g (30 mmol) Cysteamin-hydrochlorid eingetragen und nach 10 min Rühren bei Raumtemperatur 5,01 g (30 mmol) 5-Chlor-2-(chlormethyl)thiophen zugesetzt. Nach 1 h wird das Lösungsmittel i. Vak. abdestilliert, der Rückstand in 5proz. Salzsäure gelöst und mit Ether extrahiert.

Nach Alkalisieren mit Natronlauge wird die wäßrige Phase mit Methylenchlorid ausgeschüttelt, die organische Phase mit Wasser gewaschen, über Natriumsulfat getrocknet und i. Vak. eingedampft. Die zurückbleibende ölige Aminbase wird mit ethanolischer Salzsäure in das Hydrochlorid übergeführt und aus Ethnaol/Wasser umkristallisiert.
Ausb.: 4,0 g (55%); Schmp. 166°C.
$C_7H_{10}ClNS_2 \cdot HCl$ (244,2) Ber.: C 34,43 H 4,54 N 5,74
Gef.: C 34,41 H 4,63 N 5,79
$^1$H-NMR-Daten ($d_6$-DMSO, TMS als interner Standard): δ = 2,6—3,15 (m) 4H, 3,98 (s) 2H, 6,86 (d) 1H, 6,95 (d) 1H, ppm.

b) N-Benzoyl-N'-{2-[(5-chlor-2-thenyl)thio]ethyl}thioharnstoff

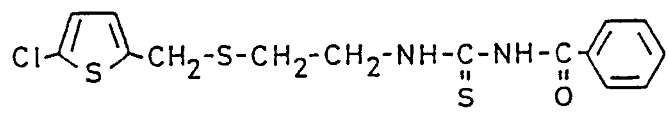

2,07 g (10 mmol) 2-[(5-Chlor-2-thenyl)thio]ethylamin-hydrochlorid werden mit der äquivalenten Menge Natriumethylat in Ethanol in die Base übergeführt, und nach Abfiltrieren des ausgefallenen Natriumchlorids und Eindampfen der ethanolischen Lösu i. Vak. analog Beispiel 70 (Vorstufe a) mit 1,63 g (10 mmol) Benzoylisothiocyanat umgesetzt.
Ausb.: 3,3 g (89%); Schmp. 104°C (Ethanol/Wasser).
$C_{15}H_{15}ClN_2OS_3$ (370,9) Ber.: C 48,57 H 4,08 N 7,55
Gef.: C 48,48 H 4,13 N 7,64
$^1$H-NMR-Daten ($CDCl_3$, TMS als interner Standard): δ = 2,84 (t) 2H, 3,91 (s) 2H, 3,91 (dt) 2H, 6,72 (d) 1H, 6,78 (d) 1H, 7,52 (m) 2H, 7,64 (m) 1H, 7,85 (m) 2H, 9,05 (br.) 1H, austauschbar mit $D_2O$, 11,0 (br.) 1H, austauschbar mit $D_2O$, ppm.

c) N'-{-[(5-chlor-2-thenyl)thio]ethyl}thioharnstoff

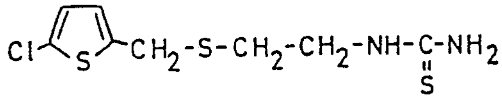

2,78 g (7,5 mmol) N-Benzoyl-N'-{2-[(5-chlor-2-thenyl)thio]ethyl}thioharnstoff werden mit 2,1 g Kaliumcarbonat in einer Mischung aus 30 ml Wasser und 100 ml Methanol 1 h unter Rückfluß erhitzt. Anschließend wird i. Vak. eingedampft und der Rückstand aus Ethanol/Wasser kristallisiert.

Ausb.: 1,8 g (90%); Schmp. 63°C.

$C_8H_{11}ClN_2S_3$ (266,8) Ber.: C 36,01 H 4,16 N 10,50
Gef.: C 36,25 H 4,23 N 10,59

$^1$H-NMR-Daten ($CDCl_3$, TMS als interner Standard): δ = 2,74 (t) 2H, 3,7 (br.) 2H, 3,87 (s) 2H, 5,99 (s) 2H, austauschbar mit $D_2O$, 6,65—6,8 (m) 3H, 1H austauschbar mit $D_2O$, ppm.

d) N-{2-[(5-Chlor-2-thenyl)thio]ethyl}-S-methyl-isothiouroniumiodid

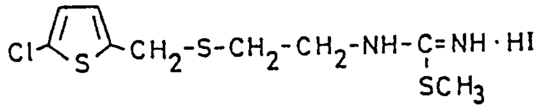

1,33 g (5 mmol) N-{2-[(5-Chlor-2-thenyl)thio]ethyl}thioharnstoff werden mit 0,4 ml Methyliodid in 100 ml Ethanol über Nacht bei Raumtemperatur gerührt. Nach dem Abdampfen des Lösungsmittels i. Vak. verbleiben 2,0 g (98%) dc reines Öl.

$C_9H_{13}ClN_2S_3 \cdot HI$ (408,8) Molmasse (MS): Ber.: 279,99295; Gef.: 279,99234

MS (FAB-Methode): m/z (rel. Int. [%] = 281 ($[M+H]^+$, 72), 131 (100), 103 (10).

$^1$H-NMR-Daten ($d_6$-DMSO, TMS als interner Standard): δ = 2,66 (s) 3H, 2,72 (t) 2H, 3,56 (t) 2H, 4,03 (s) 2H, 6,95 (m) 2H, 9,1 (br.) 3H, austauschbar mit $D_2O$, ppm.

N-{2-[(5-Chlor-2-thenyl)thio]ethyl}-N'-[3-(imidazol-4-yl)propyl]guanidin

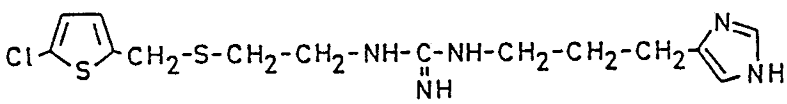

Die Herstellung und Isolierung erfolgt analog Beispiel 70, ausgehend von 1,43 g (3,5 mmol) N-{(2-(5-Chlor-2-thenyl)thio]ethyl}-S-methyl-isothiouroniumiodid.

Ausb.: 0,87 g (51%) zähes Öl.

$C_{14}H_{20}ClN_5S_2 \cdot HI$ (485,8)

MS (FAB-Methode): m/z (rel. Int. [%]) = 358 ($[M+H]^+$, 94), 131 (100) 109 (94).

$^1$H-NMR-Daten ($d_6$-DMSO, TMS als interner Standard): δ = 1,79 (m) 2H, 2,56 (t) 2H, 2,63 (t) 2H, 3,19 (dt) 2H, 3,37 (dt) 2H, 4,01 (s) 2H, 6,90 (d) 1H, 6,95 (s) 1H, 6,97 (d) 1H, 7,86 (s) 1H, ppm.

## Beispiel 79

N-[3-(Imidazol-4-yl)propyl]-N'-{2-[(5-piperidinomethyl-2-thenyl)thio]ethyl}guanidin

Herstellung der Vorstufe

N-Benzoyl-N'-{2-[(5-piperidinomethyl-2-thenyl)thio]ethyl}thioharnstoff

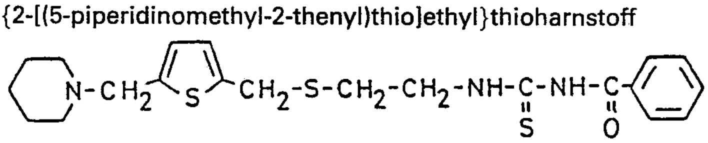

Die Herstellung erfolgt analog Beispiel 70 (Vorstufe a), ausgehend von 2,7 g (10 mmol) 2-[(5-Piperidinomethyl-2-thenyl)thio]ethylamin.

Ausb.: 3,95 g (91%); Schmp. 83°C (Methanol/Wasser).

$C_{21}H_{27}N_3OS^3$ (433,7) Ber.: C 58,16 H 6,28 N 9,69
Gef.: C 58,35 H 6,39 N 9,65

$^1$H-NMR-Daten ($CDCl_3$, TMS als interner Standard): δ = 1,42 (m) 2H, 1,57 (m) 4H, 2,41 (m) 4H, 2,84 (t) 2H, 3,62 (s) 2H, 3,90 (dt) 2H, 3,96 (s) 2H, 6,70 (d) 1H, 6,82 (d) 1H, 7,52 (m) 2H, 7,64 (m) 1H, 7,86 (d) 2H, 9,05 (br.) 1H, austauschbar mit $D_2O$, 10,97 (br.) 1H, austauschbar mit $D_2O$, ppm.

N-[3-(Imidazol-4-yl)propyl]-N'-{2-[(5-piperidinomethyl-2-thenyl)thio]ethyl}guanidin

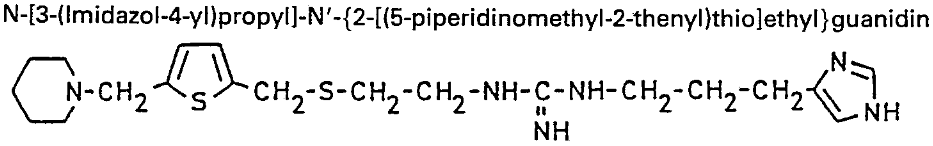

2,17 g (5 mmol) N-Benzoyl-N'-{2-[(5-piperidinomethyl-2-thenyl)thio]ethyl}thioharnstoff werden mit

1,4 g Kaliumcarbonat in einer Mischung aus 30 ml Wasser und 100 ml Methanol 1 h unter Rückfluß erhitzt. Anschließend wird das Lösungsmittel i. Vak. abdestilliert, der Rückstand in Ether aufgenommen, die organische Phase mit Wasser gewaschen, über Natriumsulfat getrocknet, i. Vak. eingedampft und unter Zusatz von 0,4 ml Methyliodid in 100 ml Ethanol über Nacht bei Raumtemp. gerührt. Das Lösungsmittel wird i. Vak. abdestilliert und der ölige Rückstand mit 0,69 g (5,5 mmol) 3-Imidazol-4-yl)propylamin in 30 ml Pyridin 3 h unter Rückfluß erhitzt. Anschließend wird der Reaktionsansatz i. Vak. eingedampft und das Produkt durch präparative Schichtchromatographie isoliert und gereinigt (Kieselgel 60 $PF_{254}$ gipshaltig; Fließmittel: Chloroform/Methanol 85 + 15, Ammoniakatmosphäre). Nach dem Eindampfen der Eluate verbleiben 0,78 g (28%) des Hydroiodids in Form eines zähen Öls.

$C_{20}H_{32}N_6S_2 \cdot HI$ (548,5)

MS (FAB-Methode): m/z (rel. Int. [%]) = 421 ($[M+H]^+$, 37), 336 (4), 194 (41), 141 (24), 109 (61), 100 (17), 98 (100), 84 (24).

$^1$H-NMR-Daten ($d_6$-DMSO, TMS als interner Standard): δ = 1,2—2,1 (m) 8H, 2,1—2,9 (m) 8H, 2,9—3,7 (m) 4H, 3,49 (s) 2H, 3,91 (s) 2H, 6,6—6,9 (m) 3H, 7,48 (d) 1H, ppm.

Beispiel 80

N-Benzoyl-N'-[3-(imidazol-4-yl)propyl]-N''-[3-phenyl-3-(pyrid-2-yl)propyl]guanidin

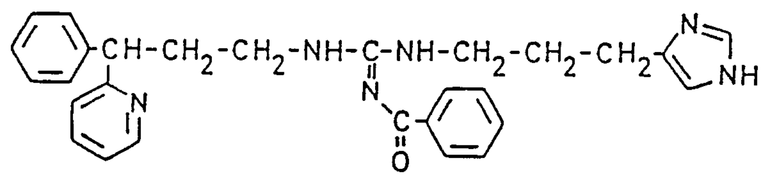

Die Herstellung erfolg analog Beispiel 71, ausgehend von 1,06 g (5 mmol) 3-Phenyl 3-(pyrid-2-yl)propylamin.

Ausb.: 1,3 g (56%) nicht kristalliner Feststoff (Schaum).

$C_{28}H_{30}N_6O$ (466,6)

MS (FAB-Methode): m/z (rel. Int. [%]) = 467 ($[M+H]^+$, 20), 196 (87), 109 (30), 105 (100), 77 (28).

$^1$H-NMR-Daten ($CDCl_3$, TMS als interner Standard): δ = 1,96 (m) 2H, 2,32 (br.) 1H, 2,70 (m) 3H, 3,1—4,05 (m) 4H, 4,20 (m) 1H, 6,73 (s) 1H, 7,0—7,7 (m) 12H, 8,13 (m) 2H, 8,57 (m) 1H, ppm.

Beispiel 81

N-[3-(Imidazol-4-yl)propyl]-N'-[3-phenyl-3-(pyrid-2-yl)propyl]guanidin

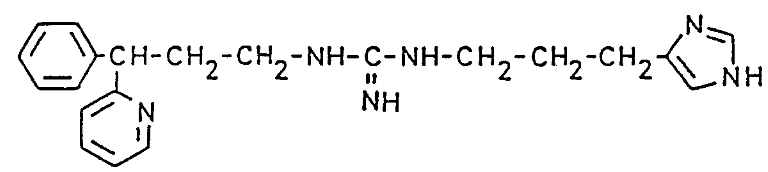

Die Herstellung erfolgt aus 0,93 g (2 mmol) N-Benzoyl-N'-[3-(imidazol-4-yl)propyl]-N''-[3-phenyl-3-(pyrid-2-yl)propyl]guanidin.

Ausb.: 0,86 g (91%) hygroskopischer, nicht kristalliner Feststoff.

$C_{21}H_{26}N_6 \cdot 3HCl$ (471,9) Molmasse (MS): Ber.: 362,22189; Gef.: 362,2223

MS (FAB-Methode): m/z (rel. Int. [%]) = 363 ($[M+H]^+$, 89), 196 (100), 168 (32), 109, (40), 100 (26).

$^1$H-NMR-Daten ($d_6$-DMSO, TMS als interner Standard): δ = 1,84 (m) 2H, 2,3—2,7 (m) 2H, 2,72 (t) 2H, 3,0—3,3 (m) 4H, 4,67 (t) 1H, 7,2—7,65 (m) 8H, 2H austauschbar mit $D_2O$, 7,74 (dd) 1H, 7,98 (m) 3H, 2H austauschbar mit $D_2O$, 8,32 (m) 1H, 8,74 (d) 1H, 9,05 (s) 1H, 14,4 (br.) 1H, austauschbar mit $D_2O$, 14.7 (br.) 1H, austauschbar mit $D_2O$, ppm.

Beispiel 82

N-Benzoyl-N'-[3-(4-chlorphenyl)-3-(pyrid-2-yl)propyl]-N''-[3-(imidazol-4-yl)propyl]guanidin

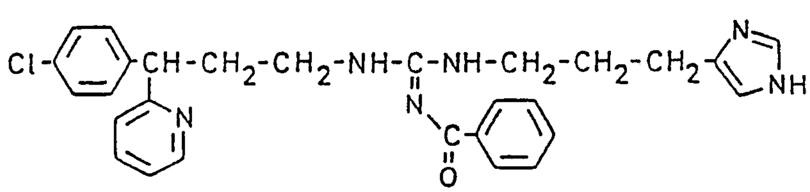

Die Herstellung erfolgt analog Beispiel 71, ausgehend von 1,23 g (5 mmol) 3-(4-Chlorphenyl)-3-(pyrid-2-yl)propylamin.

Ausb.: 1,4 g (56%) nicht kristalliner Feststoff (Schaum).

$C_{28}H_{29}ClN_6O$ (501,0)

MS (FAB-Methode): m/z (rel. Int. [%]) = 501 ($[M+H]^+$, 20), 230 (58), 167 (19), 109 (37), 105 (100), 77 (28).

$^1$H-NMR-Daten ($CDCl_3$, TMS als interner Standard): δ = 1,96 (m) 2H, 2,3 (br.) 1H, 2,55—2,8 (m) 1H, 2,70 (t) 2H, 3,1—4,0 (m) 4H, 4,18 (dd) 1H, 6,73 (s) 1H, 7,0—7,7 (m) 11H, 8,12 (m) 2H, 8,57 (m) 1H, ppm.

Beispiel 83

N-[3-(4-Chlorphenyl)-3-(pyrid-2-yl)propyl]-N'-[3-(imidazol-4-yl)propyl]guanidin

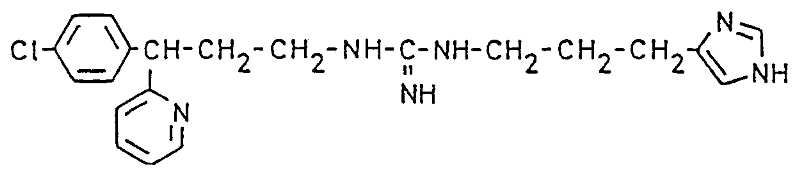

Die Herstellung erfolgt aus 1,0 g (2 mmol) N-Benzoyl-[3-(4-chlorphenyl)-3-(pyrid-2-yl)propyl]-N''-[3-(imidazol-4-yl)propyl]guanidin.

Ausb.: 0,95 g (94%) hygroskopischer, nicht kristalliner Feststoff.

$C_{21}H_{25}ClN_6 \cdot 3HCl$ (506,3) Molmasse (MS): Ber.: 396,18292 Gef.: 396,18237.

MS: m/z (rel. Int. [%]) = 396 ($M^+$., 2), 315 (14), 230 (31), 216 (57), 203 (100), 194 (41), 167 (41), 109 (16), 95 (22), 81 (14).

$^1$H-NMR-Daten ($d_6$-DMSO, TMS als interner Standard) δ = 1,84 (m) 2H, 2,41 (m) 1H, 2,55 (m) 1H, 2,72 (t) 2H, 3,11 (dt) 2H, 3,18 (dt) 2H, 4,70 (t) 1H, 7,35—7,65 (m) 7H, 2H austauschbar mit $D_2O$, 7,74 (dd) 1H, 7,96 (m) 3H, 2H austauschbar mit $D_2O$, 8,33 (dd) 1H, 8,74 (d) 1H, 9,05 (s) 1H, 14,4 (br.) 1H, austauschbar mit $D_2O$, 14,7 (br.) 1H, austauschbar mit $D_2O$, ppm.

Beispiel 84

N-Benzoyl-N'-[3-(4-bromophenyl)-3-(pyrid-2-yl)propyl]-N''-[3-(imidazol-4-yl)propyl]-guanidin

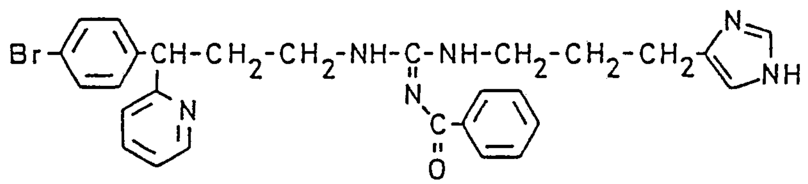

Die Herstellung erfolgt analog Beispiel 71, ausgehend von 1,46 g (5 mmol) 3-(4-Bromphenyl)-3-(pyrid-2-yl)propylamin.

Ausb.: 1,3 g (48%) nicht kristalliner Feststoff (Schaum).

$C_{28}H_{29}BrN_6O$ (545,5)

MS (FAB-Methode): m/z (rel. Int. [%]) = 545 ($[M+H]^+$, 8), 274 (36) 167 (16), 109 (40), 105 (100), 81 (11), 77 (23).

$^1$H-NMR-Daten ($CDCl_3$, TMS als interner Standard): δ = 1,96 (m) 2H, 2,3 (br.) 1H, 2,5—2,8 (m) 3H, 3,1—4,05 (m) 4H, 4,17 (dd) 1H, 6,74 (s) 1H, 6,9—7,7 (m) 11H, 8,12 (m) 2H, 8,57 (m) 1H, ppm.

Beispiel 85

N-(3-(4-Bromphenyl)-3-(pyrid-2-yl)propyl]-N'-[3-(imidazol-4-yl)propyl]guanidin

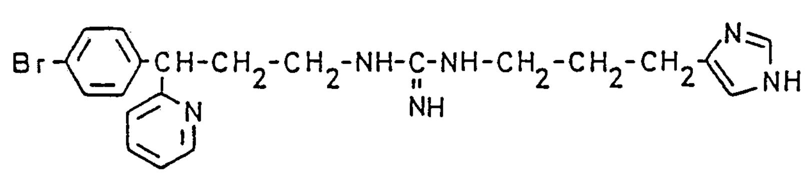

Die Herstellung erfolgt aus 1,09 g (2 mmol) N-Benzoyl-N'-[3-(4-bromphenyl)-3-(pyrid-2-yl)propyl]-N''-[3-(imidazol-4-yl)propyl]guanidin.

Ausb.: 0,98 g (89%) hygroskopischer, nicht kristalliner Feststoff.

$C_{21}H_{25}BrN_6 \cdot 3HCl$ (550,8) Molmasse (MS): Ber.: 440, 13242; Gef.: 440,13283

MS: m/z (rel., Int. [%]) = 440 ($M^{+}$., 1), 359 (7), 260 (36), 247 (100), 194 (70), 180 (29), 167 (61), 109 (18), 95 (43), 81 (32).

$^1$H-NMR-Daten ($d_6$-DMSO, TMS als interner Standard): δ = 1,85 (m) 2H, 2,40 (m) 1H, 2,55 (m) 1H, 2,73 (t) 2H, 3,12 (dt) 2H, 3,20 (dt) 2H, 4,66 (t) 1H, 7.45—7,8 (m) 8H, 2H austauschbar mit $D_2O$, 7,85—8,1 (m) 3H, 2H austauschbar mit $D_2O$, 8,28 (m) 1H, 8,72 (d) 1H, 9,05 (s) 1H, 14,4 (br.) 1H, austauschbar mit $D_2O$, 14,8 (br.) 1H austauschbar mit $D_2O$, ppm.

Beispiel 86

N-Benzoyl-N'-[3-(4-fluorphenyl)-3-(pyrid-2-yl)propyl]-N''-[3-(imidazol-4-yl)propyl] guanidin

Herstellung der Vorstufen

a) N-[3-Cyan-3-(4-fluorphenyl)-3-(pyrid-2-yl)propyl]phthalimid

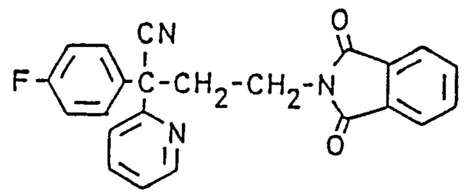

42,4 g (0,2 mol) (4-Fluorphenyl)-(pyrid-2-yl)acetonitril werden in 50 ml Dimethylformamid gelöst und in eine mit Eis gekühlte Suspension von 5,0 g Natriumhydrid (eingesetzt als 60proz. Dispersion in Mineralöl) in 150 ml Dimethylformamid getropft. Anschließend wird 15 min bei Raumtemperatur gerührt und nach Zusatz von 53,4 g (0,21 mol) N-(2-Brom-ethyl)phthalimid 5 h unter Rückfluß erhitzt. Nach dem Erkalten wird mit 500 ml Ether verdünnt, die organische Phase mit Wasser neutral gewaschen und nach Trocknen über Natriumsulfat i. Vak. eingedampft. Der ölige Rückstand kristallisiert auf Zugabe von Methanol.

Ausb.: 48,5 g (63%); Schmp. 154°C (Methanol).

$C_{23}H_{16}FN_3O_2$ (385,4) Ber.: C 71,68 H 4,18 N 10,90

Gef.: C 71,59 H 3,87 N 11,07

IR (KBr): 2240, 1770, 1715, 1605 1585, 1570 $cm^{-1}$.

$^1$H-NMR-Daten: ($CDCl_3$, TMS als interner Standard): δ = 2,9 (m) 2H, 3,88 (m) 2H, 6,8—8,0 (m) 11H, 8,55 (m) 1H, ppm.

b) 3-(4-Fluorphenyl)-3-(pyrid-2-yl)propylamin

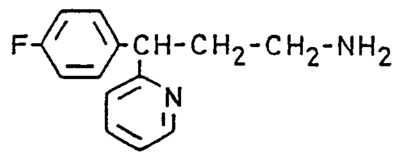

46,25 g (0,12 mol) N-[3-Cyan-3-(4-fluorphenyl)-3-(pyrid-2-yl)propyl]phthalimid werden in 100 ml 75proz. Schwefelsäure 5 h auf 150°C erhitzt. Nach dem Erkalten wird der Reaktionsansatz auf Eis gegossen, durch eine Glasfritte filtriert, mit Natronlauge alkalisiert und mit Ether extrahiert. Die vereinigten Extrakte werden mit Wasser gewaschen, über Natriumsulfat getrocknet, i. Vak. eingedampft und das Produkt durch Destillation bei 150—155°C/0,8 mm Hg isoliert.

Ausb.: 19,1 g (69%) dc reines Öl.

$C_{14}H_{15}FN_2$ (230,3) Molmasse (MS): Ber.: 230, 12193; Gef.: 230,12174.

MS: m/z (rel. Int. [%]) = 230 ($M^{+\cdot}$, 2), 229 (2), 212 (4), 200 (32), 187 (100).

$^1$H-NMR-Daten: ($CDCl_3$, TMS als interner Standard): δ = 1,7 (br.) 2H, austauschbar mit $D_2O$, 2,17 (m) 1H, 2,38 (m) 1H, 2,64 (t) 2H, 4,17 (t) 1H, 6,97 (dd) 2H, 7,09 (dd) 1H, 7,14 (d) 1H, 7,31 (dd) 2H, 7,56 (dd) 1H, 8,56 (d) 1H, ppm.

N-Benzoyl-N'-[3-(4-fluorphenyl)-3-(pyrid-2-yl)-propyl]-N''-[3-(imidazol-4-yl)propyl] guanidin

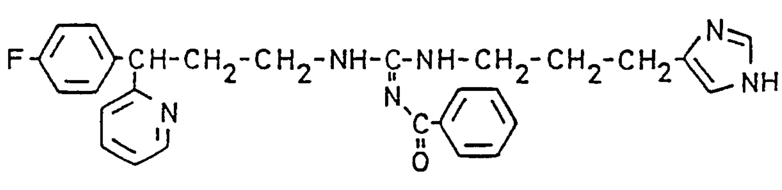

Die Herstellung erfolgt analog Beispiel 71, ausgehend von 1,15 g (5 mol) 3-(4-Fluorphenyl)-3-(pyrid-2-yl)propylamin.

Ausb.: 1,4 g (58%) nicht kristalliner Feststoff (Schaum).

$C_{28}H_{29}FN_6O$ (484,6)

MS (FAB-Methode): m/z (rel. Int. [%]) = 485 ($[M+H]^+$, 29), 214 (93), 186 (24), 109 (31), 105 (100), 77 (29).

$^1$H-NMR-Daten: ($CDCl_3$, TMS als interner Standard): δ = 1,97 (m) 2H, 2,3 (br.) 1H, 2,5—2,8 (m) 3H, 3,0—4,1 (m) 4H, 4,18 (dd) 1H, 6,72 (s) 1H, 6,8—7,8 (m) 11H, 8,12 (m) 2H, 8,56 (m) 1H, ppm.

Beispiel 87

N-[3-(4-Fluorphenyl)-3-(pyrid-2-yl)propyl]-N'-[3-(imidazol-4-yl)propyl]guanidin

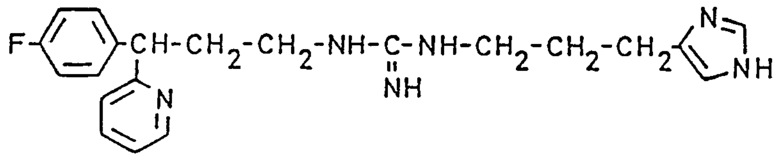

Die Herstellung erfolgt aus 0,97 g (2 mmol) N-Benzoyl-N'-[3-(4-fluorphenyl)-3-(pyrid-2-yl)propyl]-N''-[3-(imidazol-4-yl)propyl]guanidin

Ausb.: 0,9 g (92%) hygroskopischer, nicht kristalliner Feststoff.

$C_{21}H_{25}FN_6 \cdot 3HCl$ (489,9)

MS (FAB-Methode): m/z (rel. Int. [%]) = 381 ([M+H]$^+$, 100), 256 (40), 214 (86), 186 (20), 109 (44), 100 (28).

$^1$H-NMR-Daten: $d_6$-DMSO, TMS als interner Standard): δ = 1,83 (m) 2H, 2,39 (m) 1H, 2,55 (m) 1H, 2,71 (t) 2H, 3,09 (dt) 2H, 3,17 (dt) 2H, 4,68 (t) 1H, 7,19 (dd) 2H, 7,49 (s) 1H, 7,56 (m) 4H, 2H austauschbar mit $D_2O$, 7,71 (m) 1H, 7,95 (m) 3H, 2H austauschbar mit $D_2O$, 8,29 (m) 1H, 8,72 (m) 1H, 9,04 (s) 1H, 14,4 (br.) 1H, austauschbar mit $D_2O$, 14,8 (br.) 1H, austauschbar mit $D_2O$, ppm.

Beispiel 88

N-[3-(Imidazol-4-yl)propyl]-N'-[3-(pyrid-2-yl)-3-(2-thienyl)propyl]guanidin

Herstellung der Vorstufen

a) N-[3-Cyan-3-(pyrid-2-yl)-3-(2-thienyl)propyl]phthalimid

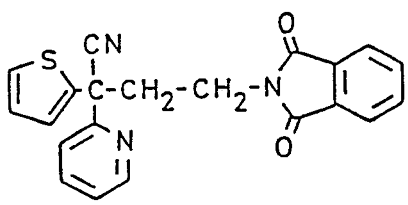

Die Herstellung erfolgt analog Beispiel 86 (Vorstufe a) aus 20,0 g (0,1 mol) (Pyrid-2-yl)-(2-thienyl)acetonitril.

Ausb.: 12,3 g (33%). Schmp. 104°C (Ethanol)

$C_{21}H_{15}N_3O_2S$ (373,4) Ber.: C 67,54 H 4,05 N 11,25

Gef.: C 67,27 H 3,87 N 11,18

IR (KBr): 2245, 1770, 1720, 1615, 1586 1572 cm$^{-1}$,

$^1$H-NMR-Daten: ($CDCl_3$, TMS als interner Standard): δ = 2,95 (m) 2H, 3,90 (t) 2H, 6,8—7,4 (m) 4H, 7,5—7,9 (m) 6H, 8,57 (m) 1H, ppm.

b) 3-(Pyrid-2-yl)-3-(2-thienyl)propylamin

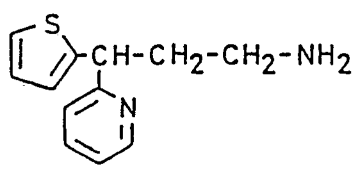

11,2 g (30 mol) N-[3-Cyan-3-(pyrid-2-yl)-3-(2-thienyl)propyl]phthalimid werden mit 30 g Kaliumhydroxid in 100 ml Butanol 8 h unter Rückfluß erhitzt. Anschließend wird mit 300 ml Ether verdünnt, mit Wasser gewaschen, über Natriumsulfat getrocknet, i. Vak eingeengt und bei 145—148°C/0,8 destilliert.

Ausb.: 3,8 g (58%) dc reines Öl.

$C_{12}H_{14}N_2S$ (218,3) Molmasse (MS): Ber.: 218,08777; Gef.: 218,08779

MS: m/z (rel. Int. [%] = 218 (M$^{+\cdot}$, 20), 201 (14), 188 (100), 175 (69).

$^1$H-NMR-Daten: ($CDCl_3$, TMS als interner Standard): δ = 1,6 (br.) 2H, austauschbar mit $D_2O$, 2,2—2,45 (m) 2H, 2,67 (t) 2H, 4,48 (t) 1H, 6,93 (m) 2H, 7,1—7,25 (m) 3H, 7,60 (m) 1H, 8,57 (m) 1H, ppm.

c) N-Benzoyl-N'-[3-(pyrid-2-yl)-3-(2-thienyl)propyl]thioharnstoff

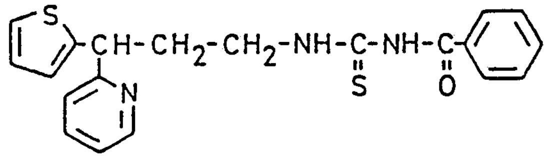

2,18 g (10 mol) 3-(Pyrid-2-yl)-3-(2-thienyl)propylamin werden mit 1,63 g (10 mmol) Benzoylisothiocyanat 1 h

in 100 ml Chloroform unter Rückfluß erhitzt. Das Lösungsmittel wird i. Vak. abdestilliert und der Rückstand aus Ethanol kristallisiert.

Ausb.: 3,43 g (90%); Schmp. 95°C.

$C_{20}H_{19}B_3OS_2$ (381,5) Ber.: C 62,96 H 5,02 N 11,01

Gef.: C 62,63 H 4,85 N 11,06

$^1$H-NMR-Daten: ($CDCl_3$, TMS als interner Standard): δ = 2,57 (m) 1H, 2,71 (m) 1H, 3,73 (dt) 2H, 4,48 (t) 1H, 6,9—7.05 (m) 2H, 7,1—7,3 (m) 3H, 7,45—7,7 (m) 4H, 7,84 (m) 2H, 8,61 (m) 1H, 9,00 (s) 1H, austauschbar mit $D_2O$, 10,8 (br.) 1H, austauschbar mit $D_2O$, ppm.

d) N-[3-(Pyrid-2-yl)-3-(2-thienyl)propyl]thioharnstoff

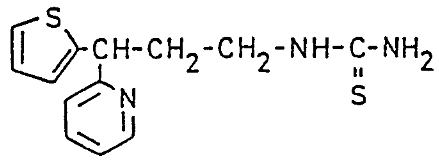

Die Herstellung erfolgt analog Beispiel 78 (Vorstufe c) aus 2,86 g (7,5 mmol) N-Benzoyl-N'-[3-(pyrid-2-yl)-3-(2-thienyl]propyl]thioharnstoff.

Ausb.: 1,93 g (93%); Schmp. 138°C (Ethanol).

$C_{13}H_{15}N_3S_2$ (277,4) Ber.: C 56,29 H 5,45 N 15,15

Gef.: C 56,28 H 5,44 N 15,21

$^1$H-NMR-Daten: ($CDCl_3$, TMS als interner Standard): δ = 2,4 (br) 1H, 2,55 (br.) 1H, 3,1—32,5 (m) 2H, 4,48 (m) 1H, 6,8—7,5 (m) 8H, 3H austauschbar mit $D_2O$, 7,65 (m) 1H, 8,51 (m) 1H, ppm.

e) S-Methyl-N-[3-(pyrid-2-yl)-3-(2-thienyl)propyl]isothiouroniumiodid

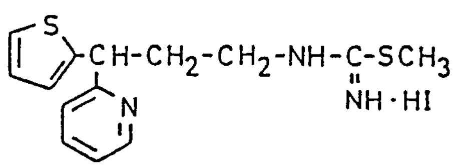

1,39 g (5 mmol)N-[3-(Pyrid-2-yl)-3-(2-thienyl)propyl]thioharnstoff werden mit 0,4 ml Methyliodid in 100 ml Ethanol über Nacht bei Raumtemperatur gerührt. Das Lösungsmittel wird. i. Vak. abdestilliert und der Rückstand aus Ethanol/Ether kristallisiert.

Ausb.: 1,78 g (85%); Schmp. 158°C.

$C_{14}H_{17}N_3S_2\cdot HI$ (419,3) Ber.: C 40.10 H 4,33 N 10,02

Gef.: C 40,17 H 4,31 N 10,05

$^1$H-NMR-Daten: ($d_6$-DMSO, TMS als interner Standard): δ = 2,25— 2,7 (m) 2H, 2,60 (s) 3H, 3,24 (m) 2H, 4,51 (t) 1H, 6,95—7,05 (m) 2H, 7,2—7,5 (m) 3H, 7,78 (m) 1H, 8,57 (m) 1H, 9,1 (br.) 3H, austauschbar mit $D_2O$, ppm.

N-[3-(Imidazol-4-yl)propyl]-N'-[3-(pyrid-2-yl)-3-(2-thienyl)propyl]guanidin

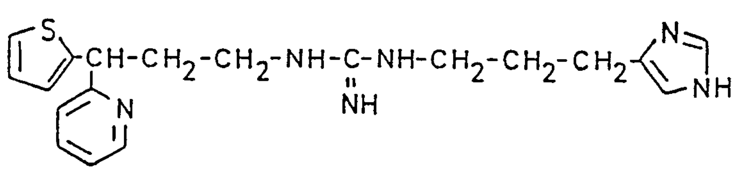

Die Herstellung erfolgt analog Beispiel 70 aus 1,47 g (3,5 mmol) S-Methyl-N-[3-(pyrid-2-yl)-3-(2-thienyl)propyl]isothiouroniumiodid.

Ausb.: 0,89 g (51%) hygroskopischer nicht kristalliner Feststoff.

$C_{19}H_{24}N_6S\cdot HI$ (496,4) Molmasse (MS): Ber.: 368,17832; Gef.: 368,1787

MS (FAB-Methode): m/z (rel. Int. [%]) = 369 ($[M+H]^-$, 100), 202 (97), 188 (10), 174 (18), 109 (49), 100 (33), 78 (69).

$^1$H-NMR-Daten: ($d_6$-DMSO, TMS als interner Standard): δ = 1,78 (m) 2H, 2,23 (m) 1H, 2,44 (m) 1H, 2,60 (t) 2H, 3,06 (br) 2H, 3,16 (dt) 2H, 4,49 (t) 1H, 6,9—7,05 (m) 2H, 7,16 (s) 1H, 7,28 (dd) 1H, 7,3—7,55 (m) 6H, 4H austauschbar mit $D_2O$, 7,77 (m) 1H, 8,33 (s) 1H, 8,56 (m) 1H, ppm.

Beispiel 89

N-Benzoyl-N'-[3-(imidazol-4-yl)propyl]-N''-[4-phenyl-4-(pyrid-2-yl)butyl]guanidin

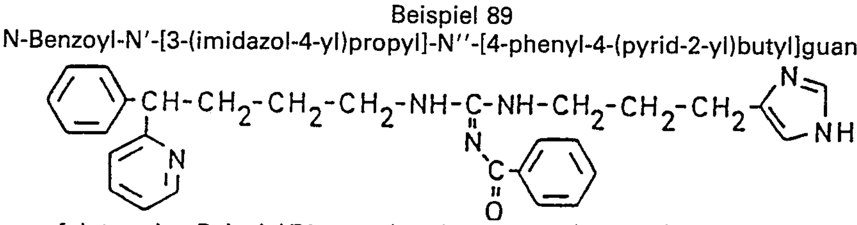

Die Herstellung erfolgt analog Beispiel 71 ausgehend von 1,13 g (5 mmol) 4-Phenyl-4-(pyrid-2-yl)butylamin.

Ausb.: 1,2 g (50%) nicht kristalliner Feststoff (Schaum).

$C_{29}H_{32}N_6O$ (480,6)

MS (FAB-Methode): m/z (rel. Int. [%]) = 481 ([M+H]$^+$, 18), 210 (48), 168 (16), 210 (47), 109 (38), 105 (100) 77 (31).

$^1$H-NMR-Daten: ($CDCl_3$, TMS als interner Standard): δ = 1,64 (m) 2H, 1,90 (m) 2H, 2,17 (m) 1H, 2,36 (m) 1H, 2,66 (t) 2H, 3,1—3,8 (m) 4H, 4,11 (t) 1H, 6,74 (s) 1H, 7,0—7,65 (m) 12H, 8,17 (m) 2H, 8,54 (m) 1H, ppm.

Beispiel 90

N-[3-(Imidazol-4-yl)propyl]-N'-(4-phenyl-4-(pyrid-2-yl)butyl]guanidin

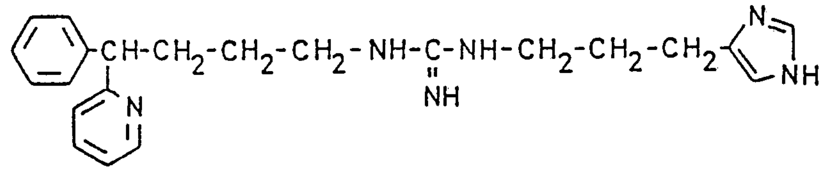

Die Herstellung erfolgt aus 0,96 g (2 mmol) N-Benzoyl-N'-[3-(imidazol-4-yl)propyl]-N''-[4-phenyl-4-(pyrid--2-yl)butyl]guanidin

$C_{22}H_{28}N_6 \cdot 3HCl$ (485,9) Molmasse (MS): Ber.: 376,23754; Gef.: 376,23645
$[M—NH_3]^{\cdot+}$: Ber.: 359,21099; Gef.: 359,21030

MS: m/z (rel. Int. [%]) = 376 ($M^{\cdot+}$, 4), 359 (3), 295 (8), 210 (31), 196 (16) 182 (100), 168 (75), 109 (12), 95 (35) 81 (22).

$^1$H-NMR-Daten: ($d_6$-DMSO, TMS als interner Standard): δ = 1,41 (m) 2H, 1,83 (m) 2H, 2,15—2,55 (m) 2H, 2,71 (t) 2H, 3,1—3,35 (m) 4H, 4,68 (t) 1H, 7,15—7,7 (m) 8H, 2H austauschbar mit $D_2O$, 7,81 (m) 1H, , 7,93 (m) 1H, austauschbar mit $D_2O$, 8,01 (m) 1H, austauschbar mit $D_2O$, 8,10 (m) 1H, 8,43 (m) 1H, 8,76 (m) 1H, 9,05 (s) 1H, 14,4 (br.) 1H, austauschbar mit $D_2O$, 14,8 (br.) 1H, austauschbar mit $D_2O$ ppm.

Beispiel 91

N-[4-(4-Fluorphenyl)-4-(pyrid-2-yl)butyl]-N'-[3-(imidazol-4-yl)propyl]guanidin

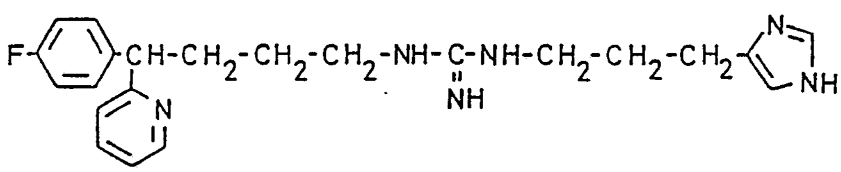

Die Herstellung erfolgt aus 1,0 g (2 mmol) N-Benzoyl-N'-[4-(4-fluorphenyl)-4-(pyrid-2-yl)butyl]-N''-[3-(imidazol-4-yl)]propyl]guanidin.

Ausb.: 0,89 g (88%) hygroskopischer nicht kristalliner Feststoff.

$C_{22}H_{27}FN_6 \cdot 3HCl$ (503,9)

$^1$H-NMR-Daten: ($d_6$-DMSO, TMS als interner Standard): δ = 1,43 (m) 2H, 1,83 (m) 2H, 2,15—2,6 (m) 2H, 2,72 (t) 2H, 3,1—3,4 (m) 4H, 4,68 (t) 1H, 7,19 (dd) 2H, 7,4—7,65 (m) 5H, 2H austauschbar mit $D_2O$, 7,7—8,15 (m) 4H, 2H austauschbar mit $D_2O$, 8,36 (m) 1H, 8,75 (m) 1H, 9,05 (s) 1H, 14,4 (br.) 1H, austauschbar mit $D_2O$, 14,8 (br.) 1H, austauschbar mit $D_2O$, ppm.

Beispiel 92

$N^1$-Benzoyl-$N^2$-[3-(4-imidazolyl)propyl]-$N^3$-[2-(N-benzyl-N-(pyrid-2-yl)-amino)ethyl]guanidin

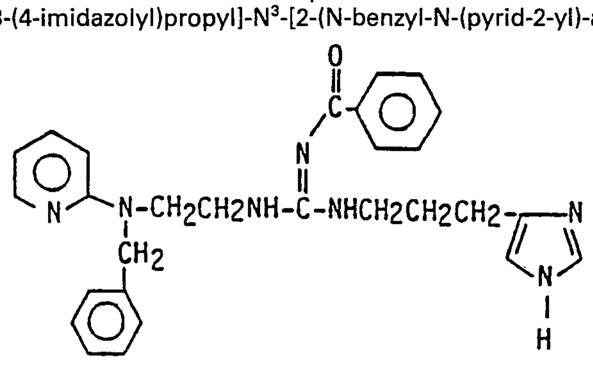

3,48 g (10 mmol) N[1]-Benzoyl-N[2]-[3-(4-imidazolyl)propyl]-O-phenylisoharnstoff und 2,27 g (10 mol) N-Benzyl-N-pyrid-2-yl-ethylendiamin werden in 50 ml Ethanol 20 h gekocht. Nach Eindsampfen im Vakuum wird das Rohprodukt mit Essigester/Ethanol (80:20) an Kieselgel chromatographiert. Die Hauptfraktion ergibt nach Eindampfen 3,51 g (73%) eines farblosen Feststoffs.

$C_{28}H_{31}N_{70}$ (481,60)

$^1$H-NMR-Daten: ($CD_3OD$, TMS als interner Standard): δ = 1,90 (m) 2H, 2,67 (t) 2H, 3,23 (t) 2H, 3,71—3,89 (m) 4H, 4,57 (s) 2H, 4,9 (breit) 3H, austauschbar mit $D_2O$, 6,43—7,57 (m) 13H, 7,96—8,21 (m) 3H, ppm.

## Beispiel 93

N[1]-Benzoyl-N[2]-[3-(1H-imidazol-4-yl)propyl]-N[3]-[2-[N-(4-chlorbenzyl)-N-(pyridin-2-yl)-amino]ethyl]-guanidin

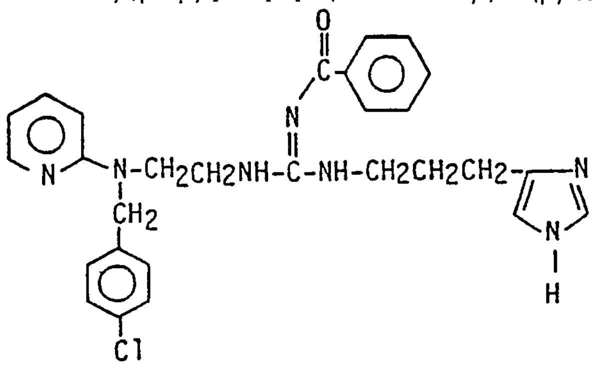

1,74 g (5 mmol) N[1]-Benzoyl-N[2]-[3-(1H-imidazol-4-yl)propyl]-O-phenyl-isoharnstoff und 1,31 g (5 mmol) N-(4-Chlorbenzyl)-N-(pyridin-2-yl)ethylendiamin werden in 30 ml Ethanol 24 Std. unter Rückfluß gekocht. Nach Eindampfen wird das erhaltene Rohprodukt mit Essigester/Ethanol (80:20) an Kieselgel gereinigt. Die Hauptfraktion wird eingedampft und der Rückstand aus Essigester/tert. Butyl-methylether (1:1) umkristallisiert. Man erhält 2,20 g (85%) der Titelverbindung in Form farbloser Kristalle vom Schmp. 166—167°C.

$C_{28}H_{30}ClN_{70}$ (516,04)

$^1$H-NMR-Daten: ($d_6$-DMSO, TMS als interner Standard): δ = 1,70—2,03 (m) 2H, 2,59 (t) 2H, 3,10—3,48 (m) 2H, 3,48—3,83 (m) 4H, 4,79 (s) 2H, 6,51—6,87 (m) 3H, 7,11—7,63 (m) 9H, 7,98—8,28 (m) 3H, 10,0 (breit) 1H, austauschbar mit $D_2O$, 10,3 (breit) 1H, austauschbar mit $D_2O$ ppm.

## Beispiel 94

N[1]-[3-(4-Imidazolyl)propyl]-N[2]-[2-(N-benzyl-N-(pyrid-2-yl)amino)ethyl]guanidin-trihydrochlorid

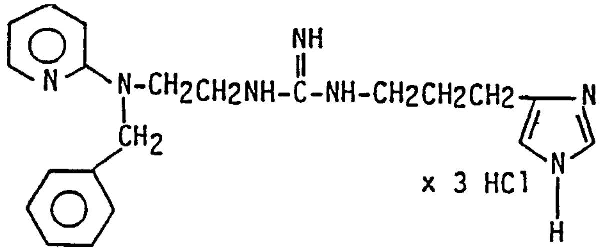

Methode A

2,41 g (5,0 mmol) N[1]-Benzoyl-N[2]-[3-(4-imidazolyl)propyl]-N[3]-[2-N-benzyl-N-pyrid-2-yl)-amino)ethyl]-guanidin (Beispiel 138) werden in 50 ml konz. Salzsäure 18 h gekocht. Nach Einengen auf die Hälfte des ursprünglichen Volumens wird dreimal mit 50 ml Ether extrahiert. Die wäßrige Phase wird filtriert und eingedampft und der Rückstand zweimal in 20 ml Ethanol aufgenommen und zur Trockne eingedampft. Umkristallisieren des Rückstandes aus Isopropanol/Ethanol ergibt 1,17 g (48%) der Titelverbindung.

$C_{21}H_{30}Cl_3N_7$ (486,88)

$^1$H-NMR-Daten: ($CD_{30}D$, TMS als interner Standard): δ = 1,80—2,19 (m) 2H, 2,87 (t) 2H, 3,34 (t) 2H, 3,65 (m) 2H, 3,78 (m) 2H, 4,16 (t) 2H, 4,9 (breit) 7H, austauschbar mit $D_2O$, 7,21—8,10 (m) 10H, 9,00 (s) 1H ppm.

Methode B

a) N[1]-Benzoyl-N[2]-[2-(N-benzyl-N-(pyridin-2-yl)-amino)ethyl]-thioharnstoff

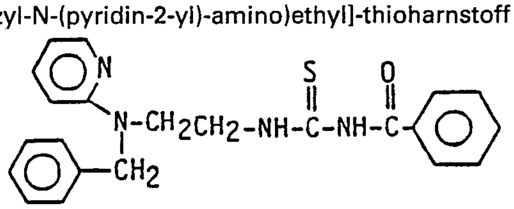

3,89 g (17 mmol) N-Benzyl-N-(pyridin-2-yl)ethylendiamin und 2,79 g (17 mmol) Benzoylisothiocyanat

werden in 70 ml Methylenchlorid 2 h unter Rückfluß gekocht. Nach Abdampfen des Lösungsmittels i. Vak. wird der Rückstand mit Essigester kristallisiert. Man erhält 3,50 g (52%) farblose Kristalle vom Schmp. 124,8—125,7°C.

$C_{22}H_{22}N_{40}S$ (390,51)

$^1$H-NMR-Daten: ($d_6$-DMSO, TMS als interner Standard): δ = 3,86 (s, verbreitert) 4H, 4,83 (s) 2H, 6,57 (dd) 1H, 6,80 (d) 1H, 7,28 (s) 5H, 7,35—7,69 (m) 4H, 7,85—8,06 (m) 2H, 8,14 (dd) 1H, 11,12 (breit 1H, austauschbvar mit $D_2O$, 11,29 (breit) 1H, austauschbar mit $D_2O$ ppm.

b) N-[2-(N-Benzyl-N-(pyridin-2-yl)-amino)ethyl]-S-methyl-isothiuroniumjodid

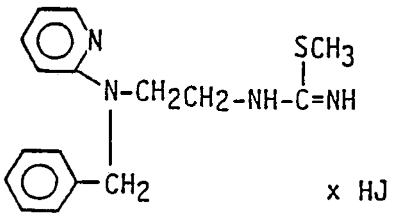

3,40 g (8,7 mmol) N[1]-Benzoyl-N[2]-[2-(N-benzyl-N-(pyridin-2-yl)-amino)ethyl]-thioharnstoff und 2,41 g (17,4 mmol) Kaliumcarbonat werden in 35 ml Wasser und 115 ml Methanol 40 min. unter Rückfluß gekocht. Nach Eindampfen im Vakuum wird der Rückstand in 50 ml Essigester aufgenommen und mit 3 × 20 ml Wasser gewaschen. Die organ. Phase wird über Natriumsulfat getrocknet, filtriert und im Vakuum eingedampft. Der erhaltene Rückstand wird in 110 ml Ethanol aufgenommen und mit 0,7 ml Methyljodid 15 h bei Raumtemperatur gerührt. Nach Abdampfen des Lösungsmittels i. Vak. wird der Rückstand mit Essigester kristallisiert. Man erhält 2,82 g (76%) des Isothiuroniumjodids in Form farbloser Kristalle vom Schmp. 152—152,8°C.

$C_{16}H_{21}JN_4S$ (428,34)

$^1$H-NMR-Daten: ($d_6$-DMSO, TMS als interner Standard): δ = 2,67 (s) 3H, 3,40—3,99 (m) 4H, 4,80 (s) 2H, 6,52—6,83 (m) 2H, 7,05—7,68 (m) 6H, 8,18 (dd) 1H, 9,53 (breit) 3H, austauschbar mit $D_2O$ ppm.

N[1]-[3-(1H-Imidazol-4-yl)propyl]-N[2]-[2-N-benzyl-N-(pyridin-2-yl)amino)-ethyl]-guanidin

1,00 g (2,3 mmol) N-[2-N-Benzyl-N-(pyridin-2-yl)-amino)ethyl]-S-methylisothiuronium-jodid und 0,28 g (2,3 mmol) 3-(1H-Imidazol-4-yl)propylamin werden in 20 ml Pyridin 3 h unter Rückfluß gekocht. Nach Abdampfen des Lösungsmittels i. Vak. wird der erhaltene Rückstand mit Essigester/Methanol (1:1) (Triethylamin) an Aluminiumoxid chromatographiert. Die Hauptfraktion ergibt nach Eindampfen 0,83 g (94%) der Titelverbindung als farblosen, amorphen Feststoff.

$C_{21}H_{27}N_7$ (377,49)

$^1$H-NMR-Daten: ($CD_{30}D$, TMS als interner Standard): δ = 1,89 (quin) 2H, 2,65 (t) 2H, 3,12—3,88 (m) 6H, 4,70 (s) 2H, 4,80 (breit) 4H, 6,56—6,78 (m) 2H, 6,86 (s) 1H, 7,14—7,64 (m) 6H, 7,60 (s) 1H, 8,13 (dd) 1H ppm.

Beispiel 95

N[1]-[3-(1H-Imidazol-4-yl)propyl]-N[2]-[2-[N-(4-chlorbenzyl)N-(pyridin-2-yl)-amino]ethyl]-guanidin

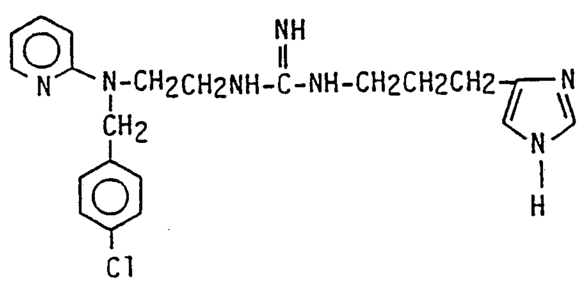

Aus 1,00 g (2,2 mmol) N-[2-[N-(4-Chlorbenzyl)N-(pyridin-2-yl)-amino]ethyl]-S-methyl-isothiuronium-jodid und 0,30 g (2,40 mmol) 3-(1H-Imidazol-4-yl)propylamin (analog Beispiel 94, Methode B) werden 0,80 g (88%) der Titelverbindung in Form eines farblosen, amorphen Feststoffes erhalten.

$C_{21}H_{26}ClN_7$ (411,94)

$^1$H-NMR-Daten: ($CD_{30}D$, TMS als interner Standard): δ = 1,75—2,21 (m) 2H, 2,70 (t) 2H, 3,16—3,93 (m) 6H, 4,70 (s) 2H, 5,4 (breit) 4H, 6,52—6,81 (m) 2H, 6,91 (s) 1H, 7,14—7,63 (m) 5H, 7,67 (s) 1H, 8,20 (dd) 1H, ppm.

Beispiel 96

$N^1$-[3-(1H-Imidazol-4-yl)propyl]-$N^2$-[2-[N-(4-methoxybenzyl)-N-(pyridin-2-yl)-amino]ethyl]-guanidin

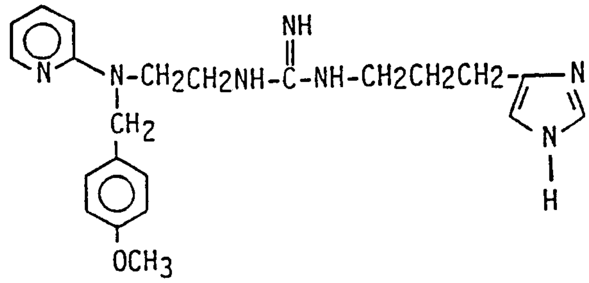

Die Titelverbindung wird analog zu Beispiel 94 aus 1,00 g (2,2 mmol) N-[2-[N-(4-Methoxybenzyl)N-(pyridin-2-yl)amino]ethyl]-S-methyl-isothiuronium-jodid und 0,30 g (2,4 mmol) 3-(1H-Imidazol-4-yl)propylamin erhalten. 0,39 g (44%) farbloser, amorpher Feststoff.

$C_{22}H_{29}N_{70}$ (407,52)

$^1$H-NMR-Daten: ($CD_{30}D$, TMS als interner Standard): δ = 1,93 (quin) 2H, 2,69 (t) 2H, 3,13—3,91 (m) 6H, 3,73 (s) 3H, 4,62 (s) 2H, 5,2 (breit) 4H, 6,56—7,73 (m) 8H, 7,64 (s) 1H, 8,16 (s) 1H ppm.

Beispiel 97

$N^1$-[3-(1H-Imidazol-4-yl)propyl]-$N^2$-[2-[N-(4-fluorbenzyl)-N-(pyridin-2-yl)-amino]ethyl]-guanidin

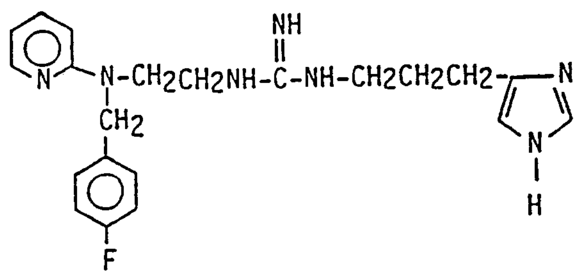

Die Herstellung erfolgt analog zu Beispiel 94 aus 2,00 g (4,48 mmol) N-[2-[N-(4-Fluorbenzyl)-N-(pyridin-2-yl)-amino]ethyl]-S-methyl-isothiuronium-jodid und 0,63 g (4,93 mmol) 3-(1H-Imidazol-4-yl)-propylamin in 40 ml Pyridin. Nach Reinigung des Rohproduktes durch präparative Schichtchromatographie und Kristallisation aus Methylenchlorid erhält man 1,08 g (61%) der Titelverbindung als beigen Feststoff. Schmp. 60—63°C.

$C_{21}H_{26}FN_7$ (395,49)

$^1$H-NMR-Daten: ($CD_{30}D$, TMS als interner Standard): δ = 1,91 (quin) 2H, 2,68 (t) 2H, 3,12—3,58 (m) 4H, 3,61—3,90 (m) 2H, 4,63 (s) 2H, 4,7 (breit) 4H, 6,58—7,65 (m) 8H, 7,73 (s) 1H, 8,16 (dd) 1H ppm.

Beispiel 98

$N^1$-[2-[N-(5-Brom-3-methyl-pyridin-2-yl)-benzylamino]ethyl]-$N^2$-[3-(1H-imidazol-4-yl)propyl]-guanidin-trihydrochlorid

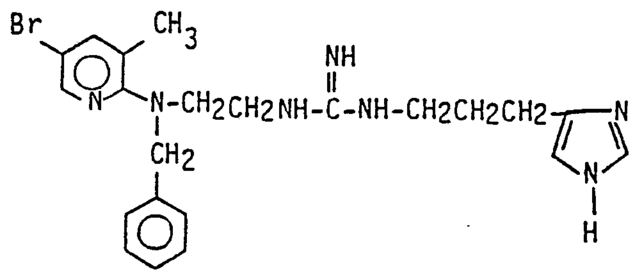

Die Herstellung erfolgt analog Beispiel 94.

0,82 g (71%) farbloser, sehr hygroskopischer Feststoff.

$C_{22}H_{31}BrCl_3N_7$ (579,80)

$^1$H-NMR-Daten: ($CD_{30}D$, TMS als interner Standard): δ = 1,80—2,18 (m) 2H, 2,61 (s) 3H, 2,89 (t) 2H, 3,34 (t) 2H, 3,60 (m) 2H, 3,83 (m) 2H, 4,15 (t) 2H, 4,9 (breit) 7H, 7,37—7,55 (m) 6H, 8,84 (d) 1H, 8,92 (d) 2H ppm.

Beispiel 99

N[1][3-(Imidazol-4-yl)propyl]-N[2]-[2-(diphenylamino)ethyl]-guanidintrihydrochlorid

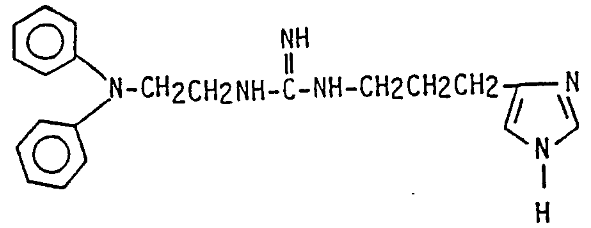

x 3 HCl

Die Herstellung erfolgt analog Beispiel 66.

$C_{21}H_{29}Cl_3N_6$ (471.9)

$^1$H-NMR-Daten: ($CD_{30}D$, TMS als interner Standard): δ = 1,81—2,20 (m) 2H, 2,90 (t) 2H, 3,30 (t) 2H, 3,60 (m) 2H, 4,13 (t) 2H, 4,85 (breit) 7H, 7,2—7,9 (m) 11H, 9,00 (s) 1H ppm.

Beispiel 100

N[1]-[3-(Imidazol-4-yl)propyl]-N[2]-[2-N-(phenyl-N-(4-fluorphenyl)amino)ethyl]-guanidin-trihydrochlorid

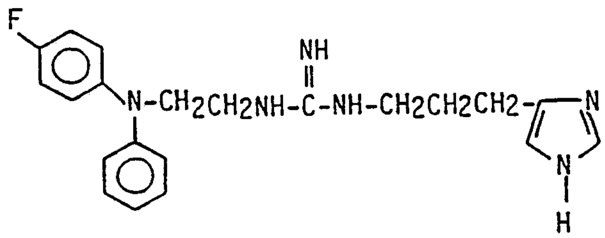

x 3 HCl

Die Herstellung erfolgt analog Beispiel 66.

$C_{21}H_{28}Cl_3FN_6$ (489,9)

$^1$H-NMR-Daten: ($CD_{30}D$, TMS als interner Standard): δ = 1,80—2,19 (m) 2H, 2,88 (t) 2H, 3,30 (t) 2H, 3,58 (m) 2H, 4,11 (t) 2H, 4,85 (breit) 7H, 7,30—7,9 (m) 10H, 9,01 (s) 1H ppm.

Beispiel 101

N[1]-[3-(Imidazol-4-yl)propyl]-N[2]-[2-(N-(2-pyridyl)-N-phenylamino)ethyl]-guanidin-trihydrochlorid

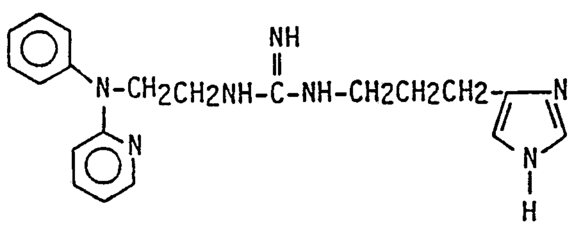

x 3 HCl

Die Herstellung erfolgt analog Beispiel 66.

$C_{20}H_{28}Cl_3N_7$ (472,9)

$^1$H-NMR-Daten: ($CD_{30}D$, TMS als interner Standard): δ = 1,81—2,20 (m) 2H, 2,87 (t) 2H, 3,32 (t) 2H, 3,60 (m) 2H, 4,12 (t) 2H, 4,90 (breit) 7H, 7,24—8,15 (m) 10H, 9,02 (s) 1H ppm.

Beispiel 102

N[1]-[3-(Imidazol-4-yl)propyl]-N[2]-[2-(N-(2-pyridyl)-N-(4-fluorphenyl)amino)ethyl]-guanidin-trihydrochlorid

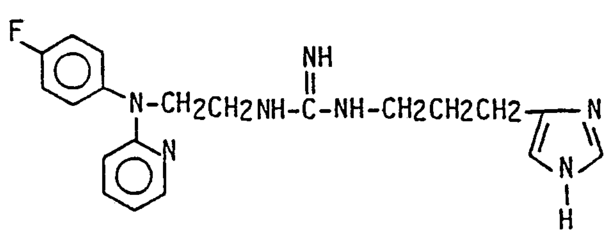

x 3 HCl

Die Herstellung erfolgt analog Beispiel 66.

$C_{20}H_{27}Cl_3FN_7$ (490,8)

$^1$H-NMR-Daten: ($CD_{30}D$, TMS als interner Standard): δ = 1,80—2,18 (m) 2H, 2,90 (t) 2H, 3,32 (t) 2H, 3,61 (m) 2H, 4,11 (t) 2H, 4,86 (breit) 7H, 7,15—8,20 (m) 9H, 9,01 (s) 1 H ppm.

**Patentansprüche für die Vertragsstaaten: DE FR GB BE NL LU IT SE CH LI**

1. Imidazolylalkylguanidinderivate der allgemeinen Formel I

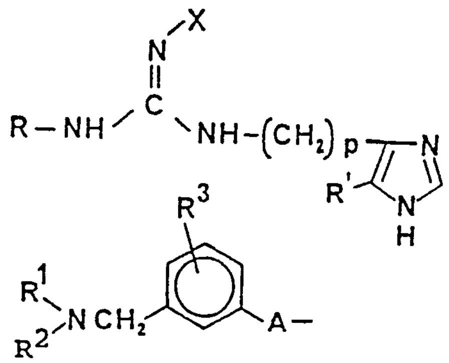

(I)

in der R die Gruppierung

bedeutet, wobei $R^1$ und $R^2$, die gleich oder verschieden sein können, jeweils für Wasserstoff, lineares $C_1$—$C_3$-Alkyl oder $C_5$—$C_6$-Cycloalkyl stehen oder $R^1$ und $R^2$ zusammen mit dem Stickstoffatom, an das sie gebunden sind, einen Pyrrolidin-, Piperidin- oder Homopiperidinring bilden, $R^3$ für ein Wasserstoffatom, ein Halogenatom oder eine $C_1$—$C_3$-Alkoxygruppe steht, A die Gruppierung

$$—O—(CH_2)_k—,\quad —O—CH_2CH(OH)CH_2—,\quad —O—CH_2CH(CH_3)—CH_2—,$$

$$—CH_2—O—CH_2—CH(OH—CH_2 \quad \text{oder} \quad —O—CH_2—CH(OH)—CH(OH)—CH_2$$

bedeutet, worin k den Wert 3 oder 4 hat, oder in der R die Gruppierung

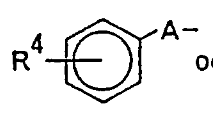

oder

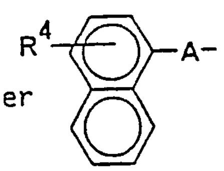

bedeutet, wobei $R^4$ für ein Wasserstoffatom, ein vorzugsweise in para-Stellung zu A gebundenes Halogenatom, eine $C_1$—$C_3$-Alkoxy- oder $C_1$—$C_3$-Alkylgruppe steht und A die oben genannte Bedeutung besitzt,
oder in der R die Gruppierung

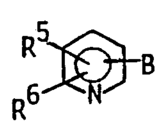

bedeutet, wobei $R^5$ und $R^6$, die gleich oder verschieden sein können, jeweils für ein Wasserstoffatom, ein Halogenatom oder eine lineare $C_1$—$C_3$-Alkyl- oder eine lineare $C_1$—$C_3$-Alkoxygruppe stehen, B in Position 2, 3 oder 4 des Pyridinrings gebunden sein kann und die Gruppierung

$$\begin{array}{l}—N—(CH_2)_1\\ \quad\; |\\ \quad\; Y\end{array}$$

bedeutet, wobei 1 den Wert 2, 3 oder 4 hat und Y für ein Wasserstoffatom oder für eine lineare $C_1$—$C_3$-Alkylgruppe steht,
oder in der R die Gruppierung R″—A′—B′— bedeutet, wobei R″ für eine unsubstituierte oder mit einem Halogenatom, einer $C_1$—$C_3$-Alkylgruppe oder einer $C_1$—$C_3$-Alkoxygruppe substituierte Phenyl- oder Naphthylgruppe steht. A′ für eine Einfachbindung oder für die Gruppierung $—CR^{1'}R^{2'}$ oder für ein — mit einer gegebenenfalls mit Halogenatomen substituierten Phenyl- oder Benzylgruppe oder linearen $C_1$—$C_3$-Alkylgruppe substituiertes — Stickstoffatom steht, wobei $R^{1'}$ ein Wasserstoffatom oder eine Methylgruppe bedeutet. $R^{2'}$ für eine gegebenenfalls mit einem Halogenatom oder einer $C_1$—$C_3$-Alkylgruppe substituierte Phenyl- oder Pyridylgruppe steht.

B′ für die Gruppierungen

$$—CH(Y)\text{-}S\text{-}(CH_2)_{m'}—,\ —CH_2—S—CH_2—CH(Y)—CH_2—,\ —CH_2—S—CH(Y)—CH_2—,$$

$$—CH_2—S—CH_2—CH(Y)—,\ —(CH_2)_{n''}—,\ —CH_2—CH(Y)—,$$

$$—(CH_2)_{n''}—CH(Y)—,\ —O—(CH_2)_2—,\ —CH_2—O—(CH_2)_{o'}—,$$

$$—CH_2—O—CH_2—CH(Y)—CH_2—,\ —O—CH_2—CH(Y)—,\ —O—CH(Y)—CH_2—,$$

$$—S—(CH_2)_q—,\ —S—CH_2—CH(Y)—,\ —S—CH(Y)—CH_2—\ \text{oder}\ —S—CH_2—CH(Y)—CH_2$$

steht, wobei Y ein Wasserstoffatom oder eine lineare $C_1—C_3$-Alkylgruppe bedeutet, m′ und o′ den Wert 2 oder 3 haben, n″ und q den Wert 2, 3, 4 oder 5 haben, oder in der R die Gruppierung R‴—A″—B″— bedeutet, wobei R‴ für eine unsubstituierte oder mit Halogenatom, $C_1—C_3$-Alkylgruppen oder der Gruppe $R^1R^2N—CH_2—$ substituierte Pyridyl-, Thiophenyl-, Furanyl-, Chinolyl- oder Benzimidazolylgruppe steht, A″ für eine Einfachbindung oder für die Gruppierung $—CR^{1'}R^{2'}$ oder für ein — mit einer gegebenenfalls mit Halogenatomen, $C_1—C_3$-Alkylgruppen oder $C_1—C_3$-Alkoxygruppen substituierte Phenyl- oder Benzylgruppe substituiertes — Stickstoffatom steht, wobei $R^{1'}$ ein Wasserstoffatom oder eine Methylgruppe bedeutet, $R^{2'}$ für eine gegebenenfalls mit Halogenatomen substituierte Phenyl- oder Thiophenylgruppe steht, mit der Maßgabe, daß A″ nicht für eine Einfachbindung steht, wenn R‴ eine Pyridylgruppe ist,

B″ für die Gruppierung

$$—CH(Y)—S—(CH_2)_{m'},\ —CH_2—S—CH_2—CH(Y)—CH_2—,\ —CH_2—S—CH(Y)—CH_2—,$$

$$—(CH_2)_{n''}—CH(Y)—,\ —CH_2—S—CH_2—CH(Y)—,\ —(CH_2)_{n''}—,$$

$$—O(CH_2)_{n''}—,\ —S—CH_2—CH(Y)—,\ —S—CH(Y)—CH_2—,$$

$$—S—(CH_2)_q—\ \text{oder}\ —S—CH_2—CH(Y)—CH_2—$$

steht, wobei Y ein Wasserstoffatom oder eine lineare $C_1—C_3$-Alkylgruppe bedeutet, m′ den Wert 2 oder 3 hat und n″ und q den Wert 2, 3, 4 oder 5 haben, X ein Wasserstoffatom oder eine Benzoylgruppe bedeutet, p den Wert 2 oder 3 hat und R′ ein Wasserstoffatom oder eine Methylgruppe bedeutet, sowie die physiologisch annehmbaren Salze davon.

2. Imidazolylalkylguanidinderivate nach Anspruch 1, dadurch gekennzeichnet, daß R für die Gruppierung

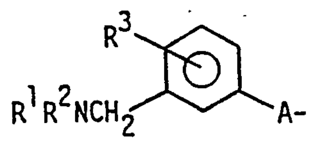

steht, $R^1$ und $R^2$ zusammen mit dem Stickstoffatom einen Pyrrolidin- oder Piperidinring bilden, $R^3$ für ein Wasserstoffatom steht, A die Gruppierung $—O—(CH_2)_k—$, $—O—CH_2CH(OH)CH_2—$ oder $—O—CH_2CH(CH_3)CH_2—$ bedeutet, X und R′ jeweils für ein Wasserstoffatom stehen und k und p jeweils denn Wert 3 haben.

3. Imidazolylalkylguanidinderivate nach Anspruch 1, dadurch gekennzeichnet, daß R für die Gruppierung

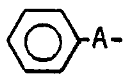

oder

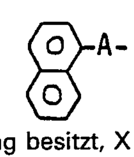

steht, wobei A die in Anspruch 1 genannte Bedeutung besitzt, X und R′ jeweils für ein Wasserstoffatom stehen und p den Wert 3 hat.

4. Imidazolylalkylguanidinderivate nach Anspruch 1, dadurch gekennzeichnet, daß R für die Gruppierung

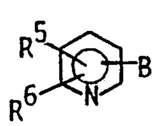

steht, wobei $R^5$ für ein in 5-Stellung des Pyridinrings gebundenes Halogenatom oder Wasserstoffatom steht, $R^6$ für ein in 3-Stellung des Pyridinrings gebundenes Wasserstoffatom oder eine Methyl- oder Methoxygruppe steht, B in Position 2, 3 oder 4 des Pyridinrings gebunden sein kann und die Gruppierung

$$—N—(CH_2)_3— \atop {|\atop CH_3}$$

bedeutet, X und R' jeweils für ein Wasserstoffatom stehen und p den Wert 3 hat.

5. Imidazolylalkylguanidinderivate nach Anspruch 1, dadurch gekennzeichnet, daß R die Gruppierung R''—A'—B'— bedeutet, wobei R'' für eine unsubstituierte Phenylgruppe steht, A' eine Einfachbindung bedeutet, B' für die Gruppierung $—CH_2—S—(CH_2)_{m'}—$ oder $—CH_2—S—CH_2—CH(Y)—CH_2—$ steht, wobei m' und Y die oben genannten Bedeutungen besitzen, X und R' jeweils für ein Wasserstoffatom stehen und p den Wert 3 hat.

6. Imidazolylalkylguanidinderivate nach Anspruch 1, dadurch gekennzeichnet, daß R die Gruppierung R''—A'—B'— bedeutet, wobei R'' für eine mit einem Halogenatom, einer $C_1—C_3$— Alkylgruppe oder einer $C_1—C_3$-Alkoxygruppe substituierte oder unsubstituierte Phenylgruppe steht, A' ein mit einer Phenyloder Benzylgruppe substituiertes Stickstoffatom bedeutet, B' für die Gruppierung $—(CH_2)_{n''}—$ steht, wobei n'' den Wert 2 oder 3 hat, X und R' jeweils für ein Wasserstoffatom stehen und p den Wert 3 besitzt.

7. Imidazolylalkylguanidinderivate nach Anspruch 1, dadurch gekennzeichnet, daß R die Gruppierung R''—A'—B'— bedeutet, wobei R'' für eine mit einem Halogenatom, einer $C_1—C_3$— Alkylgruppe oder einer $C_1—C_3$-Alkoxygruppe substituierte oder unsubstituierte Phenylgruppe steht, A' für die Gruppierung $—CR^{1'}R^{2'}$, wobei $R^{1'}$ ein Wasserstoffatom oder eine Methylgruppe bedeutet, $R^{2'}$ für eine gegebenenfalls mit einem Halogenatom oder einer $C_1—C_3$-Alkylgruppe substituierte Phenyl- oder Pyridylgruppe steht, B' die Gruppierung $—(CH_2)_{n''}$- bedeutet, wobei n'' den Wert 2, 3 oder 4 hat, X und R' jeweils fur ein Wasserstoffatom stehen und p den Wert 3 hat.

8. Imidazolylalkylguanidinderivate nach Anspruch 1, dadurch gekennzeichnet, daß R die Gruppierung R'''—A''—B''— bedeutet, wobei R''' für einen mit Halogenatomen, $C_1—C_3$-Alkylgruppen oder der Gruppe $R^1R^2NCH_2$ substituierten oder unsubstituierten Thiophenring steht, A'' eine Einfachbindung bedeutet, B'' für die Gruppierung $—CH_2—S—(CH_2)_{m'}$- oder $—CH_2—S—CH_2—CH(Y)—CH_2—$ steht, wobei m' und Y die oben genannten Bedeutungen besitzen, X und R' jeweils für ein Wasserstoffatom stehen und p den Wert 3 besitzt.

9. Imidazolylalkylguanidinderivate nach Anspruch 1, dadurch gekennzeichnet, daß R die Gruppierung R'''—A''—B''— bedeutet, wobei R''' für einen mit Halogenatomen oder $C_1—C_3$-Alkylgruppen substituierten oder unsubstituierten Pyridinring steht, A'' die Gruppierung $—CR^{1'}R^{2'}$ bedeutet, wobei $R^{1'}$ und $R^{2'}$ wie in Anspruch 1 definiert sind, B'' für die Gruppierung $—(CH_2)_{n''}—$ steht, wobei n'' die in Anspruch 1 genannte Bedeutung besitzt, X und R' jeweils für ein Wasserstoffatom stehen und p den Wert 3 besitzt.

10. Imidazolylalkylguanidinderivate nach Anspruch 1, dadurch gekennzeichnet, daß R die Gruppierung R'''—A''—B''— bedeutet, wobei R''' für einen mit Halogenatomen oder $C_1—C_3$-Alkylgruppen substituierten oder unsubstituierten Pyridinring steht, A'' für ein — mit einer gegebenenfalls mit Halogenatomen, $C_1—C_3$-Alkylgruppen oder $C_1—C_3$-Alkoxygruppen substituierten Phenyl- oder Benzylgruppe — substituiertes Stickstoffatom steht, B'' für die Gruppierung $—(CH_2)_{n''}—$ steht, wobei n'' die in Anspruch 1 genannte Bedeutung besitzt, X und R' jeweils für ein Wasserstoffatom stehen und p den Wert 3 besitzt.

11. N-[3-[(N-5-Methyl-pyridin-2-yl)-methylamino]propyl]-N'-[3-[1H-imidazol-4-yl)propyl]guanidin und die physiologisch annehmbaren Salze.

12. N-[3-(Imidazol-4-yl)propyl]-N'-(3,3-diphenylpropyl)guanidin und die physiologisch annehmbaren Salze.

13. N-[3-(Imidazol-4-yl)propyl]-N'-[2-[(1-phenylethyl)thio]ethyl]guanidin und die physiologisch annehmbaren Salze.

14. N-[3-(Imidazol-4-yl)propyl]-N'-[2-[(pyrid-3-yl)methylthio]ethyl]guanidin und die physiologisch annehmbaren Salze.

15. N-[3-(Imidazol-4-yl)propyl]-N'-[2-(2-thenylthio)ethyl]guanidin und die physiologisch annehmbaren Salze.

16. N-[3-(Imidazol-4-yl)propyl]-N'-[1-methyl-2-[(pyrid-2-yl)methylthio]ethyl]guanidin und die physiologisch annehmbaren Salze.

17. N-[3-(Imidazol-4-yl)propyl]-N'-[3-phenyl-3-(pyrid-2-yl)propyl]guanidin und die physiologisch annehmbaren Salze.

18. N-[3-(4-Chlorphenyl)-3-(pyrid-2-yl)propyl]-N'-[3-(imidazol-4-yl)propyl]guanidin und die physiologisch annehmbaren Salze.

19. N-[3-(4-Bromphenyl)-3-(pyrid-2-yl)propyl]-N'-[3-(imidazol-4-yl)propyl]guanidin und die physiologisch annehmbaren Salze.

20. N-[3-(4-Fluorphenyl)-3-(pyrid-2-yl)propyl]-N'-[3-(imidazol-4-yl)propyl]guanidin und die physiologisch annehmbaren Salze.

21. Verfahren zur Herstellung von Imidazolylalkylguanidinderivaten nach den Ansprüchen 1 bis 20 sowie der physiologisch annehmbaren Salze davon, dadurch gekennzeichnet, daß man

a) zur Herstellung von Verbindungen der allgemeinen Formel I, bei denen R, A, B, p und R′ wie in Anspruch 1 definiert sind und X für eine Benzoylgruppe steht,

($a_1$) eine Verbindung der allgemeinen Formel II

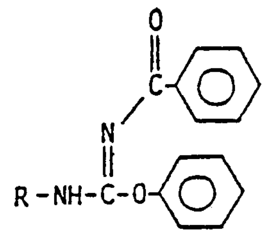

(II)

in der R die oben angegebene Bedeutung besitzt, mit einer Verbindung der allgemeinen Formel III

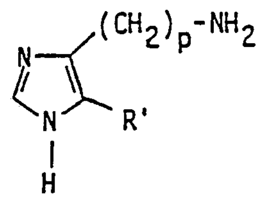

(III)

in der R′ und p die in Anspruch 1 angegebenen Bedeutungen besitzen, zu einer Verbindung der allgemeinen Formel I umsetzt oder

($a_2$) eine Verbindung der allgemeinen Formel II

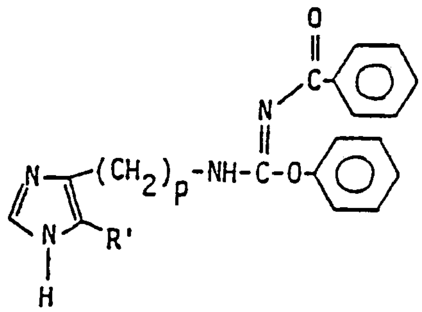

(IV)

in der R′ und p die in Anspruch 1 angegebenen Bedeutungen besitzen, mit einer Verbindung der allgemeinen Formel V

$$R{-}NH_2 \qquad (V)$$

in der R die in Anspruch 1 angegebene Bedeutung besitzt, zu einer Verbindung der allgemeinen Formel I umsetzt oder

b) zur Herstellung von Verbindungen der allgemeinen Formel I, bei denen R, A, B, p und R′ wie in Anspruch 1 definiert sind und X für ein Wasserstoffatom steht,

($b_1$) eine Verbindung der Formel Ia

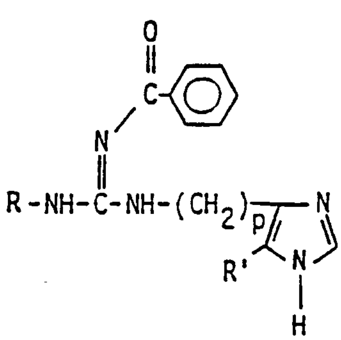

(Ia)

in der R, P und R′ die in Anspruch 1 angegebenen Bedeutungen besitzen, hydrolysiert oder

($b_2$) eine Verbindung der allgemeinen Formel VI

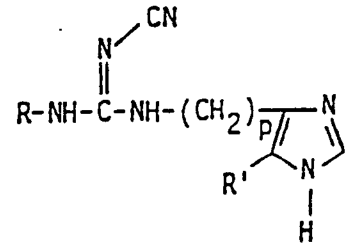

(VI)

in der R, p und R′ die in Anspruch 1 angegebenen Bedeutungen besitzen, mit Hilfe einer Säure zu einer Verbindung der allgemeinen Formel I hydrolysiert oder

($b_3$) eine Verbindung der allgemeinen Formel VII

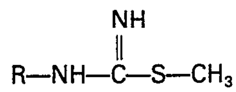

(VII)

in der R die in Anspruch 1 angegebene Bedeutung besitzt, mit einer Verbindung der oben angegebenen allgemeinen Formel III, in der R′ und p die in Anspruch 1 angegebenen Bedeutungen besitzen, zu einer Verbindung der allgemeinen Formel I umsetzt oder

($b_4$) eine Verbindung der allgemeinen Formel VIII

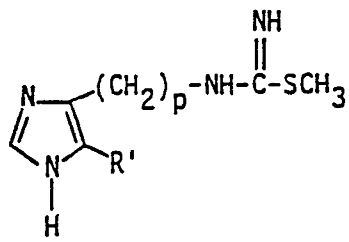

(VIII)

in der R′ und p die in Anspruch 1 angegebenen Bedeutungen besitzen, mit einer Verbindung der oben angegebenen allgemeinen Formel V, in der R die in Anspruch 1 angegebene Bedeutung besitzt, zu einer Verbindung der allgemeinen Formel I umsetzt und daß man gegebenenfalls die unter a) und b) erhaltenen Verbindungen in ihr physiologisch annehmbares Salz umwandelt.

22. Arzneimittel, dadurch gekennzeichnet, daß es eine Verbindung nach den Ansprüchen 1 bis 20 und mindestens einen inerten, pharmazeutisch annehmbaren Träger oder ein inertes, pharmazeutisch annehmbares Verdünnungsmittel enthält.

**Patentansprüche für den Vertragsstaat: AT**

1. Verfahren zur Herstellung von Imidazolylalkylguanidinderivaten der allgemeinen Formel I

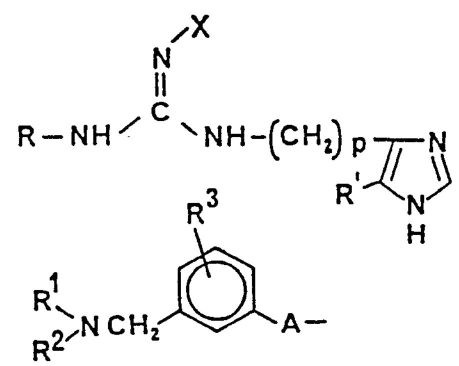

(I)

in der R die Gruppierung

bedeutet, wobei $R^1$ und $R^2$, die gleich oder verschieden sein können, jeweils für Wasserstoff, lineares $C_1$—$C_3$-Alkyl oder $C_5$—$C_6$-Cycloalkyl stehen oder $R^1$ und $R^2$ zusammen mit dem Stickstoffatom, an das sie gebunden sind, einen Pyrrolidin-, Piperidin- oder Homopiperidinring bilden, $R^3$ für ein Wasserstoffatom, ein Halogenatom oder eine $C_1$—$C_3$-Alkoxygruppe steht, A die Gruppierung

$—O—(CH_2)_k—$, $—O—CH_2CH(OH)CH_2—$, $—O—CH_2CH(CH_3)—CH_2—$,

$—CH_2—O—CH_2—CH(OH)—CH_2$ oder $—O—CH_2—CH(OH)—CH(OH)—CH_2$

bedeutet, worin k den Wert 3 oder 4 hat, oder in der R die Gruppierung

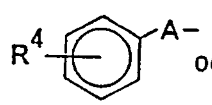 oder 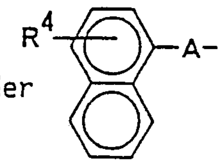

bedeutet, wobei $R^4$ für ein Wasserstoffatom, ein vorzugsweise in para-Stellung zu A gebundenes Halogenatom, eine $C_1$—$C_3$-Alkoxy- oder $C_1$—$C_3$-Alkylgruppe steht und A die oben genannte Bedeutung besitzt,
oder in der R die Gruppierung

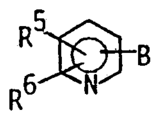

bedeutet, wobei $R^5$ und $R^6$, die gleich oder verschieden sein können, jeweils für ein Wasserstoffatom, ein Halogenatom oder eine lineare $C_1$—$C_3$-Alkyl- oder eine lineare $C_1$—$C_3$-Alkoxygruppe stehen, B in Position 2, 3 oder 4 des Pyridinrings gebunden sein kann und die Gruppierung

$$-N-(CH_2)_1 \quad (N \text{ substituiert mit } Y)$$

bedeutet, wobei 1 den Wert 2, 3 oder 4 hat und Y für ein Wasserstoffatom oder für eine lineare $C_1$—$C_3$-Alkylgruppe steht,
oder in der R die Gruppierung R''—A'—B'— bedeutet, wobei R'' für eine unsubstituierte oder mit einem Halogenatom, einer $C_1$—$C_3$-Alkylgruppe oder einer $C_1$—$C_3$-Alkoxygruppe substituierte Phenyl- oder Naphthylgruppe steht. A' für eine Einfachbindung oder für die Gruppierung —$CR^{1'}R^{2'}$ oder für ein — mit einer gegebenenfalls mit Halogenatomen substituierten Phenyl- oder Benzylgruppe oder linearen $C_1$—$C_3$-Alkylgruppe substituiertes — Stickstoffatom steht, wobei $R^{1'}$ ein Wasserstoffatom oder eine Methylgruppe bedeutet. $R^{2'}$ für eine gegebenenfalls mit einem Halogenatom oder einer $C_1$—$C_3$-Alkylgruppe substituierte Phenyl- oder Pyridylgruppe steht.
B' für die Gruppierungen

$$-CH(Y)\text{-}S\text{-}(CH_2)_{m'}-,\quad -CH_2-S-CH_2-CH(Y)-CH_2-,\quad -CH_2-S-CH(Y)-CH_2-,$$

$$-CH_2-S-CH_2-CH(Y)-,\quad -(CH_2)_{n''}-,\quad -CH_2-CH(Y)-,$$

$$-(CH_2)_{n''}-CH(Y)-,\quad -O-(CH_2)_2-,\quad -CH_2-O-(CH_2)_{o'}-,$$

$$-CH_2-O-CH_2-CH(Y)-CH_2-,\quad -O-CH_2-CH(Y)-,\quad -O-CH(Y)-CH_2-,$$

$$-S-(CH_2)_q-,\quad -S-CH_2-CH(Y)-,\quad -S-CH(Y)-CH_2- \quad \text{oder} \quad -S-CH_2-CH(Y)-CH_2$$

steht, wobei Y ein Wasserstoffatom oder eine lineare $C_1$—$C_3$-Alkylgruppe bedeutet, m' und o' den Wert 2 oder 3 haben, n'' und q den Wert 2, 3, 4 oder 5 haben,
oder in der R die Gruppierung R'''—A''—B''— bedeutet, wobei R''' für eine unsubstituierte oder mit Halogenatom, $C_1$—$C_3$-Alkylgruppen oder der Gruppe $R^1R^2N$—$CH_2$— substituierte Pyridyl-, Thiophenyl-, Furanyl-, Chinolyl- oder Benzimidazolylgruppe steht, A'' für eine Einfachbindung oder für die Gruppierung —$CR^{1'}R^{2'}$ oder für ein — mit einer gegebenenfalls mit Halogenatomen, $C_1$—$C_3$-Alkylgruppen oder $C_1$—$C_3$-Alkoxygruppen substituierte Phenyl- oder Benzylgruppe substituiertes — Stickstoffatom steht, wobei $R^{1'}$ ein Wasserstoffatom oder eine Methylgruppe bedeutet, $R^{2'}$ für eine gegebenenfalls mit Halogenatomen substituierte Phenyl- oder Thiophenylgruppe steht, mit der Maßgabe, daß A'' nicht für eine Einfachbindung steht, wenn R''' eine Pyridylgruppe ist,
B'' für die Gruppierung

$$-CH(Y)-S-(CH_2)_{m'},\quad -CH_2-S-CH_2-CH(Y)-CH_2-,\quad -CH_2-S-CH(Y)-CH_2-,$$

$$-(CH_2)_{n''}-CH(Y)-,\quad -CH_2-S-CH_2-CH(Y)-,\quad -(CH_2)_{n''}-,$$

$$-O(CH_2)_{n''}-,\quad -S-CH_2-CH(Y)-,\quad -S-CH(Y)-CH_2-,$$

$$-S-(CH_2)_q- \quad \text{oder} \quad -S-CH_2-CH(Y)-CH_2-$$

steht, wobei Y ein Wasserstoffatom oder eine lineare $C_1$—$C_3$-Alkylgruppe bedeutet, m′ den Wert 2 oder 3 hat und n″ und q den Wert 2, 3, 4 oder 5 haben, X ein Wasserstoffatom oder eine Benzoylgruppe bedeutet, p den Wert 2 oder 3 hat und R′ ein Wasserstoffatom oder eine Methylgruppe bedeutet, sowie die physiologisch annehmbaren Salze davon, dadurch gekennzeichnet, daß man

a) zur Herstellung von Verbindungen der allgemeinen Formel I, bei denen R, A, B, p und R′ wie oben definiert sind und X für eine Benzoylgruppe steht,

($a_1$) eine Verbindung der allgemeinen Formel II

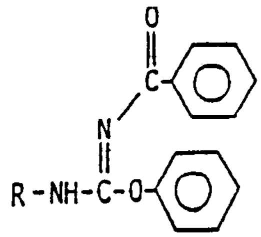

(II)

in der R die oben angegebene Bedeutung besitzt, mit einer Verbindung der allgemeinen Formel III

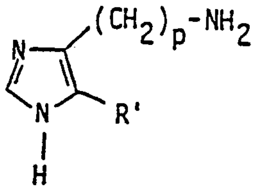

(III)

in der R′ und p die oben angegebenen Bedeutungen besitzen, zu einer Verbindung der allgemeinen Formel I umsetzt oder

($a_2$) eine Verbindung der allgemeinen Formel IV

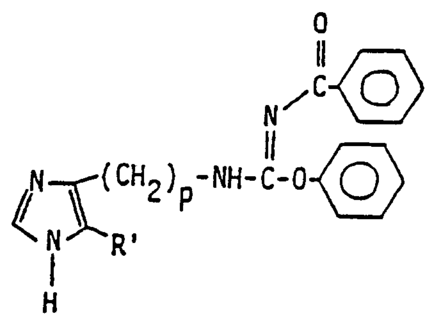

(IV)

in der R′ und p die oben angegebenen Bedeutungen besitzen, mit einer Verbindung der allgemeinen Formel V

$$R—NH_2 \qquad (V)$$

in der R die oben angegebene Bedeutung besitzt, zu einer Verbindung der allgemeinen Formel I umsetzt oder

b) zur Herstellung von Verbindungen der allgemeinen Formel I, bei denen R, A, B, p und R′ wie oben definiert sind und X für ein Wasserstoffatom steht,

($b_1$) eine Verbindung der Formel Ia

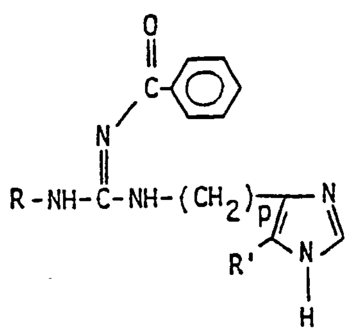

(Ia)

in der R, p und R′ die oben angegebenen Bedeutungen besitzen, hydrolysiert oder

($b_2$) eine Verbindung der allgemeinen Formel VI

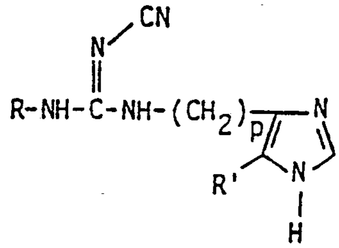

(VI)

in der R, p und R′ die oben angegebenen Bedeutungen besitzen, mit Hilfe einer Säure zu einer Verbindung der allgemeinen Formel I hydrolysiert oder

($b_3$) eine Verbindung der allgemeinen Formel VII

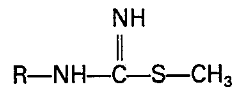

(VII)

in der R die oben angegebene Bedeutung besitzt, mit einer Verbindung der oben angegeben allgemeinen Formel III, in der R′ und p die oben angegebenen Bedeutungen besitzen, zu einer Verbindung der allgemeinen Formel I umsetzt oder

($b_4$) eine Verbindung der allgemeinen Formel VIII

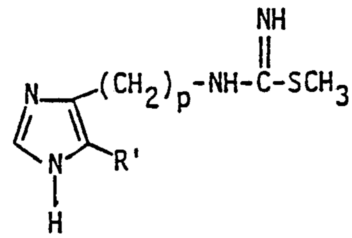

(VIII)

in der R′ und p die oben angegebenen Bedeutungen besitzen, mit einer Verbindung der oben angegebenen allgemeinen Formel V, in der R die oben angegebene Bedeutung besitzt, zu einer Verbindung der allgemeinen Formel I umsetzt und daß man gegebenenfalls die unter a) und b) erhaltenen Verbindungen in ihr physiologisch annehmbares Salz umwandelt.

2. Verfahren nach Anspruch 1 zur Herstellung von Verbindungen, bei denen R für die Gruppierung

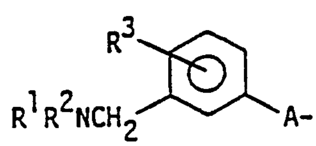

steht, $R^1$ und $R^2$ zusammen mit dem Stickstoffatom einen Pyrrolidin- oder Piperidinring bilden, $R^3$ für ein Wasserstoffatom steht, A die Gruppierung $-O-(CH_2)_k-$, $-O-CH_2CH(OH)CH_2-$ oder $-O-CH_2CH(CH_3)CH_2-$ bedeutet, X und R′ jeweils für ein Wasserstoffatom stehen und k und p jeweils den Wert 3 haben.

3. Verfahren nach Anspruch 1 zur Herstellung von Verbindungen, bei denen R für die Gruppierung

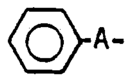 oder 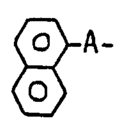

steht, wobei A die in Anspruch 1 genannte Bedeutung besitzt, X und R′ jeweils für ein Wasserstoffatom stehen und p den Wert 3 hat.

4. Verfahren nach Anspruch 1 zur Herstellung von Verbindungen, bei denen R für die Gruppierung

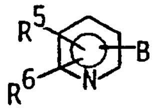

steht, wobei $R^5$ für ein in 5-Stellung des Pyridinrings gebundenes Halogenatom oder Wasserstoffatom steht, $R^6$ für ein in 3-Stellung des Pyridinrings gebundenes Wasserstoffatom oder eine Methyl- oder Methoxygruppe steht, B in Position 2, 3 oder 4 des Pyridinrings gebunden sein kann und die Gruppierung

$$-\underset{\displaystyle CH_3}{\underset{|}{N}}-(CH_2)_3-$$

bedeutet, X und R' jeweils für ein Wasserstoffatom stehen und p den Wert 3 hat.

5. Verfahren nach Anspruch 1 zur Herstellung von Verbindungen, bei denen R die Gruppierung R''—A'—B'— bedeutet, wobei R'' für eine unsubstituierte Phenylgruppe steht, A' eine Einfachbindung bedeutet, B' für die Gruppierung $-CH_2-S-(CH_2)_{m'}-$ oder $-CH_2-S-CH_2-CH(Y)-CH_2-$ steht, wobei m' und Y die oben genannten Bedeutungen besitzen, X und R' jeweils für ein Wasserstoffatom stehen und p den Wert 3 hat.

6. Verfahren nach Anspruch 1 zur Herstellung von Verbindungen, bei denen R die Gruppierung R''—A'—B'— bedeutet, wobei R'' für eine mit einem Halogenatom, einer $C_1$—$C_3$— Alkylgruppe oder einer $C_1$—$C_3$-Alkoxygruppe substituierte oder unsubstituierte Phenylgruppe steht, A' ein mit einer Phenyl- oder Benzylgruppe substituiertes Stickstoffatom bedeutet, B' für die Gruppierung $-(CH_2)_{n''}-$ steht, wobei n'' den Wert 2 oder 3 hat, X und R' jeweils für ein Wasserstoffatom stehen und p den Wert 3 besitzt.

7. Verfahren nach Anspruch 1 zur Herstellung von Verbindungen, bei denen R die Gruppierung R''—A'—B'— bedeutet, wobei R'' für eine mit einem Halogenatom, einer $C_1$—$C_3$— Alkylgruppe oder einer $C_1$—$C_3$-Alkoxygruppe substituierte oder unsubstituierte Phenylgruppe steht, A' für die Gruppierung $-CR^{1'}R^{2'}$, wobei $R^{1'}$ ein Wasserstoffatom oder eine Methylgruppe bedeutet, $R^{2'}$ für eine gegebenenfalls mit einem Halogenatom oder einer $C_1$—$C_3$-Alkylgruppe substituierte Phenyl- oder Pyridylgruppe steht, B' die Gruppierung $-(CH_2)_{n''}$- bedeutet, wobei n'' den Wert 2, 3 oder 4 hat, X und R' jeweils fur ein Wasserstoffatom stehen und p den Wert 3 hat.

8. Verfahren nach Anspruch 1 zur Herstellung von Verbindungen, bei denen R die Gruppierung R'''—A''—B''— bedeutet, wobei R''' für einen mit Halogenatomen, $C_1$—$C_3$-Alkylgruppen oder der Gruppe $R^1R^2NCH_2$ substituierten oder unsubstituierten Thiophenring steht, A'' eine Einfachbindung bedeutet, B'' für die Gruppeirung $-CH_2-S-(CH_2)_{m'}$- oder $-CH_2-S-CH_2-CH(Y)-CH_2-$ steht, wobei m' und Y die oben genannten Bedeutungen besitzen, X und R' jeweils für ein Wasserstoffatom stehen und p den Wert 3 besitzt.

9. Verfahren nach Anspruch 1 zur Herstellung von Verbindungen, bei denen R die Gruppierung R'''—A''—B''— bedeutet, wobei R''' für einen mit Halogenatomen oder $C_1$—$C_3$-Alkylgruppen substituierten oder unsubstituierten Pyridinring steht, A'' die Gruppierung $-CR^{1'}R^{2'}$ bedeutet, wobei $R^{1'}$ und $R^{2'}$ wie in Anspruch 1 definiert sind, B'' für die Gruppierung $-(CH_2)_{n''}-$ steht, wobei n'' die in Anspruch 1 genannte Bedeutung besitzt, X und R' jeweils für ein Wasserstoffatom stehen und p den Wert 3 besitzt.

10. Verfahren nach Anspruch 1 zur Herstellung von Verbindungen, bei denen R die Gruppierung R'''—A''—B''— bedeutet, wobei R''' für einen mit Halogenatomen oder $C_1$—$C_3$-Alkylgruppen substituierten oder unsubstituierten Pyridinring steht, A'' für ein — mit einer gegebenenfalls mit Halogenatomen, $C_1$—$C_3$-Alkylgruppen oder $C_1$—$C_3$-Alkoxygruppen substituierten Phenyl- oder Benzylgruppe — substituiertes Stickstoffatom steht, B'' für die Gruppierung $-(CH_2)_{n''}-$ steht, wobei n'' die in Anspruch 1 genannte Bedeutung besitzt, X und R' jeweils für ein Wasserstoffatom stehen und p den Wert 3 besitzt.

11. Verfahren nach Anspruch 1 zur Herstellung von N—[3-[(N-5-Methyl-pyridin-2-yl)-methylamino]propyl]-N'-[3-[1H-imidazol-4-yl)propyl]guanidin und die physiologisch annehmbaren Salze davon.

12. Verfahren nach Anspruch 1 zur Herstellung von N-[3-(Imidazol-4-yl)propyl]-N'-(3,3-diphenylpropyl)guanidin und die physiologisch annehmbaren Salze davon.

13. Verfahren nach Anspruch 1 zur Herstellung von N-[3-(Imidazol-4-yl)propyl]-N'-[2-[(1-phenylethyl)thio]ethyl]guanidin und der physiologisch annehmbaren Salze davon.

14. Verfahren nach Anspruch 1 zur Herstellung von N-[3-(Imidazol-4-yl)propyl]-N'-[2-[(pyrid-3-yl)methylthio]ethyl]guanidin und der physiologisch annehmbaren Salze davon.

15. Verfahren nach Anspruch 1 zur Herstellung von N-[3-(Imidazol-4-yl)propyl]-N'-[2-(2-thenylthio)ethyl]guanidin und die physiologisch annehmbaren Salze davon.

16. Verfahren nach Anspruch 1 zur Herstellung von N-[3-(Imidazol-4-yl)propyl]-N'-[1-methyl-2-[(pyrid-2-yl]methylthio]ethyl]guanidin und die physiologisch annehmbaren Salze davon.

17. Verfahren nach Anspruch 1 zur Herstellung von N-[3-(Imidazol-4-yl)propyl]-N'-[3-phenyl-3-(pyrid-2-yl)propyl]guanidin und die physiologisch annehmbaren Salze davon.

18. Verfahren nach Anspruch 1 zur Herstellung von N-[3-(4-Chlorphenyl)-3-(pyrid-2-yl)propyl]-N'-[3-(imidazol-4-yl)propyl]guanidin und der physiologisch annehmbaren Salze davon.

19. Verfahren nach Anspruch 1 zur Herstellung von N-[3-(4-Bromphenyl)-3-(pyrid-2-yl)propyl]-N'-[3-(imidazol-4-yl)propyl]guanidin und der physiologisch annehmbaren Salze davon.

20. Verfahren nach Anspruch 1 zur Herstellung von N-[3-(4-Fluorphenyl)-3-(pyrid-2-yl)propyl]-N'-[3-(imidazol-4-yl)propyl]guanidin und der physiologisch annehmbaren Salze davon.

**Revendications pour les Etats contractants: BE CH DE FR GB IT LI LU NL SE**

1. Dérivés d'imidazolylalkylguanidine de formule générale I:

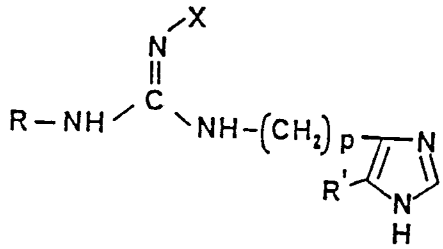

(I)

dans laquelle R signifie le groupe

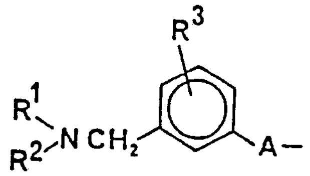

où $R^1$ et $R^2$, identiques ou différents, représentent l'hydrogène, un groupe alkyle linéaire de 1 à 3 atomes de carbone, ou bien un groupe cycloalkyle de 5 ou 6 atomes de carbone, ou bien $R^1$ et $R^2$ constituent avec l'atome d'azote lequel ils sont liés un cycle pyrrolidine, pipéridine ou homopipéridine, $R^3$ désigne l'hydrogène, un atome d'halogène ou un groupe alkyle de 1 à 3 atomes de carbone, A signifie les groupes

$$—O—(CH_2)_k—,\ —O—CH_2CH(OH)CH_2—,\ —O—CH_2CH(CH_3)—CH_2—,$$

$$—CH_2—O—CH_2—CH(OH)—CH_2 \text{ ou } —O—CH_2—CH(OH)—CH(OH)—CH_2$$

où k a la valeur 3 ou 4, ou bien dans laquelle R représente le groupe

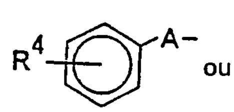 ou 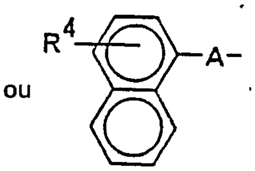

dans lequel $R^4$ signifie un atome d'hydrogène, un atome d'halogène lié avantageusement en position para par rapport à A, un groupe alcoxy de 1 à 3 atomes de carbone ou alkyle de 1 à 3 atomes de carbone et A a la signification indiquée ci-dessus, ou bien dans laquelle R représente le groupe

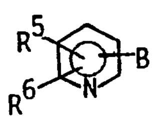

dans lequel $R^5$ et $R^6$, identiques ou différents, représentent un atome d'hydrogène, un atome d'halogène ou un groupe alkyle linéaire de 1 à 3 atomes de carbone ou un groupe alcoxy linéaire de 1 à 3 atomes de carbone, B étant lié au cycle pyridine en position 2, 3 ou 4, et le groupe

$$\begin{array}{l}—N—(CH_2)_1\\ \ \ \ |\\ \ \ \ Y\end{array}$$

dans lequel 1 a la valeur 2, 3 ou 4 représente un atome d'hydrogène ou un groupe alkyle linéaire de 1 à 3 atomes de carbone,

ou bien dans laquelle R représente le groupe R''—A'—B'— où R'' représente un groupe phényle ou naphtyle substitué ou non par un atome d'halogène, un groupe alkyle ou alcoxy de 1 à 3 atomes de carbone chacun, A' représente une simple liaison ou bien un groupement $—CR^{1'}R^{2'}$ ou bien un atome d'azote substitué par un groupe phényle, ou benzyle ou alkyle linéaire de 1 à 3 atomes de carbone substitué lui-même le cas échéant par des atomes d'halogène, dans lequel $R^{1'}$ signifie un atome d'hydrogène ou un groupe méthyle, $R^{2'}$ signifie un groupe phényle ou pyridyle substitué le cas échéant par un atome d'azote ou un groupe alkyle de 1 à 3 atomes de carbone,

B′ représente les groupes

$$—CH(Y)—S—(CH_2)_{m'}—, —CH_2—S—CH_2—CH(Y)—CH_2—, —CH_2—S—CH(Y)—CH_2—,$$

$$—CH_2—S—CH_2—CH(Y)—, —(CH_2)_{n''}—, —CH_2—CH(Y)—, —(CH_2)_{n''}—CH(Y)—, —O—(CH_2)_2—,$$

$$—CH_2—O—(CH_2)_{o'}—, —CH_2—O—CH_2—CH(Y)—CH_2—, —O—CH_2—CH(Y)—, —O—CH(Y)—CH_2—,$$

$$—S—(CH_2)_q—, —S—CH_2—CH(Y)—, —S—CH(Y)—CH_2— \text{ ou } —S—CH_2—CH(Y)—CH_2$$

où Y signifie un atome d'hydrogène ou un groupe alkyle linéaire de 1 à 3 atomes de carbone, m′ et o′ ont la valeur 2 ou 3, n′′ et q les valeurs 2, 3, 4 ou 5, ou bien dans laquelle R représente le groupe R′′′—A′′—B′′ où

R′′′ signifie un groupe pyridyle, thiophényle, furanyle, quinoléine ou benzimidazolyle substitué ou non par des atomes d'halogène, des groupes alkyles de 1 à 3 atomes de carbone ou le groupe $R^1R^2N—CH_2—$, A′′ représente une simple liaison ou bien un groupement $—CR^{1'}R^{2'}$ ou bien un atome d'azote substitué par un groupe phényle ou benzyle substitué le cas échéant par des atomes d'halogène, des groupes alkyle ou alcoxy de 1 à 3 atomes de carbone, où $R^{1'}$ signifie un atome d'hydrogène ou un groupe méthyle, $R^{2'}$ un groupe phényle ou thiophényle substitué le cas échéant par des atomes d'halogène, avec la disposition que A′′ n'est pas une simple liaison si R′′′ est un groupe pyridyle,

B′′ représente les groupements

$$—CH(Y)—S—(CH_2)_{m'}, —CH_2—S—CH_2—CH(Y)—CH_2—, —CH_2—S—CH(Y)—CH_2—, —(CH_2)_{n''}—CH(Y)—,$$

$$—CH_2—S—CH_2—CH(Y)—, —(CH_2)_{n''}—, —O(CH_2)_{n''}—, —S—CH_2—CH(Y)—, —S—CH(Y)—CH_2—,$$

$$—S—(CH_2)_q— \text{ ou } —S—CH_2—CH(Y)—CH_2—$$

où Y signifie un atome d'hydrogène ou un groupe alkyle de 1 à 3 atomes de carbone, m′ a la valeur 2 ou 3 et n′′ et q les valeurs 2, 3, 4 ou 5, X signifie un atome d'hydrogène ou un groupe benzoyle, p a la valeur 2 ou 3, et R′ signifie un atome d'hydrogène ou un groupe méthyle, ainsi les sels physiologiquement acceptables.

2. Dérivés d'imidazolylalkylguanidine selon la revendication 1, caractérisés en ce que R représente le groupe

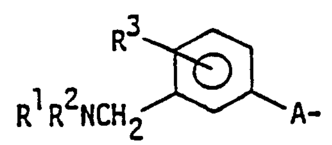

$R^1$ et $R^2$ constituent un cycle pyrrolidine ou pipéridine avec l'atome d'azote, $R^3$ représente un atome d'hydrogène, A le groupe $—O—(CH_2)_k—$, $—O—CH_2—CH(OH)—CH_2—$ ou $—O—CH_2—CH(CH_3)CH_2—$, X et R′ représentent selon le cas un atome d'hydrogène, et k et p selon le cas la valeur 3.

3. Dérivés d'imidazolylalkylguanidine selon la revendication 1, caractérisés en ce que R représente le groupe

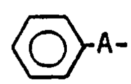 ou 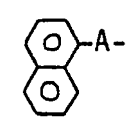

dans lequel A a la signification indiquée dans la revendication 1, X et R′ représentent selon le cas un atome d'hydrogène et p a la valeur 3.

4. Dérivés d'imidazolylalkylguanidine selon la revendication 1, caractérisés en ce que R représente le groupe

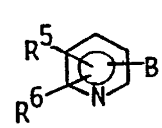

dans lequel $R^5$ représente un atome d'halogène ou d'hydrogène relié en position 5 du cycle pyridine, $R^6$ représente un groupe méthyle ou méthoxy ou bien un atome d'hydrogène lié en position 3 du cycle pyridine, B peut être lié en position 2, 3 ou 4 du cycle pyridine et signifie le groupe

$$—N(CH_3)—(CH_2)_3—,$$

X et R′ représentent le cas échéant un atome d'hydrogène et p a la valeur 3.

5. Dérivés d'imidazolylalkylguanidine selon la revendication 1, caractérisés en ce que R signifie le groupe R″—A′—B′, où R″ représente un groupe phényle non substitué, A′ signifie une double liaison, B′ représente le groupe $—CH_2—S—(CH_2)_{m'}$ ou $—CH_2—S—CH_2—CH(Y)—CH_2—$, où m′ et Y ont les significations indiquées ci-dessus, X et R′ représentent le cas échéant un atome d'hydrogène et p a la valeur 3.

6. Dérivés d'imidazolylalkylguanidine selon la revendication 1, caractérisés en ce que R signifie le groupe R″—A′—B′ où R″ représente un atome d'halogène, un groupe phényle substitué ou non par un groupe alkyle ou alcoxy de 1 à 3 atomes de carbone, A′ signifie un atome d'azote substitué par un groupe phényle ou benzyle, B′ représente le groupe $—(CH_2)_{n''}—$ où n″ a la valeur 2 ou 3, X et R′ représentent le cas échéant un atome d'hydrogène et p a la valeur 3.

7. Dérivés d'imidazolylalkylguanidine selon la revendication 1, caractérisés en ce que R signifie le groupe R″—A′—B′ où R″ représente un groupe phényle substitué ou non par un atome d'hydrogène, un alkyle ou alcoxy de 1 à 3 atomes de carbone, A′ représente le groupe $—CR^{1'}R^{2'}$ où $R^{1'}$ représente un atome d'hydrogène ou un groupe méthyle et $R^{2'}$ un groupe phényle ou pyridyle substitué le cas échéant par un atome d'azote ou un groupe alkyle de 1 à 3 atomes de carbone, B′ signifie le groupe $—(CH_2)_{n''}—$, n″ ayant la valeur 2, 3 ou 4, X et R′ représentent le cas échéant un atome d'hydrogène et p a la valeur 3.

8. Dérivés d'imidazolylalkylguanidine selon la revendication 1, caractérisés en ce que R signifie le groupe R‴—A″—B″—, où R‴ représente un atome d'halogène, des groupes alkyles de 1 à 3 atomes de carbone ou un cycle thiophène substitué ou non par le groupe $R^1R^2N—CH_2—$, A″ signifie une simple liaison, B″ représente le groupe $—CH_2—S—(CH_2)_{m'}—$ ou $—CH_2—S—CH_2—CH(Y)—CH_2—$, où m′ et Y possèdent la signification indiquée ci-dessus, X et R′ représentent selon le cas un atome d'hydrogène et p a la valeur 3.

9. Dérivés d'imidazolylalkylguanidine selon la revendication 1, caractérisés en ce que R représente le groupe R‴—A″—B″—, où R‴ représente un atome d'halogène ou bien un cycle pyridine substitué ou non par un groupe alkyle de 1 à 3 atomes de carbone, A″ signifie le groupe $CR^{1'}R^{2'}$ où $R^{1'}$ et $R^{2'}$ sont définis comme dans la revendication 1, B″ représente le groupe $—(CH_2)_{n''}—$ où n″ a la signification donnée dans la revendication 1, X et R′ représentent selon le cas l'hydrogène et p a la valeur 3.

10. Dérivés d'imidazolylalkylguanidine selon la revendication 1, caractérisés en ce que R représente le groupe R‴—A″—B″— où R‴ représente un atome d'halogène ou un cycle pyridine substitué ou non par un groupe alkyle de 1 à 3 atomes de carbone, A″ désigne un atome d'azote substitué le cas échéant par des groupes phényle ou benzyle substitués par des atomes d'halogène, des groupes alkyle ou alcoxy de 1 à 3 atomes de carbone, B″ représente un groupe $—(CH_2)_{n''}—$, où n″ a la signification indiquée dans la revendication 1, X et R′ représentent selon le cas l'atome d'hydrogène et p a la valeur 3.

11. N-[3-[(N-5-méthyl-pyridin-2-yl)-méthylamino]propyl]-N′-[3-(1H-imidazol-4-yl)propyl]guanidine et leurs sels physiologiquement acceptables.

12. N-[3-(imidazol-4-yl)propyl]-N′-(3,3-diphénylpropyl)guanidine et les sels physiologiquement acceptables.

13. N-[3-(imidazol-4-yl)propyl]-N′-[2-[(1-phényléthyl)thio]éthyl]guanidine et les sels physiologiquement acceptables.

14. N-[3-(imidazol-4-yl)propyl]-N′-[2-[(pyrid-3-yl)méthylthio]éthyl]guanidine et les sels physiologiquement acceptables.

15. N-[3-(imidazol-4-yl)propyl]-N′-[2-(2-thénylthio)éthyl]guanidine et les sels physiologiquement acceptables.

16. N-[3-(imidazol-4-yl)propyl]-N′-[1-méthyl-2-[(pyridine-2-yl)méthylthio]éthyl]guanidine et les sels physiologiquement acceptables.

17. N-[3-(imidazol-4-yl)propyl]-N′-[3-phényl-3-(pyridine-2-yl)propyl]guanidine et les sels physiologiquement acceptables.

18. N-[3-(4-chlorophényl)-3-(pyridine-2-yl)propyl]-N′-[3-(imidazol-4-yl)propyl]guanidine et les sels physiologiquement acceptables.

19. N-[3-(4-chlorophényl)-3-(pyridine-2-yl)propyl]-N′-[3-(imidazol-4-yl)propyl]guanidine et les sels physiologiquement acceptables.

20. N-[3-(4-fluorophényl)-3-(pyridine-2-yl)propyl]-N′-[3-(imidazol-4-yl)propyl]guanidine et les sels physiologiquement acceptables.

21. Procédé pour préparer des dérivés d'imidazolylalkylguanidine selon les revendications 1 à 20 ainsi que les sels physiologiquement acceptables, caratérisé en ce que:

(a) pour préparer les composés de formule I, dans lesquels R, A, B, p et R′ sont définis comme dans la revendication 1 et X représente un groupe benzoyle, on fait réagir:

($a_1$) un composé de formule générale II

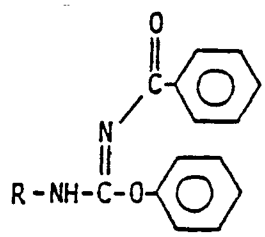

(II)

dans laquelle R a la signification indiquée ci-dessus, avec un composé de formule générale III

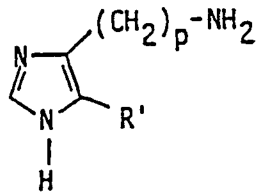

(III)

dans laquelle R′ et p ont la signification indiquée dans la revendication 1, en un composé de formule générale I,

($a_2$) un composé de formule générale IV

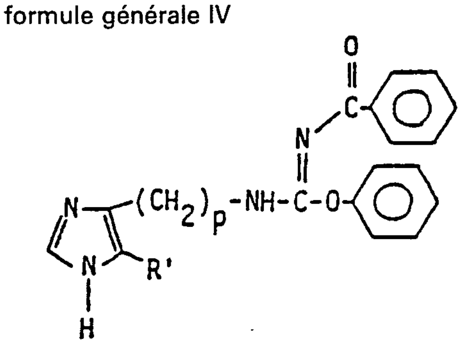

(IV)

dans laquelle R′ et p ont la signification indiquée dans la revendication 1 avec un composé de formule V:

$$R{-}NH_2 \qquad (V)$$

dans laquelle R a la signification indiquée dans la revendication 1 en un composé de formule générale I,

(b) pour préparer les composés de formule générale I dans lesquels R, A, B, p et R′ sont définis comme dans la revendication 1 et X représente un atome d'hydrogène,

($b_1$) on hydrolyse un composé de formule Ia

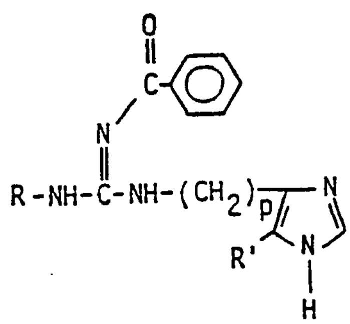

(Ia)

dans laquelle R, p et R′ ont la signification indiquée dans la revendication 1, ou bien:

($b_2$) on hydrolyse un composé de formule générale VI

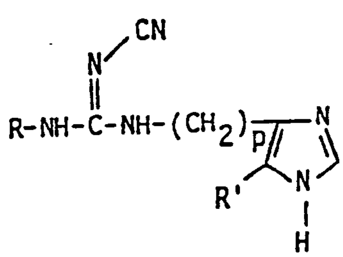

(VI)

dans laquelle R, p et R' ont la signification indiquée dans la revendication 1 à l'aide d'un acide en un composé de formule générale I, ou bien:

($b_3$) on fait réagir un composé de formule générale VII

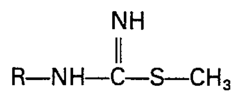

VII

dans laquelle R a la signification indiquée dans la revendication 1, avec un composé de formule générale III cité ci-dessus, en un composé de formule générale I,

($b_4$) on fait réagir un composé de formule générale VIII

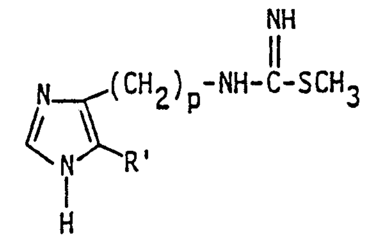

(VIII)

dans laquelle R' et p ont la signification indiquée dans la revendication 1, avec un composé de la formule générale V ci-dessus dans laquelle R a la signification donnée dans la revendication 1, en un composé de formule générale I, et en ce qu'on transforme le cas échéant les composés obtenus en (a) et (b) en leurs sels physiologiquement acceptables.

22. Médicament, caractérisé en ce qu'il renferme un compose selon l'une des revendications 1 à 20 et au moins un adjuvant acceptable pharmaceutiquement ou un diluant inerte acceptable pharmaceutiquement.

**Revendications pour l'Etat contractant: AT**

1. Procédé de préparation d'imidazolylalkylguanidine de formule générale I

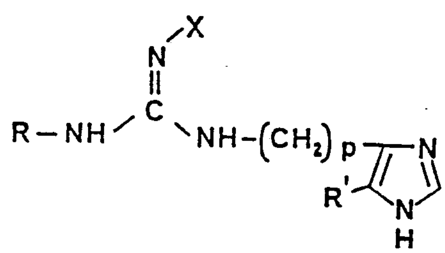

(I)

dans laquelle R signifie le groupe

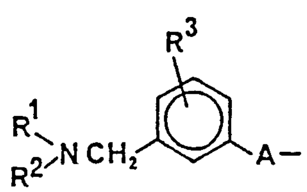

où $R^1$ et $R^2$, identiques ou différents, représentent l'hydrogène, un groupe alkyle linéaire de 1 à 3 atomes de carbone, ou bien un groupe cycloalkyle de 5 ou 6 atomes de carbone, ou bien $R^1$ et $R^2$ constituent avec l'atome d'azote lequel ils sont liés un cycle pyrrolidine, pipéridine ou homopipéridine, $R^3$ désigne l'hydrogène, un atome d'halogène ou un groupe alkyle de 1 à 3 atomes de carbone, A signifie les groupes

$$-O-(CH_2)_k-,\ -O-CH_2CH(OH)CH_2-,\ -O-CH_2CH(CH_3)-CH_2-,$$

$$-CH_2-O-CH_2-CH(OH)-CH_2 \text{ ou } -O-CH_2-CH(OH)-CH(OH)-CH_2$$

où k a la valeur 3 ou 4, ou bien dans laquelle R représente le groupe

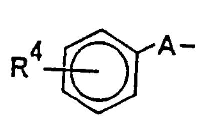

ou

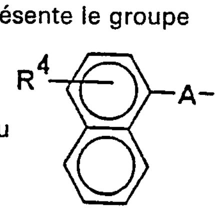

dans lequel $R^4$ signifie un atome d'hydrogène, un atome d'halogène lié avantageusement en position para par rapport à A, un groupe alcoxy de 1 à 3 atomes de carbone ou alkyle de 1 à 3 atomes de carbone et A a la signification indiquée ci-dessus, ou bien dans laquelle R représente le groupe

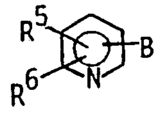

dans lequel $R^5$ et $R^6$, identiques ou différents, représentent un atome d'hydrogène, un atome d'halogène ou un groupe alkyle linéaire de 1 à 3 atomes de carbone ou un groupe alcoxy linéaire de 1 à 3 atomes de carbone, B étant lié au cycle pyridine en position 2, 3 ou 4, et le groupe

$$-N(Y)-(CH_2)_1$$

dans lequel 1 a la valeur 2, 3 ou 4 représente un atome d'hydrogène ou un groupe alkyle linéaire de 1 à 3 atomes de carbone,

ou bien dans laquelle R représente le groupe R''—A'—B'— où R'' représente un groupe phényle ou naphtyle substitué ou non par un atome d'halogène, un groupe alkyle ou alcoxy de 1 à 3 atomes de carbone chacun, A' représente une simple liaison ou bien un groupement $-CR^{1'}R^{2'}$ ou bien un atome d'azote substitué par un groupe phényle, ou benzyle ou alkyle linéaire de 1 à 3 atomes de carbone substitué lui-même le cas échéant par des atomes d'halogène, dans lequel $R^{1'}$ signifie un atome d'hydrogène ou un groupe méthyle, $R^{2'}$ signifie un groupe phényle ou pyridyle substitué le cas échéant par un atome d'azote ou un groupe alkyle de 1 à 3 atomes de carbone,

B' représente les groupes

$$-CH(Y)-S-(CH_2)_{m'}-,\ -CH_2-S-CH_2-CH(Y)-CH_2-,\ -CH_2-S-CH(Y)-CH_2-,$$

$$-CH_2-S-CH_2-CH(Y)-,\ -(CH_2)_{n''}-,\ -CH_2-CH(Y)-,\ -(CH_2)_{n''}-CH(Y)-,\ -O-(CH_2)_2-,$$

$$-CH_2-O-(CH_2)_{o'}-,\ -CH_2-O-CH_2-CH(Y)-CH_2-,\ -O-CH_2-CH(Y)-,\ -O-CH(Y)-CH_2-,$$

$$-S-(CH_2)_q-,\ -S-CH_2-CH(Y)-,\ -S-CH(Y)-CH_2-\ \text{ou}\ -S-CH_2-CH(Y)-CH_2$$

où Y signifie un atome d'hydrogène ou un groupe alkyle linéaire de 1 à 3 atomes de carbone, m' et o' ont la valeur 2 ou 3, n'' et q les valeurs 2, 3, 4 ou 5, ou bien dans laquelle R représente le groupe R'''—A''—B'' où

R''' signifie un groupe pyridyle, thiophényle, furanyle, quinoléine ou benzimidazolyle substitué ou non par des atomes d'halogène, des groupes alkyles de 1 à 3 atomes de carbone ou le groupe $R^1R^2N-CH_2-$, A'' représente une simple liaison ou bien un groupement $-CR^{1'}R^{2'}$ ou bien un atome d'azote substitué par un groupe phényle ou benzyle substitué le cas échéant par des atomes d'halogène, des groupes alkyle ou alcoxy de 1 à 3 atomes de carbone, où $R^{1'}$ signifie un atome d'hydrogène ou un groupe méthyle, $R^{2'}$ un groupe phényle ou thiophényle substitué le cas échéant par des atomes d'halogène, avec la disposition que A'' n'est pas une simple liaison si R''' est un groupe pyridyle,

B'' représente les groupements

$$-CH(Y)-S-(CH_2)_{m'},\ -CH_2-S-CH_2-CH(Y)-CH_2-,\ -CH_2-S-CH(Y)-CH_2-,\ -(CH_2)_{n''}-CH(Y)-,$$

$$-CH_2-S-CH_2-CH(Y)-,\ -(CH_2)_{n''}-,\ -O(CH_2)_{n''}-,\ -S-CH_2-CH(Y)-,\ -S-CH(Y)-CH_2-,$$

$$-S-(CH_2)_q-\ \text{ou}\ -S-CH_2-CH(Y)-CH_2-$$

où Y signifie un atome d'hydrogène ou un groupe alkyle de 1 à 3 atomes de carbone, m' a la valeur 2 ou 3 et n'' et q les valeurs 2, 3, 4 ou 5, X signifie un atome d'hydrogène ou un groupe benzoyle, p a la valeur 2 ou 3, et R' signifie un atome d'hydrogène ou un groupe méthyle, ainsi les sels physiologiquement acceptables, caractérisés en ce que:

(a) pour préparer les composés de formule I, dans R, A, B, p et R' sont définis comme dans la revendication 1 et X représente un groupe benzoyle, on fait réagir:

($a_1$) un composé de formule générale II

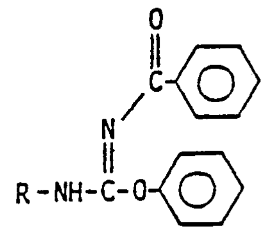

(II)

dans laquelle R a la signification indiquée ci-dessus, avec un composé de formule générale III

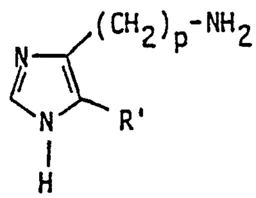

(III)

dans laquelle R′ et p ont la signification indiquée dans la revendication 1, en un composé de formule générale I,

($a_2$) un composé de formule générale IV

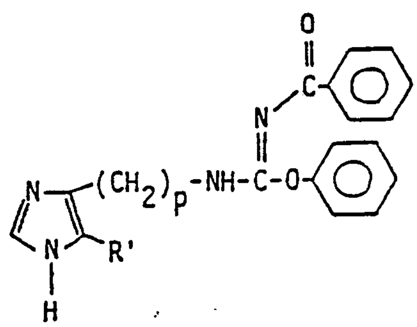

(IV)

dans laquelle R′ et p ont la signification indiquée dans la revendication 1 avec un composé de formule V:

$$R—NH_2 \qquad (V)$$

dans laquelle R a la signification indiquée dans la revendication 1 en un composé de formule générale I,

(b) pour préparer les composés de formule générale I dans lesquels R, A, B, p et R′ sont définis comme dans la revendication 1 et X représente un atome d'hydrogène,

($b_1$) on hydrolyse un composé de formule Ia

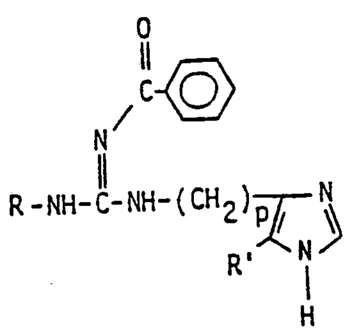

(Ia)

dans laquelle R, p et R′ ont la signification indiquée dans la revendication 1, ou bien:

($b_2$) on hydrolyse un composé de formule générale VI

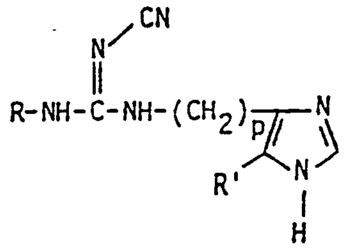

(VI)

dans laquelle R, p et R′ ont la signification indiquée dans la revendication 1 à l'aide d'un acide en un composé de formule générale I, ou bien:

($b_3$) on fait réagir un composé de formule générale VII

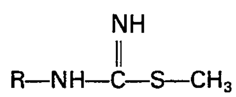

VII

dans laquelle R a la signification indiquée dans la revendication 1, avec un composé de formule générale III cité ci-dessus, en un composé de formule générale I,

($b_4$) on fait réagir un composé de formule générale VIII

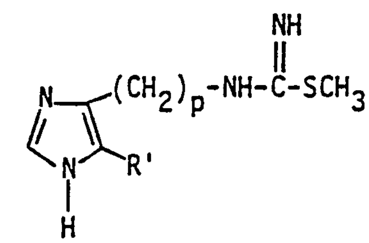

(VIII)

dans laquelle R′ et p ont la signification indiquée dans la revendication 1, avec un composé de la formule générale V ci-dessus dans laquelle R a la signification donnée dans la revendication 1, en un composé de formule générale I, et en ce qu'on transforme le cas échéant les composés obtenus en (a) et (b) en leurs sels physiologiquement acceptables.

2. Procédé selon la revendication 1 pour la préparation de composés dans lesquels R représente le groupe:

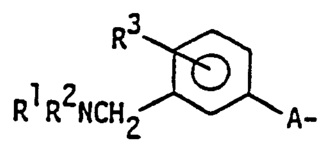

$R^1$ et $R^2$ constituent un cycle pyrrolidine ou pipéridine avec l'atome d'azote, $R^3$ représente un atome d'hydrogène, A le groupe $—O—(CH_2)_k—$, $—O—CH_2—CH(OH)—CH_2—$ ou $—O—CH_2—CH(CH_3)CH_2—$, X et R′ représentent selon le cas un atome d'hydrogène, et k et p selon le cas la valeur 3.

3. Procédé selon la revendication 1 pour la préparation de composés dans lesquels R représente le groupe

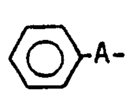

ou

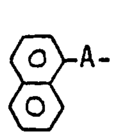

dans lequel A a la signification indiquée dans la revendication 1, X et R′ représentent selon le cas un atome d'hydrogène et p a la valeur 3.

4. Procédé selon la revendication 1 pour la préparation de composés dans lesquels R représente le groupe

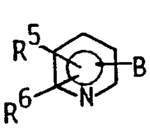

dans lequel $R^5$ représente un atome d'halogène ou d'hydrogène relié en position 5 du cycle pyridine, $R^6$ représente un groupe méthyle ou méthoxy ou bien un atome d'hydrogène lié en position 3 du cycle

pyridine, B peut être lié en position 2, 3 ou 4 du cycle pyridine et signifie le groupe

$$-N-(CH_2)_3-, \quad N\text{-}CH_3$$

X et R′ représentent le cas échéant un atome d'hydrogène et p a la valeur 3.

5. Procédé selon la revendication 1 de préparation de composés dans lesquels R signifie le groupe R″—A′—B′, où R″ représente un groupe phényle non substitué, A′ signifie une double liaison, B′ représente le groupe $-CH_2-S-(CH_2)_{m'}$ ou $-CH_2-S-CH_2-CH(Y)-CH_2-$, où m′ et Y ont les significations indiquées ci-dessus, X et R′ représentent le cas échéant un atome d'hydrogène et p a la valeur 3.

6. Procédé selon la revendication 1 de préparation de composés, dans lesquels R signifie le groupe R″—A′—B′ où R″ représente un atome d'halogène, un groupe phényle substitué ou non par un groupe alkyle ou alcoxy de 1 à 3 atomes de carbone, A′ signifie un atome d'azote substitué par un groupe phényle ou benzyle, B′ représente le group $-(CH_2)_{n''}-$ où n″ a la valeur 2 ou 3, X et R′ représentent le cas échéant un atome d'hydrogène et p a la valeur 3.

7. Procédé selon la revendication 1 de préparation de composés dans lesquels R signifie le groupe R″—A′—B′ où R″ représente un groupe phényle substitué ou non par un atome d'hydrogène, un alkyle ou alcoxy de 1 à 3 atomes de carbone, A′ représente le groupe $-CR^{1'}R^{2'}$ où $R^{1'}$ représente un atome d'hydrogène ou un groupe méthyle et $R^{2'}$ un groupe phényle ou pyridyle substitué le cas échéant par un atome d'azote ou un groupe alkyle de 1 à 3 atomes de carbone, B′ signifie le groupe $-(CH_2)_{n''}-$, n″ ayant la valeur 2, 3 ou 4, X et R′ représentent le cas échéant un atome d'hydrogène et p a la valeur 3.

8. Procédé selon la revendication 1 de préparation de composés dans lesquels R signifie le groupe R‴—A″—B″—, où R‴ représente un atome d'halogène, des groupes alkyles de 1 à 3 atomes de carbone ou un cycle thiophène substitué ou non par le groupe $R^1R^2N-CH_2-$, A″ signifie une simple liaison, B″ représente le groupe $-CH_2-S-(CH_2)_{m'}-$ ou $-CH_2-S-CH_2-CH(Y)-CH_2-$, où m′ et Y possèdent la signification indiquée ci-dessus, X et R′ représentent selon le cas un atome d'hydrogène et p a la valeur 3.

9. Procédé selon la revendication 1, pour la préparation de composés dans lesquels R signifie le groupe R‴—A″—B″—, où R‴ représente un atome d'halogène ou bien un cycle pyridine substitué ou non par un groupe alkyle de 1 à 3 atomes de carbone, A″ signifie le groupe $CR^{1'}R^{2'}$ où $R^{1'}$ et $R^{2'}$ sont définis comme dans la revendication 1, B″ représente le groupe $-(CH_2)_{n''}-$ où n″ a la signification donnée dans la revendication 1, X et R′ représentent selon le cas l'hydrogène et p a la valeur 3.

10. Procédé selon la revendication 1, pour la préparation de composés dans lesquels R représente le groupe R‴—A″—B″— où R‴ représente un atome d'halogène ou un cycle pyridine substitué ou non par un groupe alkyle de 1 à 3 atomes de carbone, A″ désigne un atome d'azote substitué le cas échéant par des groupes phényle ou benzyle substitués par des atomes d'halogène, des groupes alkyle ou alcoxy de 1 à 3 atomes de carbone, B″ représente un groupe $-(CH_2)_{n''}-$, où n″ a la signification indiquée dans la revendication 1, X et R′ représentent selon le cas l'atome d'hydrogène et p a la valeur 3.

11. Procédé selon la revendication 1 de préparation du N-[3-[(N-5-méthyl-pyridin-2-yl)méthylamino]-propyl]-N′-[3-(1H-imidazol-4-yl)propyl]guanidine et leurs sels physiologiquement acceptables.

12. Procédé selon la revendication 1 de préparation du N-[3-(imidazol-4-yl)propyl]-N′-(3,3-diphénylpropyl)guanidine et les sels physiologiquement acceptables.

13. Procédé selon la revendication 1 de préparation du N-[3-(imidazol-4-yl)propyl]-N′-[2-[(1-phényléthyl)thio]éthyl]guanidine et les sels physiologiquement acceptables.

14. Procédé selon la revendication 1 de préparation du N-[3-(imidazol-4-yl)propyl]-N′-[2-[(pyrid-3-yl)-méthylthio]éthyl]guanidine et les sels physiologiquement acceptables.

15. Procédé selon la revendication 1 de préparation du N-[3-(imidazol-4-yl)propyl]-N′-[2-(2-thénylthio)-éthyl]guanidine et les sels physiologiquement acceptables.

16. Procédé selon la revendication 1 de préparation du N-[3-(imidazol-4-yl)propyl]-N′-[1-méthyl-2-[(pyridine-2-yl)méthylthio]éthyl]guanidine et les sels physiologiquement acceptables.

17. Procédé selon la revendication 1 de préparation du N-[3-(imidazol-4-yl)propyl]-N′-[3-phényl-3-(pyridine-2-yl)propyl]guanidine et les sels physiologiquement acceptables.

18. Procédé selon la revendication 1 de préparation du N-[3-(4-chlorophényl)-3-(pyridine-2-yl)propyl]-N′-[3-(imidazol-4-yl)propyl]guanidine et les sels physiologiquement acceptables.

19. Procédé selon la revendication 1 de préparation du N-[3-(4-chlorophényl)-3-(pyridine-2-yl)propyl]-N′-[3-(imidazol-4-yl)propyl]guanidine et les sels physiologiquement acceptables.

20. Procédé selon la revendication 1 de préparation du N-[3-(4-fluorophényl)-3-(pyridine-2-yl)propyl]-N′-[3-(imidazol-4-yl)propyl]guanidine et les sels physiologiquement acceptables.

**Claims for the Contracting States: DE FR GB BE NL LU IT SE CH LI**

1. Imidazolyl alkyl guanidine derivatives corresponding to the general formula I

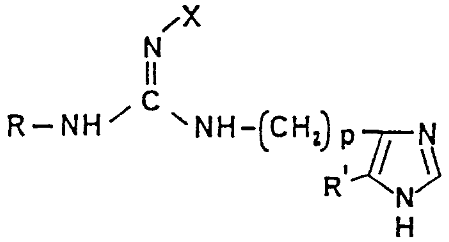

(I)

in which R denotes the group:

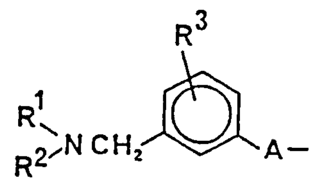

wherein $R^1$ and $R^2$, which may be identical or different, denote hydrogen, straight chain $C_1$—$C_3$ alkyl or $C_5$—$C_6$ cycloalkyl or $R^1$ and $R^2$ together with the nitrogen atom to which they are attached form a pyrrolidine, piperidine or homopiperidine ring, $R^3$ stands for a hydrogen atom, a halogen atom or a $C_1$—$C_3$ alkoxy group, A stands for the group

$$—O—(CH_2)_k—, —O—CH_2CH(OH)CH_2—, —O—CH_2CH(CH_3)—CH_2—,$$

$$—CH_2—O—CH_2—CH(OH)—CH_2 \text{ or } —O—CH_2—CH(OH)—CH(OH)—CH_2$$

wherein k has the value 3 or 4 or in which R denotes the group

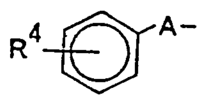
or
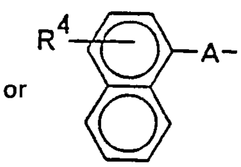

wherein $R^4$ stands for a hydrogen atom, a halogen atom preferably attached in the para position to A, a $C_1$—$C_3$ alkoxy group or a $C_1$—$C_3$ alkyl group and A has the meaning indicated above, or in which R denotes the group:

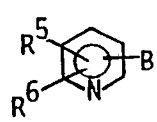

wherein $R^5$ and $R^6$, which may be identical or different, stand for a hydrogen atom, a halogen atom or a straight chain $C_1$—$C_3$ alkyl group or a straight chain $C_1$—$C_3$ alkoxy group, B may be attached in position 2, 3 or 4 of the pyridine ring and denotes the group

$$—N—(CH_2)_1$$
$$|$$
$$Y$$

wherein 1 has the value 2, 3 or 4 and Y stands for a hydrogen atom or for a straight chain $C_1$—$C_3$ alkyl group, or in which R denotes the group R″—A′—B′ wherein R″ stands for a phenyl or naphthyl group which may be unsubstituted or substituted with a halogen atom, with a $C_1$—$C_3$ alkyl group or with a $C_1$—$C_3$ alkoxy group. A′ stands for a single bond or for the group $—CR^{1'}R^{2'}$ or for a nitrogen atom substituted with an optionally halogen-substituted phenyl or benzyl group or with a straight chain $C_1$—$C_3$ alkyl group, $R^{1'}$ denoting a hydrogen atom or a methyl group. $R^{2'}$ stands for a phenyl or pyridyl group optionally substituted with a halogen atom or with a $C_1$—$C_3$ alkyl group.

B′ stands for the following groups:

$$—CH(Y)—S—(CH_2)_{m'}—, —CH_2—S—CH_2—CH(Y)—CH_2—, —CH_2—S—CH(Y)—CH_2—,$$

$$—CH_2—S—CH_2—CH(Y)—, —(CH_2)_{n'}—, —CH_2—CH(Y)—, —(CH_2)_{n'}—CH(Y)—, —O—(CH_2)_2—,$$

$-CH_2-O-(CH_2)_{o'}-$, $-CH_2-O-CH_2-CH(Y)-CH_2-$, $-O-CH_2-CH(Y)-$, $-O-CH(Y)-CH_2-$,

$-S-(CH_2)_q-$, $-S-CH_2-CH(Y)-$, $-S-CH(Y)-CH_2-$ or $-S-CH_2-CH(Y)-CH_2$

wherein Y denotes a hydrogen atom or a straight chain $C_1-C_3$ alkyl group, m' and o' have the value 2 or 3 and n'' and q have the value 2, 3, 4 or 5, or in which R denotes the group R'''—A''—B''— wherein R''' stands for a pyridyl, thiophenyl, furanyl, quinolyl or benzimidazolyl group, which groups may be unsubstituted or substituted with halogen atoms, with $C_1-C_3$ alkyl groups or with the group $R^1R^2N-CH_2-$, A'' stands for a single bond or for the group $-CR^{1'}R^{2'}$ or for a nitrogen atom substituted with a phenyl or benzyl group which is optionally substituted with halogen atoms, with $C_1-C_3$ alkyl groups or with $C_1-C_3$ alkoxy groups, $R^{1'}$ denoting a hydrogen atom or a methyl group and $R^{2'}$ denoting a phenyl or thiophenyl group optionally substituted with halogen atoms, under the condition that A'' does not stand for a single bond when R''' is a pyridyl group.

B'' stands for the group

$-CH(Y)-S-(CH_2)_{m'}-$, $-CH_2-S-CH_2-CH(Y)-CH_2-$, $-CH_2-S-CH(Y)-CH_2-$, $-(CH_2)_{n''}-CH(Y)-$,

$-CH_2-S-CH_2-CH(Y)-$, $-(CH_2)_{n''}-$, $-O(CH_2)_{n''}-$, $-S-CH_2-CH(Y)-$, $-S-CH(Y)-CH_2-$,

$-S-(CH_2)_q-$ or $-S-CH_2-CH(Y)-CH_2-$

wherein Y denotes a hydrogen atom or a straight chain $C_1-C_3$ alkyl group, m' has the value 2 or 3 and n'' and q have the value 2, 3, 4 or 5, X denotes a hydrogen atom or a benzoyl group, p has the value 2 or 3 and R' denotes a hydrogen atom or a methyl group, and the physiologically acceptable salts thereof.

2. Imidazolyl alkyl guanidine derivatives according to Claim 1, characterised in that R stands for the group:

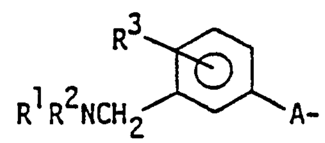

wherein $R^1$ and $R^2$ together with the nitrogen atom form a pyrrolidine or piperidine ring and $R^3$ stands for a hydrogen atom, A denotes the group $-O-(CH_2)_k-$, $-O-CH_2CH(OH)CH_2-$ or $-O-CH_2CH(CH_3)CH_2-$, X and R' stand each for a hydrogen atom and k and p have each the value 3.

3. Imidazolyl alkyl guanidine derivatives according to Claim 1, characterised in that R stands for the group

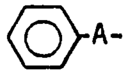 or 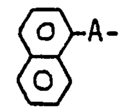

wherein A has the meaning indicated in Claim 1, X and R' stand each for a hydrogen atom and p has the value 3.

4. Imidazolyl alkyl guanidine derivatives according to Claim 1, characterised in that R stands for the group

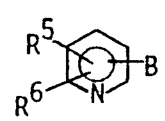

wherein $R^5$ stands for a halogen atom or hydrogen atom attached in the 5 position of the pyridine ring, $R^6$ stands for a methyl or methoxy group or a hydrogen atom attached in the 3-position of the pyridine ring, B may be attached in position 2, 3 or 4 of the pyridine ring and denotes the group

$$-\underset{\displaystyle CH_3}{\underset{|}{N}}-(CH_2)_3-,$$

X and R' stand each for a hydrogen atom and p has the value 3.

5. Imidazolyl alkyl guanidine derivatives according to Claim 1, characterised in that R denotes the group R''—A'—B'— wherein R'' stands for an unsubstituted phenyl group, A' denotes a single bond, B' stands for the group $-CH_2-S-(CH_2)_{m'}-$ or $-CH_2-S-CH_2-CH(Y)-CH_2-$ wherein m' and Y have the meanings indicated above, X and R' stand each for a hydrogen atom and p has the value 3.

6. Imidazolyl alkyl guanidine derivatives according to Claim 1, characterised in that R denotes the group R''—A'—B'— wherein R'' stands for a phenyl group which may be unsubstituted or substituted with a halogen atom, a $C_1$—$C_3$ alkyl group or a $C_1$—$C_3$ alkoxy group, A' denotes a nitrogen atom substituted with a phenyl or benzyl group, B' stands for the group $—(CH_2)_{n''}—$ wherein n'' has the value 2 or 3, X and R' stand each for a hydrogen atom and p has the value 3.

7. Imidazolyl alkyl guanidine derivatives according to Claim 1, characterised in that R denotes the group R''—A'—B'— wherein R'' stands for a phenyl group which may be unsubstituted or substituted with a halogen atom, a $C_1$—$C_3$ alkyl group or a $C_1$—$C_3$ alkoxy group, A' stands for the group $—CR^{1'}R^{2'}$ wherein $R^{1'}$ denotes a hydrogen atom or a methyl group, $R^{2'}$ stands for a phenyl or pyridyl group optionally substituted with a halogen atom or a $C_1$—$C_3$ alkyl group, B' denotes the group $—(CH_2)_{n''}—$ wherein n'' has the value 2, 3 or 4, X and R' stand each for a hydrogen atom and p has the value 3.

8. Imidazolyl alkyl guanidine derivatives according to Claim 1, characterised in that R denotes the group R'''—A''—B'' wherein R''' stands for a thiophene ring which may be unsubstituted or substituted with halogen atoms, with $C_1$—$C_3$ alkyl groups or with the group $R^1R^2NCH_2$, A'' denotes a single bond, B'' stands for the group $—CH_2—S—(CH_2)_{m'}—$ or $—CH_2—S—CH_2—CH(Y)—CH_2—$, wherein m' and Y have the meanings indicated above, X and R' stand each for a hydrogen atom and p has the value 3.

9. Imidazolyl alkyl guanidine derivatives according to Claim 1, characterised in that R denotes the group R'''—A''—B''— wherein R''' stands for a pyridine ring which may be unsubstituted or substituted with halogen atoms or with $C_1$—$C_3$ alkyl groups, A'' denotes the group $—CR^{1'}R^{2'}$ wherein $R^{1'}$ and $R^{2'}$ have the meanings defined in Claim 1, B'' stands for the group $—(CH_2)_{n''}—$ wherein n'' has the meaning indicated in Claim 1, X and R' stand each for a hydrogen atom and p has the value 3.

10. Imidazolyl alkyl guanidine derivatives according to Claim 1, characterised in that R denotes the group R'''—A''—B''— wherein R''' stands for a pyridine ring which may be unsubstituted or substituted with halogen atoms or with $C_1$—$C_3$ alkyl groups, A'' stands for a nitrogen atom substituted with a phenyl or benzyl group, which phenyl or benzyl group is optionally substituted with halogen atoms, with $C_1$—$C_3$ alkyl groups or with $C_1$—$C_3$ alkoxy groups, B'' stands for the group $—(CH_2)_{n''}—$ wherein n'' has the meaning indicated in Claim 1, X and R' stand each for a hydrogen atom and p has the value 3.

11. N-[3-[(N-5-methyl-pyridin-2-yl)-methylamino]propyl]-N'-[3-(1H-imidazol-4-yl)propyl]guanidine and the physiologically acceptable salts.

12. N-[3-(imidazol-4-yl)propyl]-N'-(3,3-diphenylpropyl)guanidine and the physiologically acceptable salts.

13. N-[3-(imidazol-4-yl)propyl]-N'-[2-[(1-phenylethyl)thio]ethyl]guanidine and the physiologically acceptable salts.

14. N-[3-(imidazol-4-yl)propyl]-N'-[2-[(pyrid-3-yl)methylthio]ethyl]guanidine and the physiologically acceptable salts.

15. N-[3-(imidazol-4-yl)propyl]-N'-[2-(2-phenylthio)éthyl]guanidine and the physiologically acceptable salts.

16. N-[3-(imidazol-4-yl)propyl]-N'-[1-methyl-2-[(pyrid-2-yl)-methylthio]ethyl]guanidine and the physiologically acceptable salts.

17. N-[3-(imidazol-4-yl)propyl]-N'-[3-phenyl-3-(pyrid-2-yl)propyl]guanidine and the physiologically acceptable salts.

18. N-[3-(4-chlorophenyl)-3-(pyrid-2-yl)propyl]-N'-[3-(imidazol-4-yl)propyl]guanidine and the physiologically acceptable salts.

19. N-[3-(4-bromophenyl)-3-(pyrid-2-yl)propyl]-N'-[3-(imidazol-4-yl)propyl]guanidine and the physiologically acceptable salts.

20. N-[3-(4-fluorophenyl)-3-(pyrid-2-yl)propyl]-N'-[3-(imidazol-4-yl)propyl]guanidine and the physiologically acceptable salts.

21. Process for the preparation of imidazolyl alkyl guanidine derivatives according to Claims 1 to 20 and the physiologically acceptable salts thereof, characterised in that

a) for the preparation of compounds corresponding to the general formula I wherein R, A, B, p and R' have the meanings defined in Claim 1 and X stands for a benzoyl group,

($a_1$) a compound corresponding to the general formula II

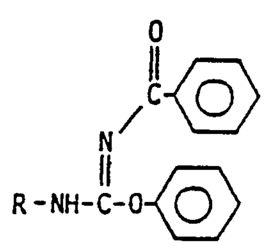

(II)

in which R has the meaning indicated above is reacted with a compound corresponding to the general formula III

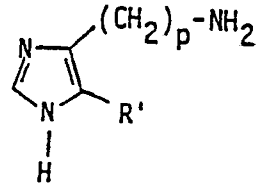

(III)

wherein R′ and p have the meanings indicated in Claim 1 to form a compound corresponding to the general formula I or

(a$_2$) a compound corresponding to the general formula IV

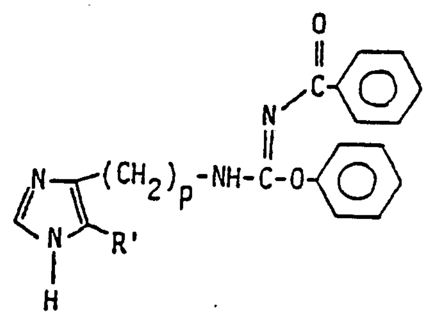

(IV)

in which R′ and p have the meanings indicated in Claim 1 is reacted with a compound corresponding to the general formula V

$$R—NH_2 \qquad (V)$$

wherein R has the meaning indicated in Claim 1 to form a compound corresponding to the general formula I or

b) for the preparation of compounds corresponding to the general formula I wherein R, A, B, p and R′ have the meanings defined in Claim 1 and X stands for a hydrogen atom,

(b$_1$) a compound corresponding to formula Ia

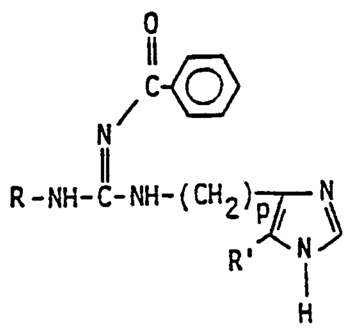

(Ia)

wherein R, p and R′ have the meanings indicated in Claim 1 is hydrolysed or

(b$_2$) a compound corresponding to the general formula VI

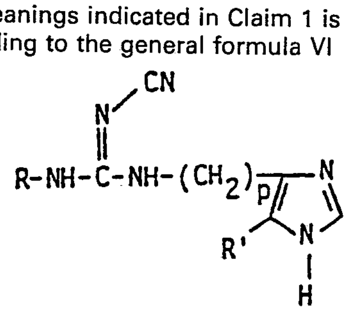

(VI)

wherein R, p and R′ have the meanings indicated in Claim 1 is hydrolysed by means of an acid to form a compound corresponding to the general formula I or

(b$_3$) a compound corresponding to the general formula VII

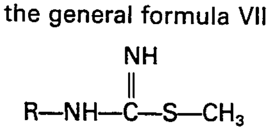

VII

wherein R has the meaning indicated in Claim 1 is reacted with a compound corresponding to the above indicated general formula III wherein R′ and p have the meanings indicated in Claim 1 to form a compound corresponding to the general formula I, or

($b_4$) a compound corresponding to the general formula VIII

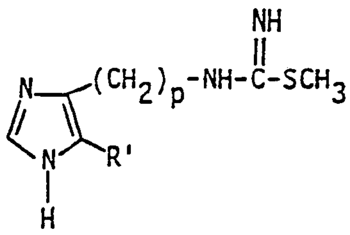

(VIII)

wherein R′ and p have the meanings indicated in Claim 1 is reacted with a compound corresponding to the above general formula V wherein R has the meaning indicated in Claim 1 to form a compound corresponding to the general formula I and in that the compounds obtained under a) and b) are optionally converted into their physiologically acceptable salt.

22. Pharmaceutical composition, characterised in that it contains a compound according to Claims 1 to 20 and at least one inert, pharmaceutically acceptable carrier or an inert, pharmaceutically acceptable diluent.

**Claims for the Contracting State: AT**

1. Process for the preparation of imidazolyl alkyl guanidine derivatives corresponding to the general formula I

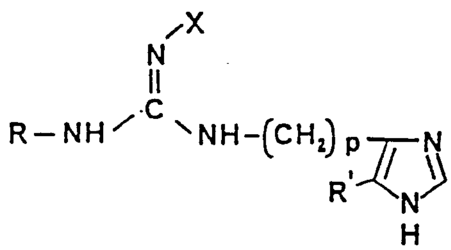

(I)

in which R denotes the group

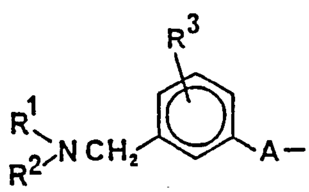

wherein $R^1$ and $R^2$, which may be identical or different, denote hydrogen, straight chain $C_1$—$C_3$ alkyl or $C_5$—$C_6$ cycloalkyl or $R^1$ and $R^2$ together with the nitrogen atom to which they are attached form a pyrrolidine, piperidine or homopiperidine ring, $R^3$ stands for a hydrogen atom, a halogen atom or a $C_1$—$C_3$ alkoxy group, A denotes the group

$$—O—(CH_2)_k—,\ —O—CH_2CH(OH)CH_2—,\ —O—CH_2CH(CH_3)—CH_2—,$$

$$—CH_2—O—CH_2—CH(OH)—CH_2 \text{ or } —O—CH_2—CH(OH)—CH(OH)—CH_2$$

wherein k has the value 3 or 4 or in which R denotes the group

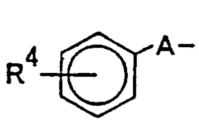

or

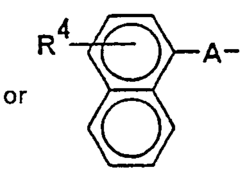

wherein $R^4$ stands for a hydrogen atom, a halogen atom preferably attached in the para position to A, a $C_1$—$C_3$ alkoxy group or a $C_1$—$C_3$ alkyl group and A has the meaning indicated above, or in which R denotes the group:

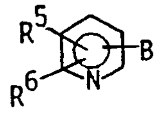

wherein $R^5$ and $R^6$, which may be identical or different, stand for a hydrogen atom, a halogen atom or a straight chain $C_1$—$C_3$ alkyl group or a straight chain $C_1$—$C_3$ alkoxy group, B may be attached in position 2, 3 or 4 of the pyridine ring and denotes the group

—N—$(CH_2)_1$
Y

wherein 1 has the value 2, 3 or 4 and Y stands for a hydrogen atom or for a straight chain $C_1$—$C_3$ alkyl group, or in which R denotes the group R''—A'—B' wherein R'' stands for a phenyl or naphthyl group which may be unsubstituted or substituted with a halogen atom, a $C_1$—$C_3$ alkyl group or a $C_1$—$C_3$ alkoxy group. A' stands for a single bond or for the group —$CR^{1'}R^{2'}$ or for a nitrogen atom substituted with an optionally halogen-substituted phenyl or benzyl group or straight chain $C_1$—$C_3$ alkyl group, $R^{1'}$ denoting a hydrogen atom or a methyl group. $R^{2'}$ stands for a phenyl or pyridyl group optionally substituted with a halogen atom or with a $C_1$—$C_3$ alkyl group.

B' stands for the groups:

—CH(Y)—S—$(CH_2)_{m'}$—, —$CH_2$—S—$CH_2$—CH(Y)—$CH_2$—, —$CH_2$—S—CH(Y)—$CH_2$—,

—$CH_2$—S—$CH_2$—CH(Y)—, —$(CH_2)_{n''}$—, —$CH_2$—CH(Y)—, —$(CH_2)_{n''}$—CH(Y)—, —O—$(CH_2)_2$—,

—$CH_2$—O—$(CH_2)_{o'}$—, —$CH_2$—O—$CH_2$—CH(Y)—$CH_2$—, —O—$CH_2$—CH(Y)—, —O—CH(Y)—$CH_2$—,

—S—$(CH_2)_q$—, —S—$CH_2$—CH(Y)—, —S—CH(Y)—$CH_2$— or —S—$CH_2$—CH(Y)—$CH_2$

wherein Y denotes a hydrogen atom or a straight chain $C_1$—$C_3$ alkyl group, m' and o' have the value 2 or 3 and n'' and q have the value 2, 3, 4 or 5, or in which R denotes the group R'''—A''—B''— wherein R''' stands for a pyridyl, thiophenyl, furanyl, quinolyl or benzimidazolyl group, which may be unsubstituted or substituted with halogen atoms, with $C_1$—$C_3$ alkyl groups or with the group $R^1R^2N$—$CH_2$—, A'' stands for a single bond or for the group —$CR^{1'}R^{2'}$ or for a nitrogen atom substituted with a phenyl or benzyl group, which phenyl or benzyl group is optionally substituted with halogen atoms, with $C_1$—$C_3$ alkyl groups or with $C_1$—$C_3$ alkoxy groups, $R^{1'}$ denotes a hydrogen atom or a methyl group and $R^{2'}$ denotes an optionally halogen-substituted phenyl or thiophenyl group, under the condition that A'' does not stand for a single bond when R''' is a pyridyl group.

B'' stands for the group

—CH(Y)—S—$(CH_2)_{m'}$—, —$CH_2$—S—$CH_2$—CH(Y)—$CH_2$—, —$CH_2$—S—CH(Y)—$CH_2$—, —$(CH_2)_{n''}$—CH(Y)—,

—$CH_2$—S—$CH_2$—CH(Y)—, —$(CH_2)_{n''}$—, —O$(CH_2)_{n''}$—, —S—$CH_2$—CH(Y)—, —S—CH(Y)—$CH_2$—,

—S—$(CH_2)_q$— or —S—$CH_2$—CH(Y)—$CH_2$—

wherein Y denotes a hydrogen atom or a straight chain $C_1$—$C_3$ alkyl group, m' has the value 2 or 3 and n'' and q have the value 2, 3, 4 or 5, X denotes a hydrogen atom or a benzoyl group, p has the value 2 or 3 and R' denotes a hydrogen atom or a methyl group, and the physiologically acceptable salts thereof, characterised in that

a) for the preparation of compounds corresponding to the general formula I in which R, A, B, p and R' are defined as above and X stands for a benzoyl group,

($a_1$) a compound corresponding to the general formula II

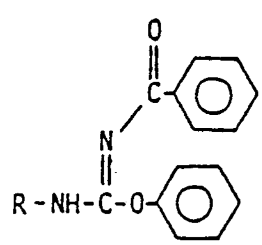

(II)

in which R has the meaning indicated above is reacted with a compound corresponding to the general formula III

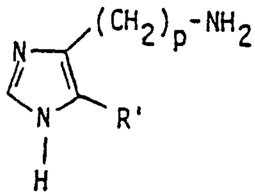

(III)

wherein R′ and p have the meanings indicated above to form a compound corresponding to the general formula I or

($a_2$) a compound corresponding to the general formula IV

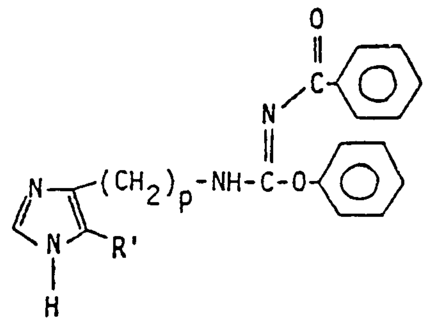

(IV)

wherein R′ and p have the meanings indicated above is reacted with a compound corresponding to the general formula V

$$R—NH_2 \qquad (V)$$

in which R has the meaning indicated above to form a compound corresponding to the general formula I or

b) for the preparation of compounds corresponding to the general formula I in which R, A, B, p and R′ are defined as above and X stands for a hydrogen atom,

($b_1$) a compound corresponding to formula Ia

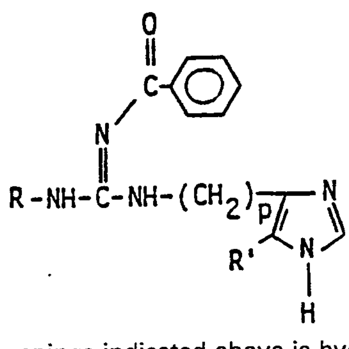

(Ia)

wherein R, p and R′ have the meanings indicated above is hydrolysed or

($b_2$) a compound corresponding to the general formula VI

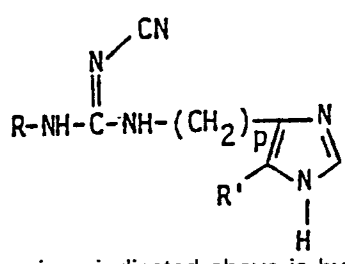

(VI)

wherein R, p and R′ have the meanings indicated above is hydrolysed by means of an acid to form a compound corresponding to the general formula I or

($b_3$) a compound corresponding to the general formula VII

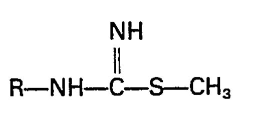

VII

wherein R has the meaning indicated above is reacted with a compound corresponding to the above indicated general formula III wherein R′ and p have the meanings indicated above to form a compound corresponding to the general formula I, or

($b_4$) a compound corresponding to the general formula VIII

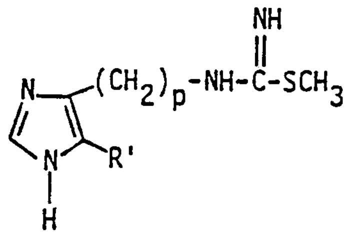

(VIII)

wherein R′ and p have the meanings indicated above is reacted with a compound corresponding to the above indicated general formula V in which R has the meaning indicated above to form a compound corresponding to the general formula I and in that the compounds obtained under a) and b) are optionally converted into their physiologically acceptable salt.

2. Process according to Claim 1 for the preparation of compounds in which R stands for the group

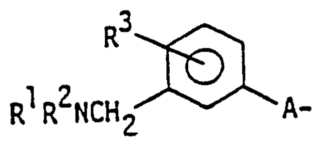

$R^1$ and $R^2$ together with the nitrogen atom form a pyrrolidine or piperidine ring and $R^3$ stands for a hydrogen atom, A denotes the group $—O—(CH_2)_k—$, $—O—CH_2CH(OH)CH_2—$ or $—O—CH_2CH(CH_3)CH_2—$, X and R′ stand each for a hydrogen atom and k and p each have the value 3.

3. Process according to Claim 1 for the preparation of compounds wherein R stands for the group

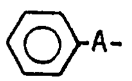

or

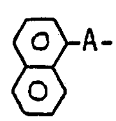

wherein A has the meaning indicated in Claim 1, X and R′ stand each for a hydrogen atom and p has the value 3.

4. Process according to Claim 1 for the preparation of compounds in which R stands for the group

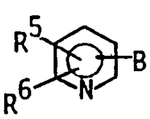

wherein $R^5$ stands for a hydrogen atom or halogen atom attached in the 5-position of the pyridine ring, $R^6$ stands for a methyl or methoxy group or a hydrogen atom attached in the 3-position of the pyridine ring, B may be attached in position 2, 3 or 4 of the pyridine ring and denotes the group

$$—N—(CH_2)_3—,$$
$$|$$
$$CH_3$$

X and R′ stand each for a hydrogen atom and p has the value 3.

5. Process according to Claim 1 for the preparation of compounds in which R denotes the group R″—A′—B′— wherein R″ stands for an unsubstituted phenyl group, A′ denotes a single bond, B′ stands for the group $—CH_2—S—(CH_2)_{m'}—$ or $—CH_2—S—CH_2—CH(Y)—CH_2—$ wherein m′ and Y have the meanings indicated above, X and R′ stand each for a hydrogen atom and p has the value 3.

6. Process according to Claim 1 for the preparation of compounds in which R denotes the group R″—A′—B′— wherein R″ stands for a phenyl group which may be unsubstituted or substituted with a halogen atom, with a $C_1—C_3$ alkyl group or with a $C_1—C_3$ alkoxy group, A′ denotes a nitrogen atom substituted with a phenyl or benzyl group, B′ stands for the group $—(CH_2)_{n''}—$ wherein n″ has the value 2 or 3, X and R′ stand each for a hydrogen atom and p has the value 3.

7. Process according to Claim 1 for the preparation of compounds in which R denotes the group R″—A′—B′— wherein R″ stands for a phenyl group which may be unsubstituted or substituted with a halogen atom, with a $C_1—C_3$ alkyl group or with a $C_1—C_3$ alkoxy group, A′ stands for the group $—CR^{1'}R^{2'}$ wherein $R^{1'}$ denotes a hydrogen atom or a methyl group, $R^{2'}$ stands for a phenyl or pyridyl group optionally substituted with a halogen atom or with a $C_1—C_3$ alkyl group, B′ denotes the group $—(CH_2)_{n''}—$ wherein n″ has the value 2, 3 or 4, X and R′ stand each for a hydrogen atom and p has the value 3.

8. Process according to Claim 1 for the preparation of compounds in which R denotes the group R'''—A''—B'' wherein R''' stands for a thiophene ring which may be unsubstituted or substituted with halogen atoms, with $C_1$—$C_3$ alkyl groups or with the group $R^1R^2NCH_2$, A'' denotes a single bond, B'' stands for the group —$CH_2$—S—$(CH_2)_{m'}$— or —$CH_2$—S—$CH_2$—CH(Y)—$CH_2$—, wherein m' and Y have the meanings indicated above, X and R' stand each for a hydrogen atom and p has the value 3.

9. Process according to Claim 1 for the preparation of compounds in which R denotes the group R'''—A''—B''— wherein R''' stands for a pyridine ring which may be unsubstituted or substituted with halogen atoms or with $C_1$—$C_3$ alkyl groups, A'' denotes the group —$CR^{1'}R^{2'}$ wherein $R^{1'}$ and $R^{2'}$ are defined as in Claim 1, B'' stands for the group —$(CH_2)_{n''}$— wherein n'' has the meaning indicated in Claim 1, X and R' stand each for a hydrogen atom and p has the value 3.

10. Process according to Claim 1 for the preparation of compounds in which R denotes the group R'''—A''—B''— wherein R''' stands for a pyridine ring which may be unsubstituted or substituted with halogen atoms or with $C_1$—$C_3$ alkyl groups, A'' stands for a nitrogen atom substituted with a phenyl or benzyl group, which phenyl or benzyl group is optionally substituted with halogen atoms, with $C_1$—$C_3$ alkyl groups or with $C_1$—$c_3$ alkoxy groups, B'' stands for the group —$(CH_2)_{n''}$— wherein n'' has the meaning indicated in Claim 1, X and R' stand each for a hydrogen atom and p has the value 3.

11. Process according to Claim 1 for the preparation of N-[3-[(N-5-methyl-pyridin-2-yl)-methylamino]propyl]-N'-[3-(1H-imidazol-4-yl)propyl]guanidine and the physiologically acceptable salts thereof.

12. Process according to Claim 1 for the preparation of N-[3-(imidazol-4-yl)propyl]-N'-(3,3-diphenylpropyl)guanidine and the physiologically acceptable salts thereof.

13. Process according to Claim 1 for the preparation of N-[3-(imidazol-4-yl)propyl]-N'-[2-[(1-phenylethyl)thio]ethyl]guanidine and the physiologically acceptable salts thereof.

14. Process according to Claim 1 for the preparation of N-[3-(imidazol-4-yl)propyl]-N'-[2-[(pyrid-3-yl)methylthio]ethyl]guanidine and the physiologically acceptable salts thereof.

15. Process according to Claim 1 for the preparation of N-[3-(imidazol-4-yl)propyl]-N'-[2-(2-phenylthio)éthyl]guanidine and the physiologically acceptable salts thereof.

16. Process according to Claim 1 for the preparation of N-[3-(imidazol-4-yl)propyl]-N'-[1-methyl-2-[(pyrid-2-yl)-methylthio]ethyl]guanidine and the physiologically acceptable salts thereof.

17. Process according to Claim 1 for the preparation of N-[3-(imidazol-4-yl)propyl]-N'-[3-phenyl-3-(pyrid-2-yl)propyl]guanidine and the physiologically acceptable salts thereof.

18. Process according to Claim 1 for the preparation of N-[3-(4-chlorophenyl)-3-(pyrid-2-yl)propyl]-N'-[3-(imidazol-4-yl)propyl]guanidine and the physiologically acceptable salts thereof.

19. Process according to Claim 1 for the preparation of N-[3-(4-bromophenyl)-3-(pyrid-2-yl)propyl]-N'-[3-(imidazol-4-yl)propyl]guanidine and the physiologically acceptable salts thereof.

20. Process according to Claim 1 for the preparation of N-[3-(4-fluorophenyl)-3-(pyrid-2-yl)propyl]-N'-[3-(imidazol-4-yl)propyl]guanidine and the physiologically acceptable salts thereof.